# EUROPEAN PATENT APPLICATION

(11) **EP 2 133 343 A1**
(43) Date of publication of application: **16.12.2009**
(21) Application number: 08721717.0
(22) Date of filing: 10.03.2008
(51) Int. Cl.: C07D 301/12, B01J 31/22, C07D 303/04, C07D 493/04, C07B 53/00, C07B 61/00

(54) **PROCESS FOR PRODUCTION OF OPTICALLY ACTIVE EPOXY COMPOUND**

(30) Priority: 10.03.2007 JP 2007061129
(71) Applicant: Nissan Chemical Industries, Ltd., Tokyo 101-0054 (JP)
(72) Inventor: KONDO, Shoichi, Funabashi-shi Chiba 274-8507 (JP); SHIMADA, Yuya, Funabashi-shi Chiba 274-8507 (JP)
(74) Representative: HOFFMANN EITLE
(86) International application number: PCT/JP2008/054301
(87) International publication number: WO 2008/111557

(57) **Abstract**

[PROBLEMS] To provide an efficient process for producing an optically active epoxy compound.

[MEANS FOR SOLVING PROBLEMS] The process for producing an optically active epoxy compound comprises asymmetrically epoxidizing an unsaturated compound with an oxidizing agent in the presence of an optically active titanium-salen complex, an optically active titanium-salalen complex or an optically active titanium-salan complex, with addition of a buffering agent or a buffer solution. The process can inhibit catalyst degradation, reduce the amount of the catalyst used in the reaction, and inhibit a by-product, compared with the prior art, and can provide an optically active epoxy compound in high chemical yield and optical yield and with high quality, and therefore is an industrially useful process.

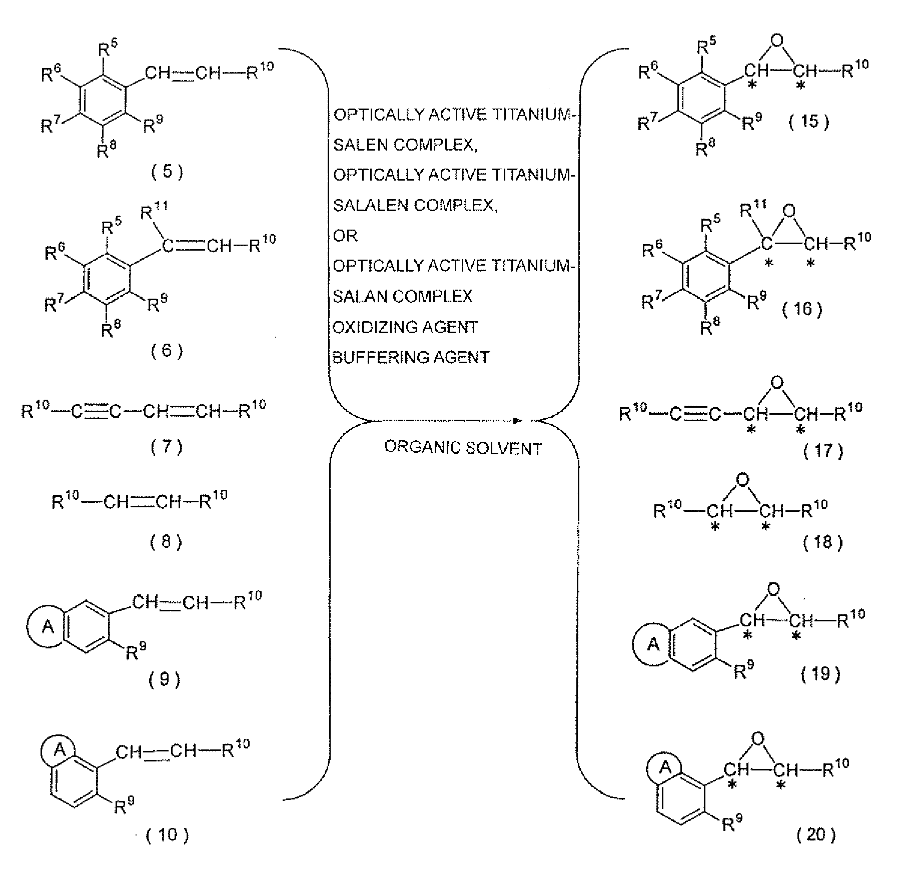

## Description

### TECHNICAL FIELD

The present invention relates to a process for producing an optically active epoxy compound.

### BACKGROUND ART

In optically active titanium complexes, it was reported in 2005 that using di-µ-oxotitanium-salalen complexes leads to progress in asymmetric epoxidation reactions with aqueous hydrogen peroxide as an oxidizing agent for various olefins with high enantioselectivity. However, the optically active titanium complex used in the art has a huge molecular weight of nearly 2000, and the synthesis of the titanium-salalen complex is costly and time-consuming. Furthermore, the synthesis of the titanium-salalen complex involves an intramolecular Meerwein-Ponndorf-Verley reduction, so that there is a problem that the complex is insufficient for applications (for example, see Non-Patent Document 1).

Then, it was reported in 2006 that using di-µ-oxotitanium-salan complexes leads to progress in asymmetric epoxidation reactions with aqueous hydrogen peroxide as an oxidizing agent for various olefins with high enantioselectivity. The optically active titanium complex used in the art has such a small molecular weight of about 1000 that the cost and time for the synthesis could be reduced in comparison with the titanium-salalen complex. However, there are problems that the reaction using the titanium-salan complex reduces the enantioselectivity and the chemical yield of the optically active epoxy compound and requires 5 mol% of the catalyst amount to be used, so that the amount of used catalyst cannot be reduced (for example, see Patent Document 1 and Non-Patent Document 2).

In addition, it was reported in 2006 that in the asymmetric epoxidation reaction using the titanium-salan complex as a catalyst, the substituents on the salan ligand were investigated to successfully improve the catalyst performance (for example, see Non-Patent Document 3). However, there is a problem that a part of the epoxy compound formed from the substrate becomes by-products under the reaction condition, leading to a reduction in the chemical yield. The purification of the by-products is costly and time-consuming, and therefore, a sufficiently satisfactory method has been required. Furthermore, the amount of the catalyst needs 4 to 6 mol%. Therefore, from the viewpoint of the purification of the catalyst and degradation products from the catalyst after reaction, a further reduction of the amount of the catalyst has also been required.

[Patent Document 1]
   International Publication WO 06/087874 pamphlet
[Non-Patent Document 1]
   K. Matsumoto, Y. Sawada, B. Saito, K. Sakai, T. Katsuki, Angew. Chem. Int. Ed. (2005),44,4935-4939.
[Non-Patent Document 2]
   Y. Sawada, K. Matsumoto, S. Kondo, H. Watanabe, T. Ozawa, K. Suzuki, B. Saito, K. Sakai, T. Katsuki, Angew. Chem. Int. Ed. (2006), 45, 3478-3480.
[Non-Patent Document 3]
   K. Matsumoto, Y. Sawada, T. Katsuki, SYNLETT (2006), 20, 3545-3547.

### DISCLOSURE OF THE INVENTION

### [Problem to be Solved by the Invention]

The problem to be solved by the invention is, in order to solve the problems in the related arts described above, to provide an industrially useful process for producing an optically active epoxy compound in which the degradation of a catalyst is inhibited or the efficiency of the catalyst to be used is improved to reduce the amount of used catalyst or to inhibit a side reaction while the reaction progresses sufficiently.

### [Means for Solving the Problem]

As a result of the intensive studies for an industrially useful process for producing an optically active epoxy compound, the present inventors have found that in the presence of an optically active titanium-salen complex, an optically active titanium-salalen complex or an optically active titanium-salan complex, in an asymmetric epoxidation using an oxidizing agent for an unsaturated compound having a prochiral carbon-carbon double bond in the molecule, addition of a buffering agent or a buffer solution to the reaction system can inhibit catalyst degradation, reduce the amount of the catalyst used in the reaction, and inhibit a by-product. Thus, the inventors have completed a practically and industrially useful process for producing an optically active epoxy compound in high chemical yield and optical yield and with high quality.

That is, the present invention provides:
1. a process for producing an optically active epoxy compound represented by any one of Formula (15), Formula (16), Formula (17), Formula (18), Formula (19) and Formula (20): 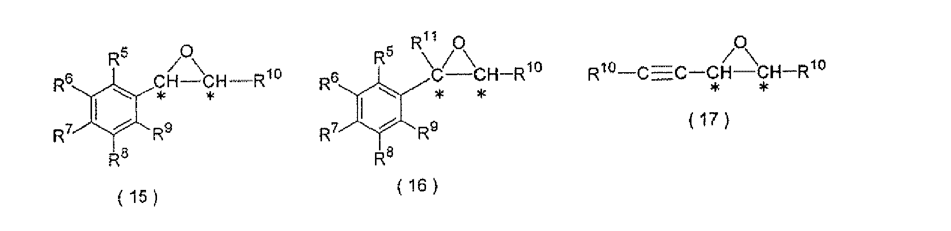 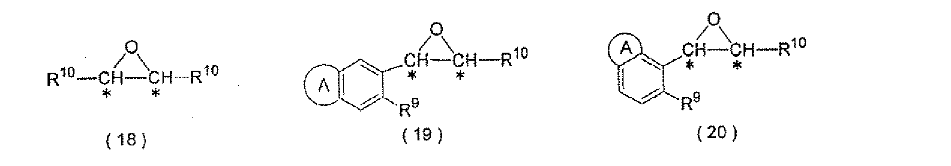 characterized by including:
   asymmetrically epoxidizing an unsaturated compound represented by any one of Formula (5), Formula (6), Formula (7), Formula (8), Formula (9) and Formula (10): 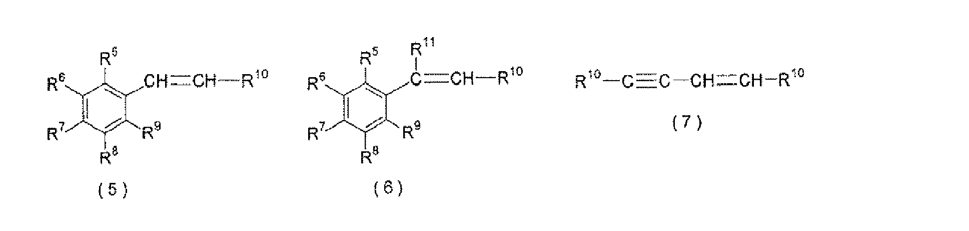 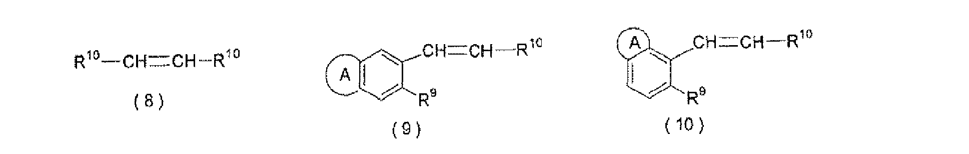 with an oxidizing agent; in the presence of an optically active titanium-salen complex, an optically active titanium-salalen complex or an optically active titanium-salan complex obtained from a reaction of an optically active ligand represented by any one of Formula (1), Formula (1'), Formula (2), Formula (2'), Formula (3), Formula (3'), Formula (4) and Formula (4'): 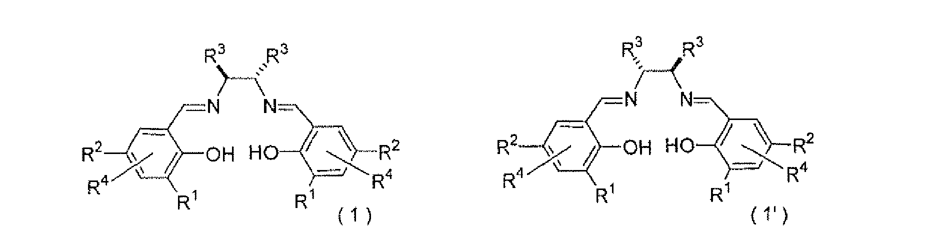 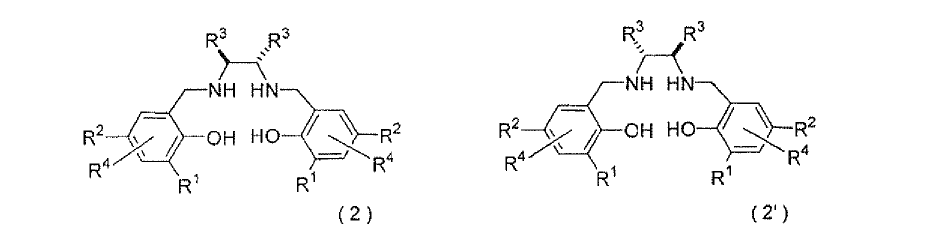 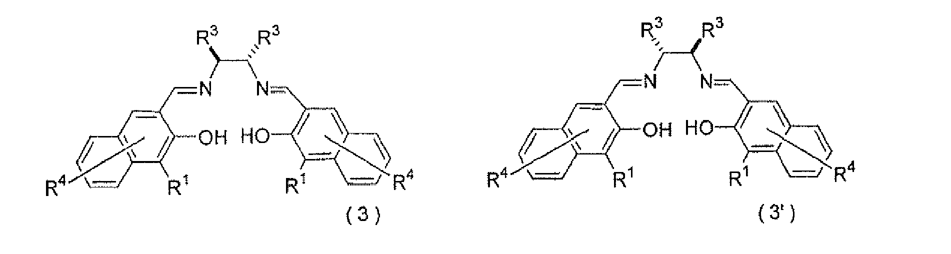 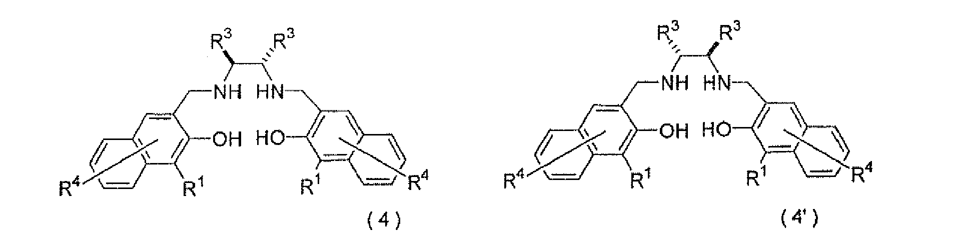 (Formula (1), Formula (1'), Formula (3) and Formula (3') are salen ligands and Formula (2), Formula (2'), Formula (4) and Formula (4') are salan ligands) and a titanium compound, and a buffering agent or a buffer solution, in which in Formula (1), Formula (1'), Formula (2), Formula (2'), Formula (3), Formula (3'), Formula (4) and Formula (4'),
      R¹ represents a hydrogen atom, a halogen atom, a C₁₋₄ alkyl group, a C₁₋₄ alkoxy group, a C₆₋₁₂ aryloxy group or a C₆₋₂₂ aryl group (the aryl group is unsubstituted or optionally substituted with a C₁₋₄ alkyl group (the alkyl group is unsubstituted or substituted with a halogen atom), a benzyloxy group or a C₁₋₄ alkoxy group, and is optically active or optically inactive),
      R² represents a hydrogen atom, a halogen atom or a C₁₋₄ alkyl group,
      R³ represents a C₆₋₁₈ aryl group or, when two R³ are joined together to form a ring, a C₃₋₅ bivalent group, and
      each R⁴ independently represents a hydrogen atom, a halogen atom, a C₁₋₄ alkyl group, a C₁₋₄ alkoxy group, a nitro group or a cyano group,
      in Formula (5) and Formula (6),
      each of R⁵, R⁶, R⁷ and R⁸ independently represents a hydrogen atom, a cyano group, a nitro group, an amino group (the amino group is not protected with a protective group or protected), a halogen atom, a C₁₋₄ alkyl group (the alkyl group is unsubstituted or substituted with a halogen atom), a C₁₋₄ alkoxy group, a carboxy group, a formyl group, a C₁₋₄ alkylcarbonyl group (the alkylcarbonyl group is unsubstituted or substituted with a halogen atom), a C₇₋₁₁ arylcarbonyl group (the arylcarbonyl group is unsubstituted or substituted with a halogen atom), a carbamoyl group, a C₁₋₄ alkylsulfinyl group, a C₆₋₁₀ arylsulfinyl group, a C₁₋₄ alkylsulfonyl group, a C₆₋₁₀ arylsulfonyl group, a sulfamoyl group, a monoalkylaminosulfonyl group or a C₂₋₈ dialkylaminosulfonyl group,
      in Formula (5), Formula (6), Formula (9) and Formula (10),
      R⁹ represents a hydrogen atom, a C₁₋₄ alkyl group or a C₁₋₄ alkoxy group,
      in Formula (5), Formula (6), Formula (9) and Formula (10),
      R¹⁰ represents a hydrogen atom, a C₁₋₂₂ alkyl group, a C₁₋₄ alkoxy group or a C₆₋₁₀ aryl group (the aryl group is unsubstituted or substituted with a halogen atom, a C₁₋₄ alkyl group or a C₁₋₄ alkoxy group),
      in Formula (6),
      R¹¹ represents a C₁₋₄ alkyl group (the alkyl group is unsubstituted or substituted with a halogen atom),
      in Formula (7) and Formula (8),
      each R¹⁰ independently represents a hydrogen atom, a C₁₋₂₂ alkyl group, a C₁₋₄ alkoxy group or a C₆₋₁₀ aryl group (the aryl group is unsubstituted or substituted with a halogen atom, a C₁₋₄ alkyl group or a C₁₋₄ alkoxy group),
      R⁹ and R¹⁰ in Formula (5), Formula (6), Formula (8) and Formula (9) may bind each other to form a bivalent group represented by any one of Formula (10), Formula (11), Formula (12) and Formula (13): 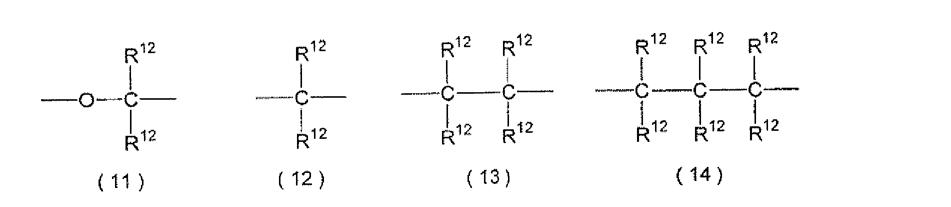
      (in Formula (11), Formula (12), Formula (13) and Formula (14),
      each R¹² independently represents a hydrogen atom or a C₁₋₆ alkyl group),
      in Formula (9) and Formula (10),
      a partial ring structure A represents any one of 5-, 6- and 7-membered rings fused with a benzene ring (each of the 5-, 6- and 7-membered rings is unsubstituted or substituted with h pieces of R¹³ (R¹³ represents a halogen atom, a hydroxyl group, a C₁₋₆ alkyl group (the alkyl group is unsubstituted or substituted with a halogen atom, a hydroxyl group, a cyano group, an amino group, a nitro group, a C₁₋₄ alkoxy group, a C₁₋₄ alkylcarbonyloxy group, a C₁₋₄ alkylcarbonylamino group or a C₁₋₄ alkoxycarbonyl group (the alkoxy group is unsubstituted or the alkylcarbonyloxy group, the alkylcarbonylamino group or the alkoxycarbonyl group is substituted with a halogen atom)), a C₁₋₆ alkoxy group (the alkoxy group is unsubstituted or substituted with a halogen atom, a hydroxyl group, a cyano group, an amino group, a nitro group, a C₁₋₄ alkoxy group, a C₁₋₄ alkylcarbonyloxy group, a C₁₋₄ alkylcarbonylamino group or a C₁₋₄ alkoxycarbonyl group (the alkoxy group, the alkylcarbonyloxy group, the alkylcarbonylamino group and the alkoxycarbonyl group are unsubstituted or substituted with halogen atoms)), a nitro group, a cyano group, a formyl group, a formamide group, a carbamoyl group, a sulfo group, a sulfoamino group, a sulfamoyl group, a sulfonyl group, an amino group, a carboxyl group, a C₁₋₆ alkylamino group, a di-C₁₋₆ alkylamino group, a C₁₋₆ alkylcarbonylamino group, a C₁₋₆ alkylsulfonamide group, a C₆₋₁₄ arylsulfonamide group, a C₁₋₆ alkylaminocarbonyl group, a di-C₁₋₆ alkylaminocarbonyl group, a C₁₋₆ alkylcarbonyl group, a C₁₋₆ alkoxycarbonyl group, a C₁₋₆ alkylsulfonyl group, a C₆₋₁₄ arylsulfonyl group or a C₆₋₁₄ arylcarbonyl group (the alkylamino group, the dialkylamino group, the alkylcarbonylamino group, the alkylsulfonamide group, the arylsulfonamide group, the alkylaminocarbonyl group, the dialkylaminocarbonyl group, the alkylcarbonyl group, the alkoxycarbonyl group, the alkylsulfonyl group, the arylsulfonyl group and the arylcarbonyl group are unsubstituted or substituted with halogen atoms), h means an integer from 1 to 6, and when h is 2 to 6, each R¹³ may be the same or different), and may include 1 to 3 atoms of an oxygen atom, a nitrogen atom or a sulfur atom singly or in combination as a ring constituent atom,
      the number of unsaturated bonds in the ring is 1, 2 or 3 including the unsaturated bond in the fused benzene ring, and a carbon atom constituting the ring may be carbonyl or thiocarbonyl), and
      in Formula (15), Formula (16), Formula (17), Formula (18), Formula (19) and Formula (20),
      R⁵, R⁶, R⁷, R⁸, R⁹, R¹⁰ and R¹¹ are the same as the above, and the absolute configuration of the carbon atom shown with an asterisk (*) means (R) or (S);
2. the process for producing an optically active epoxy compound according to the above-mentioned 1,
   in which the optically active ligand is represented by Formula (1), Formula (1'), Formula (2), Formula (2'), Formula (3), Formula (3'), Formula (4) and Formula (4')
   (in Formula (1), Formula (1'), Formula (2), Formula (2'), Formula (3), Formula (3'), Formula (4) and Formula (4'),
   R¹ represents a phenyl group (the phenyl group is substituted with a 2-C₁₋₃ alkyl group (the 2-C₁₋₃ alkyl group is substituted with at least one halogen atom), a benzyloxy group or a 2-C₁₋₄ alkoxy group), a 2-phenyl-1-naphtyl group or a 2-methoxy-1-naphtyl group,
   R² represents a hydrogen atom,
   R³ represents a tetramethylene group as the bivalent group, and
   R⁴ represents a hydrogen atom);
3. the process for producing an optically active epoxy compound according to the above-mentioned 1, in which the optically active epoxy compound is represented by Formula (21) or Formula (22): 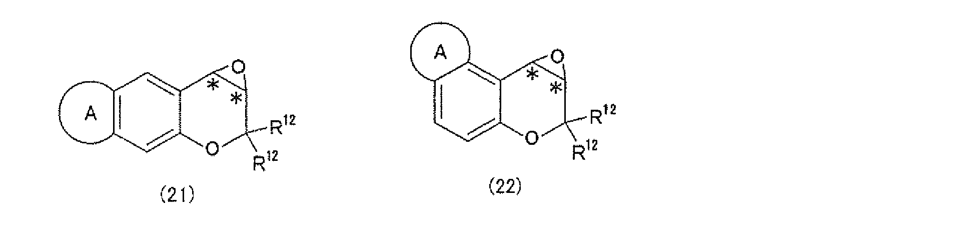 (in Formula (21) and Formula (22),
   the partial ring structure A and R¹² are the same as the above, and the absolute configuration of the carbon atom shown with * means (R) or (S)), and characterized in that the unsaturated compound is asymmetrically epoxidized with the oxidizing agent
   in which the partial ring structure A in Formula (9) or Formula (10) is represented by any one of Formula (a), Formula (b), Formula (c), Formula (d), Formula (e), Formula (f), Formula (g), Formula (h), Formula (i), Formula (j), Formula (k), Formula (l), Formula (m), Formula (n), Formula (o), Formula (p), Formula (q), Formula (r), Formula (s), Formula (t), Formula (u), Formula (v), Formula (w), Formula (x), Formula (y), Formula (z), Formula (aa), Formula (ab), Formula (ac), Formula (ad), Formula (ae), Formula (af), Formula (ag) and Formula (ah): 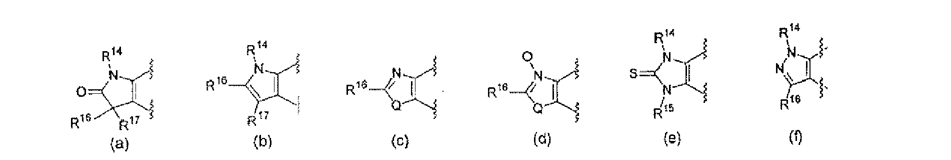 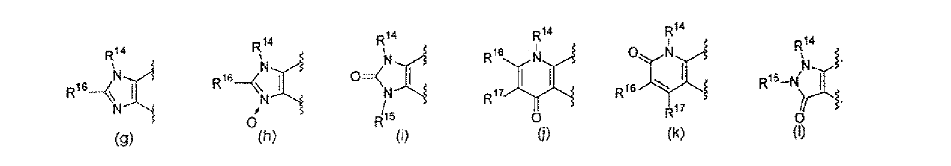 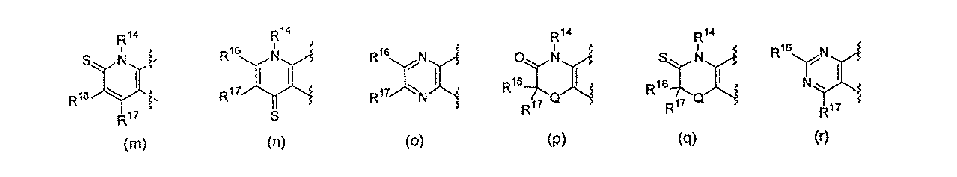 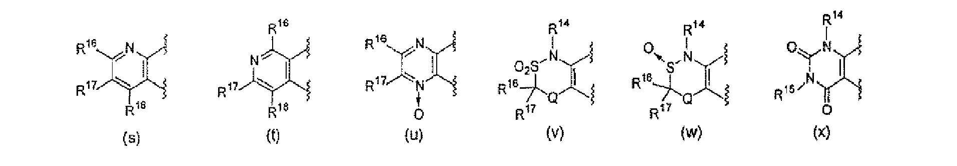 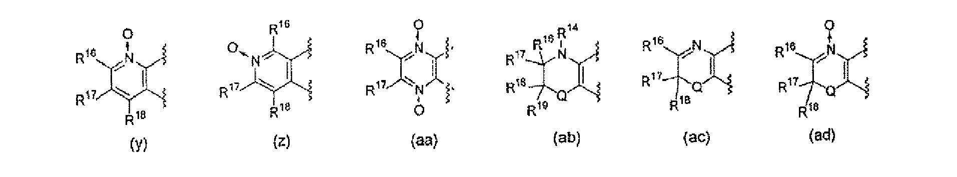 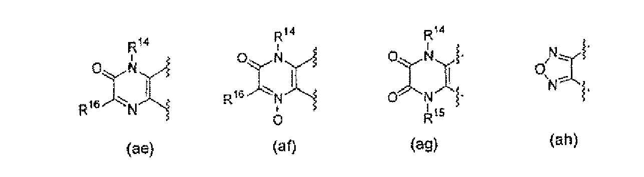 (in Formula (a), Formula (b), Formula (e), Formula (f), Formula (g), Formula (h), Formula (i), Formula (j), Formula (k), Formula (l), Formula (m), Formula (n), Formula (p), Formula (q), Formula (v), Formula (w), Formula (x), Formula (ab), Formula (ae), Formula (af) and Formula (ag),
   each of R¹⁴ and R¹⁵ independently represents a hydrogen atom, a C₁₋₆ alkyl group (the alkyl group is unsubstituted or substituted with a halogen atom, a C₁₋₆ alkoxy group (the alkoxy group is unsubstituted or substituted with a halogen atom), an amino group, a hydroxyl group, a C₆₋₁₄ aryl group, a C₂₋₉ heteroaryl group (each of the aryl group and the heteroaryl group is unsubstituted or substituted with q pieces of R²⁰ (R²⁰ means the same as R¹³ , q represents an integer from 1 to 3, and when q is 2 or 3, R²⁰ may be the same or different)), a C₁₋₆ alkylaminocarbonyl group, a di-C₁₋₆ alkylaminocarbonyl group, a C₁₋₆ alkylcarbonyloxy group, a C₁₋₆ alkylcarbonyl group (the alkylcarbonyloxy group and the alkylcarbonyl group are unsubstituted or substituted with halogen atoms), a C₁₋₆ alkylcarbonylamino group, a C₃₋₈ cycloalkylcarbonyl group, a C₁₋₆ alkoxycarbonyl group, a C₁₋₆ alkylsulfonyl group (the cycloalkylcarbonyl group, the alkoxycarbonyl group and the alkylsulfonyl group are unsubstituted or substituted with halogen atoms), a carboxyl group, a C₆₋₁₄ arylcarbonyl group (the arylcarbonyl group is unsubstituted or substituted with a halogen atom) or a C₂₋₉ heteroarylcarbonyl group), a C₆₋₁₄ aryl group, a C₂₋₉ heteroaryl group (each of the aryl group and the heteroaryl group is unsubstituted or substituted with q pieces of R²⁰ (R²⁰ means the same as R¹³, q represents an integer from 1 to 3, and when q is 2 or 3, R²⁰ may be the same or different)), a C₁₋₆ alkylaminocarbonyl group, a di-C₁₋₆ alkylaminocarbonyl group, a C₁₋₆ alkylcarbonyl group, a C₃₋₈ cycloalkylcarbonyl group, a C₁₋₆ alkoxycarbonyl group, a C₁₋₆ alkylsulfonyl group, a C₆₋₁₄ arylsulfonyl group, a C₂₋₉ heteroarylsulfonyl group (each of the arylsulfonyl group and the heteroarylsulfonyl group is unsubstituted or substituted with q pieces of R²⁰ (R²⁰ means the same as R¹³, q represents an integer from 1 to 3, and when q is 2 or 3, R²⁰ may be the same or different)), a carboxyl group, a C₆₋₁₄ arylcarbonyl group or a C₂₋₉ heteroarylcarbonyl group (each of the arylcarbonyl group and the heteroarylcarbonyl group is unsubstituted or substituted with q pieces of R²⁰ (R²⁰ means the same as R¹³, q represents an integer from 1 to 3, and when q is 2 or 3, R²⁰ may be the same or different)),
   in Formula (a), Formula (b), Formula (c), Formula (d), Formula (f). Formula (g), Formula (h), Formula (j), Formula (k), Formula (m), Formula (n), Formula (o), Formula (p), Formula (q), Formula (r), Formula (s), Formula (t), Formula (u), Formula (v), Formula (w), Formula (y), Formula (z), Formula (aa), Formula (ab), Formula (ac), Formula (ad), Formula (ae) and Formula (af),
   each of R¹⁶, R¹⁷, R¹⁸ and R¹⁹ independently represents a hydrogen atom, a halogen atom, a C₁₋₆ alkyl group (the alkyl group is unsubstituted or substituted with a halogen atom, a C₁₋₆ alkoxy group (the alkoxy group is unsubstituted or substituted with a halogen atom), an amino group, a hydroxyl group, a C₆₋₁₄ aryl group, a C₂₋₉ heteroaryl group (each of the aryl group and the heteroaryl group is unsubstituted or substituted with r pieces of R²¹ (R²¹ means the same as R¹³ and r means the same as q)), a C₁₋₆ alkylaminocarbonyl group, a di-C₁₋₆ alkylaminocarbonyl group, a C₁₋₆ carbonyloxy group, a C₁₋₆ alkylcarbonyl group (the alkylcarbonyloxy group and the alkylcarbonyl group are unsubstituted or substituted with a halogen atom), a C₁₋₆ alkylcarbonylamino group, a C₃₋₈ cycloalkylcarbonyl group, a C₁₋₆ alkoxycarbonyl group, a C₁₋₆ alkylsulfonyl group (the cycloalkylcarbonyl group, the alkoxycarbonyl group and the alkylsulfonyl group are unsubstituted or substituted with a halogen atom), a carboxyl group, a C₆₋₁₄ arylcarbonyl group (the arylcarbonyl group is unsubstituted or substituted with a halogen atom) or a C₂₋₉ heteroarylcarbonyl group), a C₃₋₈ cycloalkyl group (the cycloalkyl group is unsubstituted or substituted with a halogen atom, a C₁₋₆ alkoxy group (the alkoxy group is unsubstituted or substituted with a halogen atom), an amino group or a hydroxyl group), a C₁₋₆ alkoxy group (the alkoxy group is unsubstituted or substituted with a halogen atom, a C₁₋₆ alkoxy group (the alkoxy group is unsubstituted or substituted with a halogen atom), a carboxyl group, an amino group, a hydroxyl group, a C₆₋₁₄ aryl group or a C₂₋₉ heteroaryl group (each of the aryl group and the heteroaryl group is unsubstituted or substituted with r pieces of R²¹ (R²¹ means the same as R¹³ and r means the same as q))), a C₁₋₆ thioalkoxy group (the thioalkoxy group is unsubstituted or substituted with a halogen atom, a C₁₋₆ alkoxy group (the alkoxy group is unsubstituted or substituted with a halogen atom), a carboxyl group, a hydroxyl group, a C₆₋₁₄ aryl group, or a C₂₋₉ heteroaryl group (each of the aryl group and the heteroaryl group is unsubstituted or substituted with r pieces of R²¹ (R²¹ means the same as R¹³ and r means the same as q))), a hydroxyl group, a C₆₋₁₄ aryl group, a C₂₋₉ heteroaryl group (each of the aryl group and the heteroaryl group is unsubstituted or substituted with r pieces of R²¹ (R²¹ means the same as R¹³ and r means the same as q)), a C₁₋₆ alkylcarbonyloxy group, a nitro group, a cyano group, a formyl group, a formamide group, an amino group, a sulfo group, a C₁₋₆ alkylamino group, a di-C₁₋₆ alkylamino group, a C₆₋₁₄ arylamino group, a C₂₋₉ heteroarylamino group (each of the arylamino group and the heteroarylamino groups is unsubstituted or substituted with r pieces of R²¹ (R²¹ means the same as R¹³ and r means the same as q)), a C₁₋₆ alkylcarbonylamino group, a C₁₋₆ alkylsulfonamide group, a carbamoyl group, a C₁₋₆ alkylaminocarbonyl group, a di-C₁₋₆ alkylaminocarbonyl group, a C₁₋₆ alkylcarbonyl group, a C₆₋₁₄ arylcarbonyl group, a C₂₋₉ heteroarylcarbonyl group (each of the arylcarbonyl group and the heteroarylcarbonyl group is unsubstituted or substituted with r pieces of R²¹ (R²¹ means the same as R¹³ and r means the same as q)), a C₁₋₆ alkoxycarbonyl group, a sulfamoyl group, a C₁₋₆ alkylsulfonyl group, a C₆₋₁₄ arylsulfonyl group, a C₂₋₉ heteroarylsulfonyl group (each of the arylsulfonyl group and the heteroarylsulfonyl group is unsubstituted or substituted with r pieces af R²¹ (R²¹ means the same as R¹³ and r means the same as q)), a carboxyl group or a C₂₋₉ heterocyclyl group (the heterocyclyl group is unsubstituted or substituted with a halogen atom, a C₁₋₆ alkyl group (the alkyl group is unsubstituted or substituted with a halogen atom, a C₁₋₆ alkoxy group (the alkoxy group is unsubstituted or substituted with a halogen atom), an amino group, a carboxyl group or a hydroxyl group), a C₁₋₆ alkoxy group (the alkoxy group is unsubstituted or substituted with a halogen atom), a C₆₋₁₄ aryl group, a C₂₋₉ heteroaryl group (each of the aryl group and the heteroaryl group is unsubstituted or substituted with r pieces of R²¹ (R²¹ means the same as R¹³ and r means the same as q)), a hydroxyl group, a nitro group, a cyano group, a formyl group, a formamide group, an amino group, a C₁₋₆ alkylamino group, a di-C₁₋₆ alkylamino group, a C₁₋₆ alkylcarbonylamino group, a C₁₋₆ alkylsulfonamide group, a carbamoyl group, a C₁₋₆ alkylaminocarbonyl group, a di-C₁₋₆ alkylaminocarbonyl group, a C₁₋₆ alkylcarbonyl group, a C₁₋₆ alkoxycarbonyl group, a sulfamoyl group, a C₁₋₆ alkylsulfonyl group, a carboxyl group or a C₆₋₁₄ arylcarbonyl group), and
   in Formula (c), Formula (d), Formula (p), Formula (q), Formula (v), Formula (w), Formula (ab), Formula (ac) and Formula (ad),
   Q represents an oxygen atom (O) a sulfur atom (S), a sulfinyl group (SO) or a sulfonyl group (SO₂)),
   R⁹ and R¹⁰ in Formula (9) and Formula (10) bind each other to form a bivalent group represented by Formula (11): 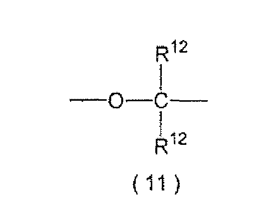
   (R¹² in Formula (11) represents a methyl group, the oxygen atom is bonded to the phenyl group, and the carbon atom is bonded to a vinyl group);
4. the process for producing an optically active epoxy compound according to any one of the above-mentioned 1 to 3, characterized in that the oxidizing agent is hydrogen peroxide;
5. the process for producing an optically active epoxy compound according to any one of the above-mentioned 1 to 4, characterized in that pH of a reaction solution is 5 to 12 by an addition of a buffering agent or a buffer solution;
6. the process for producing an optically active epoxy compound according to any one of the above-mentioned 1 to 5, characterized in that the optically active ligand is any one of the optically active ligands represented by Formula (2), Formula (2'), Formula (4) and Formula (4'); and
7. the process for producing an optically active epoxy compound according to any one of the above-mentioned I to 6, characterized in that the optically active ligand is any one of the optically active ligands represented by Formula (1), Formula (1'), Formula (3) and Formula (3'), and the unsaturated compound is asymmetrically epoxidized in the presence of an optically active titanium-salalen complex obtained from a reaction of the optically active ligand and a titanium compound, and a buffering agent or a buffer solution.

### [Effects of the Invention]

According to the present invention, an unsaturated compound with a prochiral carbon-carbon double bond in the molecule can be epoxidized with high enantioselectivity to produce an optically active epoxy compound with higher quality and in higher yield and more economically in comparison to the related art. In addition, the optically active epoxy compound obtained from the production process in the present invention are useful as optically active pharmaceutical intermediates for the compounds effective in the treatments of hypertension, asthma and the like.

### BEST MODES FOR CARRYING OUT THE INVENTION

In the present specification, "n" means normal, "i" means iso, "s" means secondary, "t" means tertiary, "c" means cyclo, "o" means ortho, "m" means meta, "p" means para and "Me" means a methyl group.

Hereinafter, the present invention will be described in detail. The process for producing an optically active epoxy compound of the present invention is characterized by, in the presence of an optically active titanium-salen complex, an optically active titanium-salalen complex or an optically active titanium-salan complex obtained from a reaction of the optically active ligand represented by any one of Formula (1), Formula (1'), Formula (2), Formula (2'), Formula (3), Formula (3'), Formula (4) and Formula (4'): 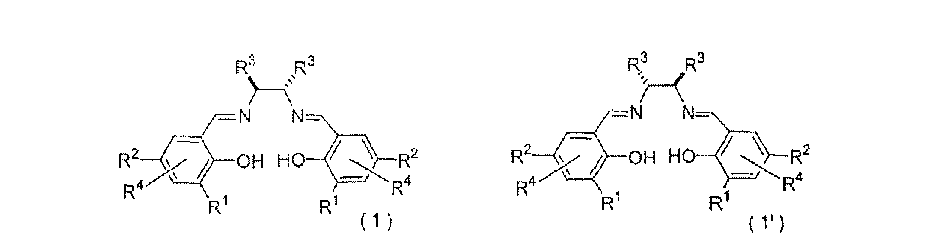 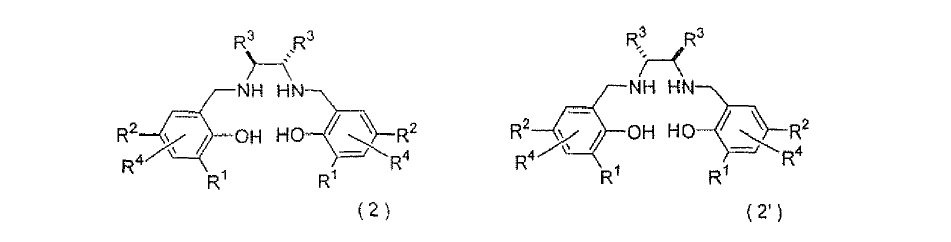 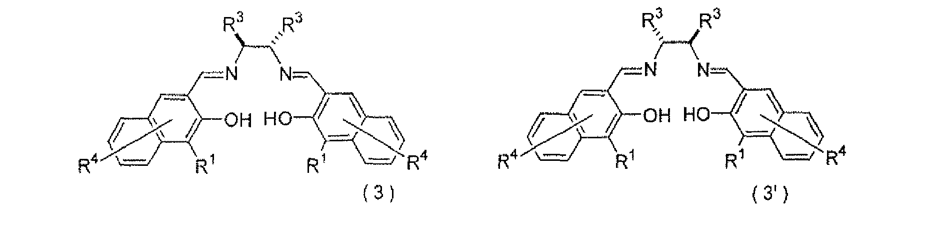 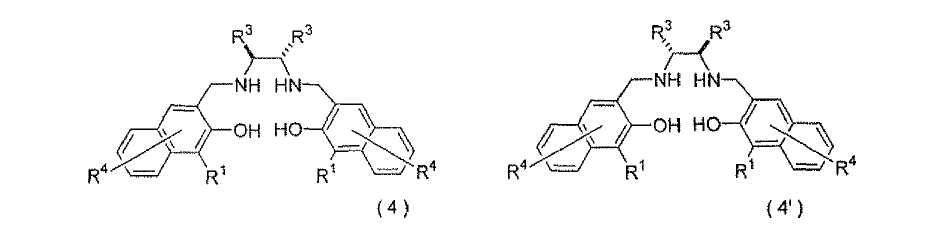 (Formula (1), Formula (1'), Formula (3) and Formula (3') are salen ligands and Formula (2), Formula (2'), Formula (4) and Formula (4') are salan ligands) (in Formula (1), Formula (1'), Formula (2), Formula (2'), Formula (3), Formula (3'), Formula (4) and Formula (4'), R¹ is a hydrogen atom, a halogen atom, a C₁₋₄ alkyl group, a C₁₋₄ alkoxy group, a C₆₋₁₂ aryloxy group or a C₆₋₂₂ aryl group (the aryl group is unsubstituted or optionally substituted with a C₁₋₄ alkyl group (the alkyl group is unsubstituted or substituted with a halogen atom), a benzyloxy group or a C₁₋₄ alkoxy group, and is optically active or optically inactive), R² is a hydrogen atom, a halogen atom or a C₁₋₄ alkyl group, R³ is a C₆₋₁₈ aryl group or, when two R³ are joined together to form a ring, a C₃₋₅ bivalent group, and each R⁴ is independently a hydrogen atom, a halogen atom, a C₁₋₄ alkyl group, a C₁₋₄ alkoxy group, a nitro group or a cyano group) and a titanium compound, and a buffering agent or a buffer solution, asymmetrically epoxidizing, with an oxidizing agent, the unsaturated compound represented by any one of Formula (5), Formula (6), Formula (7), Formula (8), Formula (9) and Formula (10): 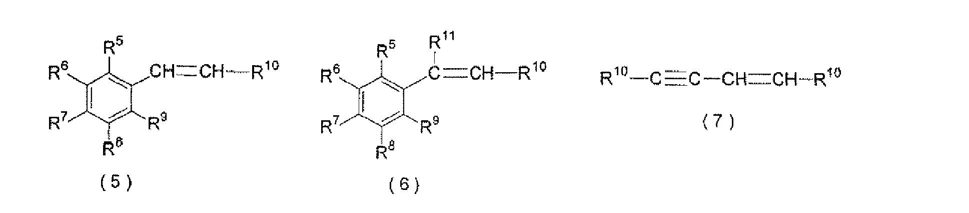 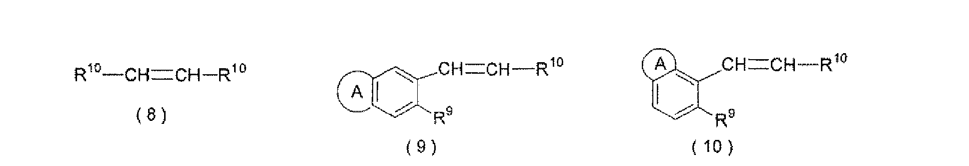
(each of R⁵, R⁶, R⁷ and R⁸ in Formula (5) and Formula (6) is independently a hydrogen atom, a cyano group, a nitro group, an amino group (the amino group is not protected with a protective group or protected), a halogen atom, a C₁₋₄ alkyl group (the alkyl group is unsubstituted or substituted with a halogen atom), a C₁₋₄ alkoxy group, a carboxy group, a formyl group, a C₁₋₄ alkylcarbonyl group (the alkylcarbonyl group is unsubstituted or substituted with a halogen atom), a C₇₋₁₁ arylcarbonyl group (the arylcarbonyl group is unsubstituted or substituted with a halogen atom), a carbamoyl group, a C₁₋₄ alkylsulfinyl group, a C₆₋₁₀ arylsulfinyl group, a C₁₋₄ alkylsulfonyl group, a C₆₋₁₀ arylsulfonyl group, a sulfamoyl group, a monoalkylaminosulfonyl group or a C₂₋₈ dialkylaminosulfonyl group, R⁹ in Formula (5), Formula (6), Formula (9) and Formula (10) is a hydrogen atom, a C₁₋₄ alkyl group or a C₁₋₄ alkoxy group, R¹⁰ in Formula (5), Formula (6), Formula (9) and Formula (10) is a hydrogen atom, a C₁₋₂₂ alkyl group, a C₁₋₄ alkoxy group, a C₆₋₁₀ aryl group (the aryl group is unsubstituted or substituted with a halogen atom, a C₁₋₄ alkyl group or a C₁₋₄ alkoxy group), R¹¹ in Formula (6) is a C₁₋₄ alkyl group (the alkyl group is unsubstituted or substituted with a halogen atom), each R¹⁰ in Formula (7) and Formula (8) is independently a hydrogen atom, a C₁₋₂₂ alkyl group, a C₁₋₄ alkoxy group, a C₆₋₁₀ aryl group (the aryl group is unsubstituted or substituted with a halogen atom, a C₁₋₄ alkyl group or a C₁₋₄ alkoxy group). However, R⁹ and R¹⁰ in Formula (5), Formula (6), Formula (8) and Formula (9) may bind each other to form the bivalent group represented by any one of Formula (10), Formulae (11), Formula (12) and Formula (13): 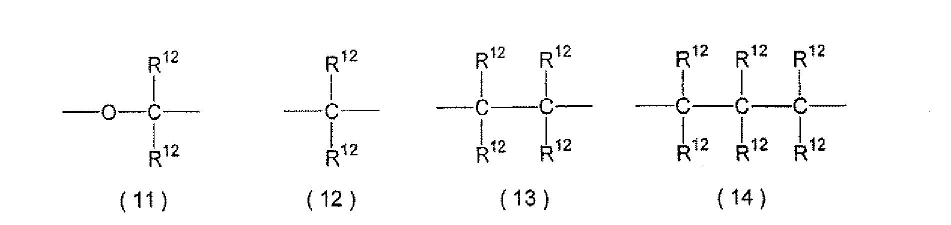
(each R¹² in Formula (11), Formula (12), Formula (13) and Formula (14) is independently a hydrogen atom or a C₁₋₆ alkyl group), each of the partial ring structure A in Formula (9) and Formula (10) is any of 5-, 6- and 7-membered rings fused with a benzene ring (each of the 5-, 6- and 7-membered rings is unsubstituted or substituted with h pieces of R¹³ (R¹³ is a halogen atom, a hydroxyl group, a C₁₋₆alkyl group (the alkyl group is unsubstituted or substituted with a halogen atom, a hydroxyl group, a cyano group, an amino group, a nitro group, a C₁₋₄ alkoxy group, a C₁₋₄ alkylcarbonyloxy group, a C₁₋₄ alkylcarbonylamino group or a C₁₋₄ alkoxycarbonyl group (the alkoxy group is unsubstituted or the alkylcarbonyloxy group, the alkylcarbonylamino group or the alkoxycarbonyl group is substituted with a halogen atom)), a C₁₋₆ alkoxy group (the alkoxy group is unsubstituted or substituted with a halogen atom, a hydroxyl group, a cyano group, an amino group, a nitro group, a C₁₋₄ alkoxy group, a C₁₋₄ alkylcarbonyloxy group, a C₁₋₄ alkylcarbonylamino group or a C₁₋₄ alkoxycarbonyl group (the alkoxy group, the alkylcarbonyloxy group, the alkylcarbonylamino group and the alkoxycarbonyl group are unsubstituted or substituted with a halogen atom)), a nitro group, a cyano group, a formyl group, a formamide group, a carbamoyl group, a sulfo group, a sulfoamino group, a sulfamoyl group, a sulfonyl group, an amino group, a carboxyl group, a C₁₋₆ alkylamino group, a di-C₁₋₆ alkylamino group, a C₁₋₆ alkylcarbonylamino group, a C₁₋₆ alkylsulfonamide group, a C₆₋₁₄ arylsulfonamide group, a C₁₋₆ alkylaminocarbonyl group, a di-C₁₋₆ alkylaminocarbonyl group, a C₁₋₆ alkylcarbonyl group, a C₁₋₆ alkoxycarbonyl group, a C₁₋₆ alkylsulfonyl group, a C₆₋₁₄ arylsulfonyl group or a C₆₋₁₄ arylcarbonyl group (the alkylamino group, the dialkylamino group, the alkylcarbonylamino group, the alkylsulfonamide group, the arylsulfonamide group, the alkylaminocarbonyl group, the dialkylaminocarbonyl group, the alkylcarbonyl group, the alkoxycarbonyl group, the alkylsulfonyl group, the arylsulfonyl group and the arylcarbonyl group are unsubstituted or substituted with a halogen atom), h means an integer from 1 to 6, and when h is 2 to 6, each R¹³ may be the same or different), and may include 1 to 3 atoms of an oxygen atom, a nitrogen atom or a sulfur atom singly or in combination as a ring constituent atom, the number of unsaturated bonds in the ring is 1, 2 or 3 including the unsaturated bond in the fused benzene ring, and the carbon atom constituting the ring may be carbonyl or thiocarbonyl)), allowing for production of the optically active epoxy compound represented by any one of Formula (15), Formula (16), Formula (17), Formula (18), Formula (19) and Formula (20): 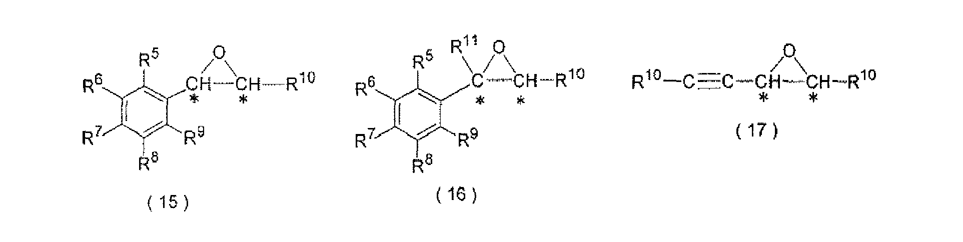 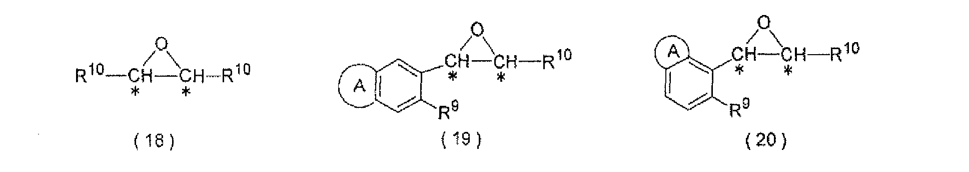
(R⁵, R⁶, R⁷, R⁸, R⁹, R¹⁰ and R¹¹ in Formula (15), Formula (16), Formula (17), Formula (18), Formula (19) and Formula (20) are the same as the above and the absolute configuration of the carbon atom shown with * means (R) or (S)).

Each substituent in Formula (1), Formula (1'), Formula (2), Formula (2'), Formula (3), Formula (3'), Formula (4) and Formula (4') will be described.

R¹ in Formula (1), Formula (1'), Formula (2), Formula (2'), Formula (3), Formula (3'), Formula (4) and Formula (4') is a hydrogen atom, a halogen atom, a C₁₋₄ alkyl group, a C₁₋₄ alkoxy group, a C₆₋₁₂ aryloxy group or a C₆₋₂₂ aryl group (the aryl group is unsubstituted or optionally substituted with a C₁₋₄ alkyl group (the alkyl group is unsubstituted or substituted with a halogen atom), a benzyloxy group or a C₁₋₄ alkoxy group, and is optically active or optically inactive).

R¹ in Formula (1), Formula (1'), Formula (2), Formula (2'), Formula (3), Formula (3'), Formula (4) and Formula (4') will be specifically described. Examples of the halogen atom include a fluorine atom, a chlorine atom, a bromine atom and an iodine atom; examples of the C₁₋₄ alkyl group include a methyl group, a trifluoromethyl group, a monochloromethyl group, an ethyl group, a pentafluoroethyl group, an n-propyl group, an i-propyl group, an n-butyl group, an i-butyl group, an s-butyl group, a t-butyl group and the like; examples of the C₁₋₄ alkoxy group include a methoxy group, an ethoxy group, an n-propoxy group, an i-propoxy group, an n-butoxy group, an i-butoxy group, an s-butoxy group, a t-butoxy group and the like; examples of the C₆₋₁₂ aryloxy group include a phenyloxy group, a I-naphtyloxy group, a 2-naphtyloxy group, a 2-biphenylyloxy group, a 3-biphenylyloxy group, a 4-biphenylyloxy group and the like; and examples of the C₆₋₂₂ aryl group include a phenyl group, a 2-methylphenyl group, a 2-trifluoromethylphenyl group, a 4-methylphenyl group, a 2-ethylphenyl group, a 2-pentafluoroethylphenyl group, a 3,5-dimethylphenyl group, a 2-methoxyphenyl group, a 3-methoxyphenyl group, a 4-methoxyphenyl group, a 2-ethoxyphenyl group, a 2-i-propoxyphenyl group, a 2-benzyloxyphenyl group, a 3,5-dimethoxyphenyl group, a 1-naphtyl group, a 2-naphtyl group, a 2-biphenylyl group, a 3-biphenylyl group, a 4-biphenylyl group, a 2-methyl-1-naphtyl group, a 2-phenyl-1-naphtyl group, a 2-methoxy-1-naphtyl group, a 2-(3,5-dimethylphenyl)-1-naphtyl group, a 2-(4-methylphenyl)-1-naphtyl group, a 2-(p-(t-butyldimethylsilyl)phenyl)-1-naphtyl group, a 2-(o-biphenylyl)-1-naphtyl group, a 2-(m-biphenylyl)-1-naphtyl group, a 2-(p-biphenylyl)-1-naphtyl group and the like.

Preferred R¹ in Formula (1), Formula (1'), Formula (2), Formula (2'), Formula (3), Formula (3'), Formula (4) and Formula (4') is a phenyl group (the phenyl group is substituted with a 2-C₁₋₃ alkyl group (the 2-C₁₋₃ alkyl group is substituted with at least one halogen atom), a benzyloxy group or a 2-C₁₋₄ alkoxy group), a 2-phenyl-1-naphtyl group or a 2-methoxy-1-naphtyl group. Examples of the phenyl group include a 2-trifluoromethylphenyl group, a 2-pentafluoroethylphenyl group, a 2-benzyloxyphenyl group, a 2-methoxyphenyl group, a 2-ethoxyphenyl group, a 2-i-propoxyphenyl group, a 2-n-butoxyphenyl group and the like.

R¹ in Formula (1), Formula (1'), Formula (2), Formula (2'), Formula (3), Formula (3'), Formula (4) and Formula (4') is preferably a 2-methoxyphenyl group, a 2-ethoxyphenyl group, a 2-i-propoxyphenyl group, a 2-benzyloxyphenyl group, a 2-trifluoromethylphenyl group, a 2-phenyl-1-naphtyl group or a 2-methoxy-1-naphtyl group, and more preferably a 2-methoxyphenyl group, a 2-trifluoromethylphenyl group, a 2-phenyl-1-naphtyl group or a 2-methoxy-1-naphtyl group.

R² in Formula (1), Formula (1'), Formula (2), Formula (2'), Formula (3), Formula (3'), Formula (4) and Formula (4') is a hydrogen atom, a halogen atom or a C₁₋₄ alkyl group.

R² in Formula (1), Formula (1'), Formula (2), Formula (2'), Formula (3), Formula (3'), Formula (4) and Formula (4') will be specifically described. Examples of the halogen atom include a fluorine atom, a chlorine atom, a bromine atom and an iodine atom; and examples of the C₁₋₄ alkyl group include a methyl group, an ethyl group, an n-propyl group, an i-propyl group, an n-butyl group, an i-butyl group, an s-butyl group, a t-butyl group and the like.

R² in Formula (1), Formula (1'), Formula (2), Formula (2'), Formula (3), Formula (3'), Formula (4) and Formula (4') is preferably a hydrogen atom, a fluorine atom, a chlorine atom, a bromine atom, an iodine atom, a methyl group, an ethyl group, an n-propyl group, an i-propyl group, an n-butyl group or a t-butyl group, and among them, R² is more preferably a hydrogen atom.

R³ in Formula (1), Formula (1'), Formula (2), Formula (2'), Formula (3), Formula (3'), Formula (4) and Formula (4') is a C₆₋₁₈ aryl group or, when two R³ are joined together to form a ring, a C₃₋₅ bivalent group.

R³ in Formula (1), Formula (1'), Formula (2), Formula (2'), Formula (3), Formula (3'), Formula (4) and Formula (4') will be specifically described. Examples of the C₆₋₁₈ aryl group include a phenyl group, a 3,5-dimethylphenyl group, a 2,4,6-trimethylphenyl group and a 4-methylphenyl group, and when two R³ are joined together to form a ring, a the C₃₋₅ bivalent group including a trimethylene group, a tetramethylene group and the like.

R³ in Formula (1), Formula (1'), Formula (2), Formula (2'), Formula (3), Formula (3'), Formula (4) and Formula (4') is preferably a phenyl group, a 3,5-dimethylphenyl group, a 2,4,6-trimethylphenyl group or a tetramethylene group formed by bonding two R³.

Each R⁴ in Formula (1), Formula (1'), Formula (2), Formula (2'), Formula (3), Formula (3'), Formula (4) and Formula (4') is independently a hydrogen atom, a halogen atom, a C₁₋₄ alkyl group, a C₁₋₄ alkoxy group, a nitro group or a cyano group.

R⁴ in Formula (1), Formula (1'), Formula (2), Formula (2'), Formula (3), Formula (3'), Formula (4) and Formula (4') will be specifically described. Examples of the halogen atom include a fluorine atom, a chlorine atom, a bromine atom and an iodine atom; examples of the C₁₋₄ alkyl group include a methyl group, an ethyl group, an n-propyl group, an i-propyl group, an n-butyl group, an i-butyl group, an s-butyl group, a t-butyl group and the like; and examples of the C₁₋₄ alkoxy group include a methoxy group, an ethoxy group, an n-propoxy group, an i-propoxy group, an n-butoxy group, an i-butoxy group, an s-butoxy group, a t-butoxy group and the like.

R⁴ in Formula (1), Formula (1'), Formula (2), Formula (2'), Formula (3), Formula (3'), Formula (4) and Formula (4') is preferably a hydrogen atom, a fluorine atom, a chlorine atom, a bromine atom, a methyl group, an ethyl group, an n-propyl group, an i-propyl group, an n-butyl group, an i-butyl group, an s-butyl group, a t-butyl group, a methoxy group, an ethoxy group, an n-propoxy group, an i-propoxy group, an n-butoxy group, an i-butoxy group, an s-butoxy group or a t-butoxy group, and among them for R⁴, a hydrogen atom is more preferable.

Among the optically active ligands represented by Formula (1), Formula (1'), Formula (2), Formula (2'), Formula (3), Formula (3'), Formula (4) and Formula (4') used for the production process of the present invention, especially preferred ligands are the salan ligands represented by Formula (2), Formula (2'), Formula (4) and Formula (4').

Optically active titanium-salalen complexes can be prepared according to the method described in Patent Document: International Publication WO 2006/08787, pamphlet and Non-Patent Document: Angew. Chem. Int. Ed. (2005), 44, 4935-4939. It is known from the literatures that a salen ligand is reacted with a titanium compound to be converted to the titanium-salalen complex through the Meerwein-Ponndorf-Verley reduction. Accordingly, the present invention also includes the production process using a titanium-salalen complex generated in the reaction system.

Optically active titanium-salan complexes can be prepared according to the methods described in Patent Document: International Publication WO 2006/08787, pamphlet and Non-Patent Document: Angew. Chem. Int. Ed. (2006), 45, 3478-3480.

Examples of the titanium compound to be used include titanium tetrachloride, titanium tetrabromide and titanium alkoxides. Furthermore, examples of the titanium alkoxide include titanium tetramethoxide, titanium tetraethoxide, titanium tetra-n-propoxide, titanium tetra-i-propoxide, titanium tetra-n-butoxide, titanium tetra-t-butoxide and the like. Among the titanium compounds, titanium tetra-n-propoxide, titanium tetra-i-propoxide, titanium tetra-n-butoxide and titanium tetra-t-butoxide are preferable and titanium tetra-iso-propoxide (Ti(Oi-Pr)₄) is more preferable. The amount of used titanium compound is preferably in the range from 0.6 to 10 molar equivalent per molar equivalent of the salen ligand or the salan ligand, and more preferably in the range from 0.8 to 1.2 molar equivalent.

The optically active titanium-salalen complex and the optically active titanium-salan complex can be prepared by either a method in which the complex is once isolated as an oxotitanium complex or a method in which the prepared complex in a reaction system is not isolated (an in situ method), to produce an optically active epoxy compound. Furthermore, the addition of water when the oxotitanium complex is prepared can be performed with water in aqueous hydrogen peroxide as the oxidizing agent used in the reaction.

The addition of a buffering agent or a buffer solution to be used to the reaction system includes a method in which the buffering agent or the buffer solution is added to the reaction solution without treatment and a method in which the buffering agent or the buffer solution is added after the buffering agent or the buffer solution is dissolved in an oxidizing agent to be used such as a 30% aqueous hydrogen peroxide. The methods can be properly selected according to the substrate to be used or the reaction condition.

Examples of the components used for the buffering agent include boric acid, phosphoric acid, citric acid, an alkaline phosphate, an alkali hydrogen phosphate, disodium hydrogen phosphate, dipotassium hydrogen phosphate, sodium dihydrogen phosphate, dipotassium hydrogen phosphate, disodium dihydrogen pyrophosphate, tetrasodium diphosphate, ammonium phosphate, ammonium hydrogen phosphate, ammonium nitrate, sodium acetate, ammonium acetate, sodium hydrogen carbonate, ammonium hydrogen carbonate, nitrilotriacetic acid (NTA) and alkaline salts thereof, ethylenedinitriloacetic acid (EDTA) or alkaline salts thereof, triethylenetetraminehexaacetic acid (TTHA), trisodium (N-(2-hydroxyethyl)-ethylenediamine-N,N,N'-triacetate, ethylene glycol-bis(β-aminoethyl ether)-N,N-tetraacetic acid (EGTA), 3-aza-3-(carboxymethyl)-pentamethylenedinitrilotetraacetic acid (DTPA), 1,2-cyclohexanediamine-N,N,N,N'-tetraacetic acid, tris(hydroxymethyl)aminomethane (Tris) and the like, and disodium hydrogen phosphate, dipotassium hydrogen phosphate, sodium dihydrogen phosphate, potassium dihydrogen phosphate and tris(hydroxymethyl)aminomethane (Tris) are preferred.

The buffer solution can be prepared according to the method described in Non-Patent Document: D.D. Perrin; B. Dempsey, "Buffers for pH and Metal Ion Control" ed by CHAPMAN AND HALL, London (1974) or Non-Patent Document: Kagaku Binran (Handbook of Chemistry) 3rd revised edition, Kiso-hen II (basics II) ed. by The Chemical Society of Japan (1984), pp. 354-357. Examples of the buffer solution include a citric acid-NaOH buffer solution, a citric acid-sodium citrate buffer solution, a phosphate buffer solution, a KH₂PO₄-NaOH buffer solution, a Tris-HCl buffer solution, a boric acid-NaOH buffer solution, a sodium borate-HCl buffer solution and the like, and a citric acid-NaOH buffer solution, a citric acid-sodium citrate buffer solution, a boric acid-NaOH buffer solution, a phosphate buffer solution and a KH₂PO₄-NaOH buffer solution are preferred.

Non-Patent Document: Jikkenkagakukoza (Experimental Chemical Course), 4th edition, vol. 23; Organic Synthesis V; Oxidation Reaction, ed. by The Chemical Society of Japan (1991), pp. 245-246 shows that in order to prevent production of by-products when epoxy compounds are unstable to acids, sodium hydrogen carbonate or sodium dihydrogen phosphate is added, as described above. In this case, it is known that the addition of sodium hydrogen carbonate or sodium dihydrogen phosphate inhibits the ring-opening of a formed epoxy compound by an acid component.

However, it is not sufficiently known which alkali component, buffering agent or buffer solution is added to inhibit the catalyst degradation with respect to the substrate of the present invention. Furthermore, there is no knowledge whether the optically active titanium complex used in the present invention can be stably present as a catalyst and whether it can work for the asymmetric epoxidation reaction in the presence of an alkali component, a buffering agent or a buffer solution. Therefore, comparative experiments were performed as follows: the optically active titanium complex as a catalyst was exposed under the reaction condition, after 15 minutes of stirring, an aqueous solution of sodium thiosulfate was added to reduce hydrogen peroxide, and the remaining amount of the catalyst was measured by an internal standard method. As a result, it was shown that the remaining amount of the catalyst in the system with the additive was higher than that without the additive (Reference Experiment A).

From the above investigation, it is found that in the presence of the optically active titanium-salen complex, the optically active titanium-salalen complex or the optically active titanium-salan complex, in the reaction of an unsaturated compound having a prochiral carbon-carbon double bond in its molecule and an oxidizing agent, the addition of the additive into the reaction system can reduce the amount of used catalyst, inhibit production of by-products and produce the optically active epoxy compound in high chemical yield, high optical yield and with good quality when compared to a related art.

The amount of the additive to be used is 0.001 to 200 mol% with respect to an unsaturated compound as the substrate, preferably the range from 0.01 to 100 mol%, and more preferably the range from 0.35 to 40 mol%.

The oxidizing agents used in the present invention are preferably aqueous hydrogen peroxide and urea-hydrogen peroxide adduct (UHP), and more preferably aqueous hydrogen peroxide. Here, the concentration of aqueous hydrogen peroxide is not specifically limited, but from the view point of safety, industry and availability, it is preferable that commercially available aqueous hydrogen peroxide with a concentration of about 30 to 60% is used. In addition, aqueous hydrogen peroxide may be diluted to be used or added dropwise in small portions to a solution for the epoxidation reaction, and in this case, the amount of used catalyst can be reduced. The amount of used oxidizing agent is preferably the range from 1 to 10 molar equivalent with respect to an unsaturated compound as the substrate, and more preferably the range from 1 to 1.5 molar equivalent.

The amount of used catalyst is preferably the range from 0.001 to 100 mol% with respect to an unsaturated compound as the substrate, and more preferably the range from 0.01 to 10 mol%.

The process for producing an optically active epoxy compound in the present invention is generally performed in an organic solvent. The organic solvent is preferably an aprotic organic solvent, and specific examples thereof include halogenated hydrocarbons such as dichloromethane (CH₂Cl₂), 1,2-dichloroethane (CH₂ClCH₂Cl) and chlorobenzene, aromatic hydrocarbons such as toluene, esters such as ethyl acetate, and ethers such as tetrahydrofuran (THF). Furthermore, when aqueous hydrogen peroxide is used as the oxidizing agent, the reaction solution may be the two phase system of an organic phase and an aqueous phase.

The reaction temperature in the process for producing an optically active epoxy compound in the present invention is not specifically limited, but the reaction is preferably carried out at the reflux temperature of a solvent to be used or at 0°C or higher, and more preferably carried out at from 25 to 40°C. If the reaction temperature is too high or too low, the enantiomeric excess of a product becomes low. Furthermore, the reaction time is not specifically limited and properly selected in accordance with the above reaction temperature.

According to the production process in the present invention, only one enantiomer can be produced in high selectivity. Furthermore, according to the production process in the present invention, the selection between the complex of Formula (1) and the complex of Formula (1') can lead to the selective production of each enantiomer of optically active epoxy compounds.

Each substituent in Formula (5), Formula (6), Formula (7), Formula (8), Formula (9), Formula (10), Formula (11), Formula (12), Formula (13), Formula (14), Formula (15), Formula (16), Formula (17), Formula (18), Formula (19) and Formula (20) will be described.

Each of R⁵, R⁶, R⁷ and R⁸ is independently a hydrogen atom, a cyano group, a nitro group, an amino group (the amino group is not protected with a protective group or protected), a halogen atom, a C₁₋₄ alkyl group (the alkyl group is unsubstituted or substituted with a halogen atom), a C₁₋₄ alkoxy group, a carboxy group, a formyl group, a C₁₋₄ alkylcarbonyl group (the alkylcarbonyl group is unsubstituted or substituted with a halogen atom), a C₇₋₁₁ arylcarbonyl group (the arylcarbonyl group is unsubstituted or substituted with a halogen atom), a carbamoyl group, a C₁₋₄ alkylsulfinyl group, a C₆₋₁₀ arylsulfinyl group, a C₁₋₄ alkylsulfonyl group, a C₆₋₁₀ arylsulfonyl group, a sulfamoyl group, a monoalkylaminosulfonyl group or a C₂₋₈ dialkylaminosulfonyl group.

R⁵, R⁶, R⁷ and R⁸ will be specifically described. Examples of the amino group include an amino group, a tosylamino group, a benzylamino group, a C₂₋₇ acylamino group, a C₂₋₅ alkoxycarbonylamino group and the like; examples of the acylamino group include an acetylamino group, a propionylamino group, a benzoylamino group and the like; and examples of the alkoxycarbonylamino group include a methoxycarbonylamino group, an ethoxycarbonylamino group, an n-propoxycarbonylamino group, an i-propoxycarbonylamino group, an n-butoxycarbonylamino group, an i-butoxycarbonylamino group, an s-butoxycarbonylamino group, a t-butoxycarbonylamino group and the like. Examples of the halogen atom include a fluorine atom, a chlorine atom, a bromine atom and an iodine atom; examples of the C₁₋₄ alkyl group include a methyl group, a trifluoromethyl group, a monochloromethyl group, an ethyl group, a pentafluoroethyl group, an n-propyl group, an i-propyl group, an n-butyl group, an i-butyl group, an s-butyl group and a t-butyl group; examples of the C₁₋₄ alkoxy group include a methoxy group, an ethoxy group, an n-propoxy group, an i-propoxy group, an n-butoxy group, an i-butoxy group, an s-butoxy group and a t-butoxy group; examples of the C₂₋₅ alkylcarbonyl group include a methylcarbonyl group, a trifluoromethylcarbonyl group, a monochloromethylcarbonyl group, an ethylcarbonyl group, a pentafluoroethylcarbonyl group, an n-propylcarbonyl group, an i-propylcarbonyl group, an n-butylcarbonyl group, an i-butylcarbonyl group, an s-butylcarbonyl group, a t-butylearbonyt group and the like; examples of the C₇₋₁₃ arylcarbonyl group include a phenylcarbonyl group, an o-toluoyl group, an m-toluoyl group, a p-toluoyl group, an o-biphenylylcarbonyl group, an m-biphenylylcarbonyl group, a p-biphenylylcarbonyl group, a 1-naphtylcarbonyl group, a 2-naphtylcarbonyl group and the like; examples of the C₁₋₄ alkylsulfinyl group include a methylsulfinyl group, an ethylsulfinyl group, an n-propylsulfinyl group, an i-propylsulfinyl group, an n-butylsulfinyl group, an i-butylsulfinyl group, an s-butylsulfinyl group, a t-butylsulfinyl group and the like; examples of the C₆₋₇ arylsulfinyl group include a benzenesulfinyl group, an o-toluenesulfinyl group, an m-toluenesulfinyl group, a p-toluenesulfinyl group and the like; examples of the C₁₋₄ alkylsulfonyl group include a methylsulfonyl group, an ethylsulfonyl group, an n-propylsulfonyl group, an i-propylsulfonyl group, an n-butylsulfonyl group, an i-butylsulfonyl group, an s-butylsulfonyl group, a t-butylsulfonyl group and the like; examples of the C₁₋₃ monoalkylaminosulfonyl group include a methylaminosulfonyl group, an ethylaminosulfonyl group, an n-propylaminosulfonyl group, an i-propylaminosulfonyl group and the like; and examples of the C₂₋₆ dialkylaminosulfonyl group include a dimethylaminosulfonyl group, a diethylaminosulfonyl group, a di-n-propylaminosulfonyl group, a di-i-propylaminosulfonyl group and the like.

Each of R⁵, R⁶, R⁷ and R⁸ is preferably independently an amino group, a tosylamino group, a benzylamino group, an acetylamino group, a propionylamino group, a benzoylamino group, a methoxycarbonylamino group, an ethoxycarbonylamino group, an n-propoxycarbonylamino group, an i-propoxycarbonylamino group, a fluorine atom, a chlorine atom, bromine atom, an iodine atom, a methyl group, a trifluoromethyl group, a monochloromethyl group, an ethyl group, a pentafluoroethyl group, an n-propyl group, an i-propyl group, an n-butyl group, an i-butyl group, an s-butyl group, a t-butyl group, a methoxy group, an ethoxy group, an n-propoxy group, an i-propoxy group, an n-butoxy group, an i-butoxy group, an s-butoxy group, a t-butoxy group, a methylcarbonyl group, a trifluoromethylcarbonyl group, a monochloromethylcarbonyl group, an ethylcarbonyl group, a pentafluoroethylcarbonyl group, an n-propylcarbonyl group, an i-propylcarbonyl group, an n-butylcarbonyl group, an i-butylcarbonyl group, an s-butylcarbonyl group, a t-butylcarbonyl group, a phenylcarbonyl group, an o-toluoyl group, an m-toluoyl group, a p-toluoyl group, an o-biphenylylcarbonyl group, an m-biphenylylcarbonyl group, a p-biphenylylcarbonyl group, a 1-naphtylcarbonyl group, a 2-naphtylcarbonyl group, a methylsulfinyl group, an ethylsulfinyl group, an n-propylsulfinyl group, an i-propylsulfinyl group, an n-butylsulfinyl group, an i-butylsulfinyl group, an s-butylsulfinyl group, a t-butylsulfinyl group, a benzenesulfinyl group, an o-toluenesulfinyl group, an m-toluenesulfinyl group, a p-toluenesulfinyl group, a methylsulfonyl group, an ethylsulfonyl group, an n-propylsulfonyl group, an i-propylsulfonyl group, an n-butylsulfonyl group, an i-butylsulfonyl group, an s-butylsulfonyl group, a t-butylsulfonyl group, a methylaminosulfonyl group, an ethylaminosulfonyl group, an n-propylaminosulfonyl group, an i-propylaminosulfonyl group, a dimethylaminosulfonyl group, a diethylaminosulfonyl group, a di-n-propylaminosulfonyl group or a di-i-propylaminosulfonyl group.

R⁹ is a hydrogen atom, a C₁₋₄ alkyl group or a C₁₋₄ alkoxy group.
R⁹ will be specifically described. Examples of the C₁₋₄ alkyl group include a methyl group, a trifluoromethyl group, a monochloromethyl group, an ethyl group, a pentafluoroethyl group, an n-propyl group, an i-propyl group, an n-butyl group, an i-butyl group, an s-butyl group and a t-butyl group; and examples of the C₁₋₄ alkoxy group include a methoxy group, an ethoxy group, an n-propoxy group, an i-propoxy group, an n-butoxy group, an i-butoxy group, an s-butoxy group and a t-butoxy group.
R⁹ is preferably a hydrogen atom, a methyl group, a trifluoromethyl group, a monochloromethyl group, an ethyl group, a pentafluoroethyl group, an n-propyl group, an i-propyl group, an n-butyl group, an i-butyl group, an s-butyl group, a t-butyl group, a methoxy group, an ethoxy group, an n-propoxy group, an i-propoxy group, an n-butoxy group, an i-butoxy group, an s-butoxy group or a t-butoxy group.

R¹⁰ in Formula (5), Formula (6), Formula (9) and Formula (10) is a hydrogen atom, a C₁₋₂₂ alkyl group, a C₁₋₄ alkoxy group or a C₆₋₁₀ aryl group (the aryl group is unsubstituted or substituted with a halogen atom, a C₁₋₄ alkyl group or a C₁₋₄ alkoxy group), R¹¹ in Formula (6) is a C₁₋₄ alkyl group (the alkyl group is unsubstituted or substituted with a halogen atom), and each R¹⁰ in Formula (7) and Formula (8) is independently a hydrogen atom, a C₁₋₂₂ alkyl group, a C₁₋₄ alkoxy group or a C₆₋₁₀ aryl group (the aryl group is unsubstituted or substituted with a halogen atom, a C₁₋₄ alkyl group ar a C₁₋₄ alkoxy group). R¹¹ in Formula (6) is a C₁₋₄ alkyl group (the alkyl group is unsubstituted or substituted with a halogen atom). However, R⁹ and R¹⁰ in Formula (5), Formula (6), Formula (8) and Formula (9) may bind each other to form the bivalent group represented by any one of Formula (10), Formula (11), Formula (12) and Formula (13) (each R¹² in Formula (11), Formula (12), Formula (13) and Formula (14) is independently a hydrogen atom or a C₁₋₆ alkyl group).

R¹⁰ will be specifically described. Examples of the C₁₋₂₂ alkyl group include a methyl group, an ethyl group, an n-propyl group, an i-propyl group, an n-butyl group, an i-butyl group, an s-butyl group, a t-butyl group, a pentyl group, an isopentyl group, a neopentyl group, a hexyl group, a heptyl group, an octyl group, a 2-ethylhexyl group, a nonyl group, a decyl group, an undecyl group, a dodecyl group, a tridecyl group, an isotridecyl group, a myristyl group, a palmityl group, a stearyl group, an icosyl group, a docosyl group and the like; examples of the C₁₋₄ alkoxy group include a methoxy group, an ethoxy group, an n-propoxy group, an i-propoxy group, an n-butoxy group, an i-butoxy group, an s-butoxy group, a t-butoxy group and the like; and examples of the C₆₋₁₀ aryl group include a phenyl group, an o-fluorophenyl group, an m-fluorophenyl group, a p-fluorophenyl group, an o-chlorophenyl group, an m-chlorophenyl group, a p-chlorophenyl group, an o-bromophenyl group, an m-bromophenyl group, a p-bromophenyl group, an o-tolyl group, an m-tolyl group, a p-tolyl group, an o-ethylphenyl group, an m-ethylphenyl group, a p-ethylphenyl group, an o-(t-butyl)phenyl group, an m-(t-butyl)phenyl group, a p-(t-butyl)phenyl group, a 3,5-dimethylphenyl group, an o-methoxyphenyl group, an m-thoxyphenyl group, a p-methoxyphenyl group and the like.

Each R¹⁰ is preferably independently a hydrogen atom, a methyl group, an ethyl group, an n-propyl group, an i-propyl group, an n-butyl group, an i-butyl group, an s-butyl group, a t-butyl group, a pentyl group, an isopentyl group, a neopentyl group, a hexyl group, a heptyl group, an octyl group, a 2-ethylhexyl group, a nonyl group, a decyl group, an undecyl group, a dodecyl group, a tridecyl group, an isotridecyl group, a myristyl group, a palmityl group, a stearyl group, an icosyl group, a docosyl group, a methoxy group, an ethoxy group, an n-propoxy group, an i-propoxy group, an n-butoxy group, an i-butoxy group, an s-butoxy group, a t-butoxy group, a phenyl group, an o-fluorophenyl group, an m-fluorophenyl group, a p-fluorophenyl group, an o-chlorophenyl group, an m-chlorophenyl group, a p-chlorophenyl group, an o-bromophenyl group, an m-bromophenyl group, a p-bromophenyl group, an o-tolyl group, an m-tolyl group, a p-tolyl group, an o-ethylphenyl group, an m-ethylphenyl group, a p-ethylphenyl group, an o-(t-butyl)phenyl group, an m-(t-butyl)phenyl group, a p-(t-butyl)phenyl group, a 3,5-dimethylphenyl group, an o-methoxyphenyl group, an m-methoxyphenyl group or a p-methoxyphenyl group.

R¹¹ is a C₁₋₄ alkyl group (the alkyl group is unsubstituted or substituted with a halogen atom).
R¹¹ will be specifically described. Examples of the C₁₋₂₂ alkyl group include a methyl group, an ethyl group, an n-propyl group, an i-propyl group, an n-butyl group, an i-butyl group, an s-butyl group, a t-butyl group and the like.
R¹¹ is preferably a methyl group.

Each R¹² is independently a hydrogen atom or a C₁₋₆ alkyl group.
R¹² will be specifically described. Examples of the alkyl group include a methyl group, an ethyl group, an n-propyl group, an i-propyl group, an n-butyl group, an i-butyl group, an s-butyl group, a t-butyl group and the like.
Each R¹² is preferably independently a hydrogen atom, a methyl group or an ethyl group.

R⁹ and R¹⁰ in Formula (5), Formula (6), Formula (9) and Formula (10) may bind each other to form a bivalent group, and when R⁹ and R¹⁰ bind each other to form the group represented by Formula (11), a 6-membered ring is formed, so that examples of the compound include benzopyran, derivatives thereof and the like. Furthermore, when R⁸ and R⁹ bind each other to form the group represented by Formula (12), a 5-membered ring is formed, so that examples of the compound includes indene, derivatives thereof and the like. In addition, when R⁸ and R⁹ bind each other to form the group represented by Formula (13), a 6-membered ring is formed, so that examples of the compound include 1,2-dihydronaphthalene, derivatives thereof and the like. Moreover, when R⁸ and R⁹ bind each other to form the group represented by Formula (14), a 7-membered ring is formed, so that examples of the compound include 1,2-benzo-1,3-cycloheptadiene, derivatives thereof and the like.

The partial ring structure A in Formula (9) and Formula (10) will be described. The partial ring structure A means the partial structure represented by the 5-, 6- or 7-membered ring fused with a benzene ring (each of the 5-, 6- and 7-membered rings is unsubstituted or substituted with h pieces of R¹³ (R¹³ is a halogen atom, a hydroxyl group, a C₁₋₆ alkyl group (the alkyl group is unsubstituted or substituted with a halogen atom, a hydroxyl group, a cyano group, an amino group, a nitro group, a C₁₋₄ alkoxy group, a C₁₋₄ alkylcarbonyloxy group, a C₁₋₄ alkylcarbonylamino group or a C₁₋₄ alkoxycarbonyl group (the alkoxy group is unsubstituted or the alkylcarbonyloxy group, the alkylcarbonylamino group or the alkoxycarbonyl group is substituted with a halogen atom)), a C₁₋₆ alkoxy group (the alkoxy group is unsubstituted or substituted with a halogen atom, a hydroxyl group, a cyano group, an amino group, a nitro group, a C₁₋₄ alkoxy group, a C₁₋₄ alkylcarbonyloxy group, a C₁₋₄ alkylcarbonylamino group or a C₁₋₄ alkoxycarbonyl group (the alkoxy group, the alkylcarbonyloxy group, the alkylcarbonylanino group and the alkoxycarbonyl group are unsubstituted or substituted with a halogen atom)), a nitro group, a cyano group, a formyl group, a formamide group, a carbamoyl group, a sulfo group, a sulfoamino group, a sulfamoyl group, a sulfonyl group, an amino group, a carboxyl group, a C₁₋₆ alkylamino group, a di-C₁₋₆ alkylamino group, a C₁₋₆ alkylcarbonylamino group, a C₁₋₆ alkylsulfonamide group, a C₆₋₁₄ arylsulfonamide group, a C₁₋₆ alkylaminocarbonyl group, a di-C₁₋₆ alkylaminocarbonyl group, a C₁₋₆ alkylcarbonyl group, a C₁₋₆ alkoxycarbonyl group, a C₁₋₆ alkyl sulfonyl group, a C₁₋₁₄ arylsulfonyl group or a C₆₋₁₄ arylcarbonyl group (the alkylamino group, the dialkylamino group, the alkylcarbonylamino group, the alkylsulfonamide group, the arylsulfonamide group, the alkylaminocarbonyl group, the dialkylaminocarbonyl group, the alkylcarbonyl group, the alkoxycarbonyl group, the alkylsulfonyl group, the arylsulfonyl group and the arylcarbonyl group are unsubstituted or substituted with a halogen atom), h means an integer from 1 to 6, and when h is 2 to 6, each R¹³ may be the same or different), may contain 1 to 3 atoms of an oxygen atom, a nitrogen atom or a sulfur atom singly or in combination as a ring constituent atom, the number of unsaturated bonds in the ring is 1, 2 or 3 including the unsaturated bond in the fused benzene ring, and the carbon atom constituting the ring may be carbonyl or thiocarbonyl).

The above-nientioned R¹³ will be specifically described.
Examples of the halogen atom include a fluorine atom, a chlorine atom, a bromine atom and an iodine atom; examples of the C₁₋₆ alkyl group include a methyl group, a trifluoromethyl group, an ethyl group, an n-propyl group, an i-propyl group, an n-butyl group, an i-butyl group, an s-butyl group, a t-butyl group, a 1-pentyl group, a 2-pentyl group, a 3-pentyl group, an i-pentyl group, a neopentyl group, a 2,2-dimethylpropyl group, a 1-hexyl group, a 2-hexyl group, a 3-hexyl group, a 1-methyl-n-pentyl group, a 1,1,2-trimethyl-n-propyl group, a 1,2,2-trimethyl-n-propyl group, a 3,3-dimethyl-n-butyl group, a methylcarbonyloxymethyl group, an ethylcarbonyloxymethyl group, a methylcarbonyloxyethyl group, an ethylcarbonyloxyethyl group, a methylcarbonylaminomethyl group, a trifluoromethylcarbonylaminomethyl group, an ethylcarbonylaminomethyl group, a methylcarbonylaminoethyl group, an ethylcarbonylaminoethyl group, a methoxycarbonylmethyl group, a trifluoromethoxycarbonylmethyl group, an ethoxycarbonylmethyl group, a methoxycarbonylethyl group, an ethoxycarbonylethyl group and the like; examples of the C₁₋₆ alkoxy group include a methoxy group, a trifluoromethoxy group, an ethoxy group, an n-propoxy group, an i-propoxy group, an n-butoxy group, an i-butoxy group, an s-butoxy group, a t-butoxy group, a 1-pentyloxy group, a 2-pentyloxy group, a 3-pentyloxy group, an i-pentyloxy group, a neopentyloxy group, a 2,2-dimethylpropoxy group, a 1-hexyloxy group, a 2-hexyloxy group, a 3-hexyloxy group, a 1-methyl-n-pentyloxy group, a 1,1,2-trimethyl-n-propoxy group, a 1,2,2-trimethyl-n-propoxy group, a 3,3-dimethyl-n-butoxy group, a methylcarbonyloxymethoxy group, an ethylcarbonyloxymethoxy group, a methylcarbonyloxyethoxy group, an ethylcarbonyloxyethoxy group, a methylcarbonylaminomethoxy group, a trifluoromethylcarbonylaminomethoxy group, an ethylcarbonylaminomethoxy group, a methylcarbonylaminoethoxy group, an ethylcarbonylaminoethoxy group, a methoxycarbonylmethoxy group, a trifluoromethoxycarbonylmethoxy group, an ethoxycarbonylmethoxy group, a methoxycarbonylethoxy group, an ethoxycarbonylethoxy group and the like; examples of the C₁₋₆ alkylamino group include a methylamino group, a trifluoromethylamino group, an ethylamino group, an n-propylamino group, an i-propylamino group, a c-propylamino group, an n-butylamino group, an i-butylamino group, an s-butylamino group, a t-butylamino group, a c-butylamino group, a 1-pentylamino group, a 2-pentylamino group, a 3-pentylamino group, an i-pentylamino group, a neopentylamino group, a t-pentylamino group, a c-pentylamino group, a 1-hexylamino group, a 2-hexylamino group, a 3-hexylamino group, a e-hexylamino group, a 1-methyl-n-pentylamino group, a 1,1,2-trimethyl-n-propylamino group, a 1,2,2-trimethyl-n-propylamino group, a 3,3-dimethyl-n-butylamino group and the like; examples of the di-C₁₋₆ alkylamino group include a dimethylamino group, a di-(trifluoromethyl)amino group, a diethylamino group, a di-n-propylamino group, a di-i-propylamino group, a di-c-propylamino group, a di-n-butylamino group, a di-i-butylamino group, a di-s-butylamino group, a di-t-butylamino group, a di-c-butylamino group, a di-1-pentylamino group, a di-2-pentylamino group, a di-3-pentylamino group, a di-i-pentylamino group, a di-neopentylamino group, a di-t-pentylamino group, a di-c-pentylamino group, a di-1-hexylamino group, a di-2-hexylamino group, a di-3-hexylamino group, a di-c-hexylamino group, a di-(1-methyl-n-pentyl)amino group, a di-(1,1,2-trimethyl-n-propyl)amino group, a di-(1,2,2-trimethyl-n-propyl)amino group, a di-(3,3-dimethyl-n-butyl)amino group, a methyl(ethyl)amino group, a methyl(n-propyl)amino group, a methyl(i-propyl)amino group, a methyl(c-propyl)amino group, a methyl(n-butyl)amino group, a methyl(i-butyl)amino group, a methyl(s-butyl)amino group, a methyl(t-butyl)amino group, a methyl(c-butyl)amino group, an ethyl(n-propyl)amino group, an ethyl(i-propyl)amino group, an ethyl(c-propyl)amino group, an ethyl(n-butyl)amino group, an ethyl(i-butyl)amino group, an ethyl(s-butyl)amino group, an ethyl(t-butyl)amino group, an ethyl(c-butyl)amino group, an n-propyl(i-propyl)amino group, an n-propyl(c-propyl)amino group, an n-propyl(n-butyl)amino group, an n-propy)(i-butyl)amino group, an n-propyl(s-butyl)amino group, an n-propyl(t-butyl)amino group, an n-propyl(c-butyl)amino group, an i-propyl(c-propyl)amino group, an i-propyl(n-butyl)amino group, an i-propyl(i-butyl)amino group, an i-propyl(s-butyl)amino group, an i-propyl(t-butyi)amino group, an i-propyl(c-butyl)amino group, a c-propyl(n-butyl)amino group, a c-propyl(i-butyl)amino group, a c-propyl(s-butyl)amino group, a c-propyl(t-butyl)amino group, a c-propyl(c-butyl)amino group, an n-butyl(i-butyl)amino group, an n-butyl(s-butyl)amino group, an n-butyl(t-butyl)amino group, an n-butyl(c-butyl)amino group, an i-butyl(s-butyl)amino group, an i-butyl(t-butyl)amino group, an i-butyl(c-butyl)amino group, an s-butyl(t-butyl)amino group, an s-butyl(c-butyl)amino group, a t-butyl(c-butyl)amino group and the like; examples of the C₁₋₆ alkylcarbonylamino group include a methylcarbonylamino group, a trifluoromethylcarbonylamino group, an ethylcarbonylamino group, an n-prapylcarbonylamino group, an i-propylcarbonylamino group, an n-butylcarbonylamino group, an i-butylearbonylamino group, an s-butylcarbonylamino group, a t-butylcarbonylamino group, a 1-pentylcarbonylamino group, a 2-pentylcarbonylamino group, a 3-pentylcarbonylamino group, an i-pentylcarbonylamino group, neopentylcarbonylamino, a t-pentylcarbonylamino group, a 1-hexylcarbonylamino group, a 2-hexylcarbonylamino group, a 3-hexylcarbonylamino group and the like; examples of the C₁₋₆ alkylsulfonamide group include a methanesulfonamide group, a trifluoromethanesulfonamide group, an ethanesulfonamide group, an n-propanesulfonamide group, an i-propanesulfonamide group, an n-butanesulfonamide group, an i-butanesulfonamide group, an s-butanesulfonamide group, a t-butanesulfonamide group, a 1-pentanesulfonamide group, a 2-pentanesulfonamide group, a 3-pentanesulfonamide group, an i-pentanesulfonamide group, a neopentanesulfonamide group, a t-pentanesulfonamide group, a 1-hexanesulfonamide group, a 2-hexanesulfonamide group, a 3-hexanesulfonamide group and the like; examples of the C₆₋₁₄ arylsulfonamide group include a benzenesulfonamide group, a p-toluenesulfonamide group, an o-biphenylsulfonamide group, an m-biphenylsulfonamide group, a p-biphenylsulfonamide group, a 1-naphthalenesulfonamide group, a 2-naphthalenesulfonamide group, a 1-anthracenesulfonamide group, a 2-anthracenesulfonamide group, a 9-anthracenesulfonamide group, a 1-phenanthrenesulfonamide group, a 2-phenanthrenesulfonamide group, a 3-phenanthrenesulfonamide group, a 4-phenanthrenesulfonamide group, a 9-phenanthrenesulfonamide group and the like; examples of the C₁₋₆ alkylaminocarbonyl group include a methylaminocarbonyl group, a trifluoromethylaminocarbonyl group, an ethylaminocarbonyl group, an n-propylaminocarbonyl group, an i-propylaminocarbonyl group, an n-butylaminocarbonyl group, an i-butylaminocarbonyl group, an s-butylaminocarbonyl group, a t-butylaminocarbonyl group, a 1-pentylaminocarbonyl group, a 2-pentylaminocarbonyl group, a 3-pentylaminocarbonyl group, an i-pentylaminocarbonyl group, neopentylaminocarbonyl, a t-pentylaminocarbonyl group, a I-hexylaminacarbanyl group, a 2-hexylaminocarbonyl group, a 3-hexylaminocarbonyl group and the like; examples of the di-C₁₋₆ alkylaminocarbonyl group include a dimethylaminocarbonyl group, a di(trifluoromethyl)aminocarbonyl group, a dicthylaminocarbonyl group, a di-n-propylaminocarbonyl group, a di-i-propylaminocarbonyl group, a di-c-propylaminocarbonyl group, a di-n-butylaminocarbonyl group, a di-i-butylaminocarbonyl group, a di-s-butylaminocarbonyl group, a di-t-butylaminocarbonyl group, a di-c-butylaminocarbonyl group, a di-1-pentylaminocarbonyl group, a di-2-pentylaminocarbonyl group, a di-3-pentylaminocarbonyl group, a di-i-pentylaminocarbonyl group, a di-neopentylaminocarbonyl group, a di-t-pentylaminocarbonyl group, a di-c-pentylaminoearbonyl group, a di-1-hexylaminocarbonyl group, a di-2-hexylaminocarbonyl group, a di-3-hexylaminocarbonyl group, a di-c-hexylaminocarbonyl group, a di-(1-methyl-n-pentyl)aminocarbonyl group, a di-(1,1,2-trimethyl-n-propyl)aminocarbonyl group, a di-(1,2,2-trimethyl-n-propyl)aminocarbonyl group, a di-(3,3-dimethyl-n-butyl)aminocarbonyl group, a methyl(ethyl)aminocarbonyl group, a trifluoromethyl(ethyl)aminocarbonyl group, a methyl(n-propyl)amincarbonyl group, a methyl(i-propyl)aminocarbonyl group, a methyl(c-propyl)aminocarbonyl group, a methyl(n-butyl)aminocarbonyl group, a methyl(i-butyl)aminocarbonyl group, a anethyl(s-butyl)aminocarbonyl group, a methyl(t-butyl)aminocarbonyl group, a methyl(c-butyl)aminocarbonyl group, an ethyl(n-propyl)aminocarbonyl group, an ethyl(i-propyl)aminocarbonyl group, an ethyl(c-propyl)aminocarbonyl group, an ethyl(n-butyl)aminocarbonyl group, an ethyl(i-butyl)aminocarbonyl group, an ethyl(s-butyl)aminocarbanyl group, an ethyl(t-butyl)aminocarbonyl group, an ethyl(c-butyo)aminocarbonyl group, an n-propyl(i-propyl)aminocarbonyl group, an n-propyl(c-propyl)aminocarbonyl group, an n-propyl(n-butyl)aminocarbonyl group, an n-propyl(i-butyl)aminocarbonyl group, an n-propyl(s-butyl)aminocarbonyl group, an n-propyl(t-butyl)aminocarbonyl group, an n-propyl(c-butyl)aminocarbonyl group, an i-propyl(c-propyl)aminocarbonyl group, an i-propyl(n-butyl)aminocarbonyl group, an i-propyl(i-butyl)aminocarbonyl group, an i-propyl(s-butyl)aminocarbonyl group, an i-propyl(t-butyl)aminocarbonyl group, an i-propyl(c-butyl)aminocarbonyl group, a c-propyl(n-butyl)aminocarbonyl group, a c-propyl(i-butyl)aminocarbonyl group, a c-propyl(s-butyl)aminocarbonyl group, a c-propyl(t-butyl)aminocarbonyl group, a c-propyl(c-butyl)aminocarbonyl group, an n-butyl(i-butyl)aminocarbonyl group, an n-butyl(s-butyl)aminocarbonyl group, an n-butyl(t-butyl)aminocarbonyl group, an n-butyl(e-butyl)aminocarbonyl group, an i-butyl(s-butyl)aminocarbonyl group, an i-butyl(t-butyl)aminocarbonyl group, an i-butyl(c-butyl)aminocarbonyl group, an s-butyl(t-butyl)aminocarbonyl group, an s-butyl(c-butyl)aminocarbonyl group, a t-butyl(c-butyl)aminocarbonyl group and the like; examples of the C₁₋₆ alkylcarbonyl group include a methylcarbonyl group, a trifluoromethylcarbonyl group, an ethylcarbonyl group, an n-propylcarbonyl group, an i-propylcarbonyl group, an n-butylcarbonyl group, an i-butylcarbonyl group, an s-butylcarbonyl group, a t-butylcarbonyl group, a 1-pentylcarbonyl group, a 2-pentylcarbonyl group, a 3-pentylcarbonyl group, an i-pentylcarbonyl group, a neopentylcarbonyl group, a t-pentylcarbonyl group, a 1-hexylcarbonyl group, a 2-hexylcarbonyl group, a 3-hexylcarbonyl group and the like; examples of the C₁₋₆ alkoxycarbonyl group include a methoxycarbonyl group, a trifluoromethoxycarbonyl group, an ethoxycarbonyl group, an n-propoxycarbonyl group, an i-propoxycarbonyl group, an n-butoxycarbonyl group, an i-butoxycarbonyl group, an s-butoxycarbonyl group, a t-butoxycarbonyl group, a 1-pentyloxycarbonyl group, a 2-pentyloxycarbonyl group, a 3-pentyloxycarbonyl group, an i-pentyloxycarbonyl group, a neopentyloxycarbonyl group, a t-pentyloxycarbonyl group, a 1-hexyloxycarbonyl group, a 2-hexyloxycarbonyl group, a 3-hexyloxycarbonyl group and the like; examples of the C₁₋₆ alkylsulfonyl group include a methanesulfonyl group, a trifluoromethanesulfonyl group, an ethanesulfonyl group, an n-propanesulfonyl group, an n-butanesulfonyl group and the like; examples of the C₆₋₁₄ arylsulfonyl group include a benzenesulfonyl group, a p-fluorobenzenesulfonyl group, a p-toluenesulfonyl group, an o-biphenylsulfonyl group, an m-biphenylsulfonyl group, a p-biphenylsulfonyl group, a i-naphthalenesulfonyl group, a 2-naphthalenesulfonyl group, a 1-anthracenesulfonyl group, a 2-anthracenesulfonyl group, a 9-anthracenesulfonyl group, a 1-phenanthrenesulfonyl group, a 2-phenanthrenesulfonyl group, a 3-phenanthrenesulfonyl group, a 4-phenanthrenesulfonyl group, a 9-phenanthrenesulfonyl group and the like; and examples of the C₆₋₁₄ arylcarbonyl group include a phenylcarbonyl group, a p-fluorophenylcarbonyl group, an o-biphenylylcarbonyl group, an m-biphenylylcarbonyl group, a p-biphenylylcarbonyl group, a 1-naphtylcarbonyl group, a 2-naphtylcarbonyl group, a 1-anthrylcarbonyl group, a 2-anthrylcarbonyl group, a 9-anthrylcarbonyl group, a 1-phenanthrylcarbonyl group, a 2-phenanthrylcarbonyl group, a 3-phenanthrylcarbonyl group, a 4-phenanthrylcarbonyl group, a 9-phenanthrylcarbonyl group and the like.

Preferred atoms and substituents of the above-mentioned R¹³ will be specifically described. R¹³ is preferably a fluorine atom, a chlorine atom, a bromine atom, a methyl group, a trifluoromethyl group, an ethyl group, an n-propyl group, an i-propyl group, an n-butyl group, an n-pentyl group, an i-pentyl group, a 3,3-dimethyl-n-butyl group, a methylcarbonyloxymethyl group, an ethylcarbonyloxymethyl group, a methylcarbonyloxyethyl group, an ethylcarbonyloxyethyl group, a methylcarbonylaminomethyl group, a trifluoromethylcarbonylaminomethyl group, an ethylcarbonylaminomethyl group, a methylcarbonylaminoethyl group, an ethylcarbonylaminoethyl group, a methoxycarbonylmethyl group, a trifluoromethoxycarbonylmethyl group, an ethoxycarbonylmethyl group, a methoxycarbonylethyl group, an ethoxycarbonylethyl group, a methoxy group, a trifluoromethoxy group, an ethoxy group, an n-propoxy group, an i-propoxy group, a 3,3-dimethyl-n-butoxy group, a methylcarbonyloxymethoxy group, an ethylcarbonyloxymethoxy group, a methylcarbonyloxyethoxy group, an ethylcarbonyloxyethoxy group, a methylcarbonylaminomethoxy group, a trifluoromethylcarbonylaminomethoxy group, an ethylcarbonylaminomethoxy group, a methylcarbonylaminoethoxy group, an ethylcarbonylaminoethoxy group, a methoxycarbonylmethoxy group, a trifluoromethoxycarbonylmethoxy group, an ethoxycarbonylmethoxy group, a methoxycarbonylethoxy group, an ethoxycarbonylethoxy group, a methylamino group, a trifluoromethylamino group, an ethylamino group, an n-propylamino group, an i-propylamino group, an n-butylamino group, a dimethylamino group, a di-(trifluoromethyl)amino group, a diethylamino group, a di-n-propylamino group, a di-i-propylamino group, a di-n-butylamino group, a methylcarbonylamino group, a trifluoromethylcarbonylamino group, an ethylcarbonylamino group, an n-propylcarbonylamino group, an i-propylcarbonylamino group, an n-butylcarbonylamino group, a methanesulfonamide group, a trifluoromethanesulfonamide group, an ethanesulfonamide group, an n-propanesulfonamide group, an i-propanesulfonamide group, an n-butanesulfonamide group, a benzenesulfonamide group, a p-toluenesulfonamide group, a methylaminocarbonyl group, a trifluoromethylaminocarbonyl group, an ethylaminocarbonyl group, an n-propylaminocarbonyl group, an i-propylaminocarbonyl group, an n-butylaminocarbonyl group, a dimethylaminocarbonyl group, a di(trifluoromethyl)aminocarbonyl group, a diethylaminocarbonyl group, di-n-propylaminocarbonyl, a di-i-propylaminocarbonyl group, a di-c-propylaminocarbonyl group, a di-n-butylaminocarbonyl group, a methyl(ethyl)aminocarbonyl group, a trifluoromethyl(ethyl)aminocarbonyl group, a methylcarbonyl group, a trifluoromethylcarbonyl group, an ethylcarbonyl group, an n-propylcarbonyl group, an i-propylcarbonyl group, an n-butylcarbonyl group, a methoxycarbonyl group, a trifluoromethoxycarbonyl group, an ethoxycarbonyl group, an n-propoxycarbonyl group, an i-propoxycarbonyl group, an n-butoxycarbonyl group, an i-butoxycarbonyl group, an s-butoxycarbonyl group, a t-butoxycarbonyl group, a methanesulfonyl group, a trifluoromethanesulfonyl group, an ethanesulfonyl group, a benzenesulfonyl group, an o-biphenylsulfonyl group, an m-biphenylsulfonyl group, a p-biphenylsulfonyl group, a 1-naphthalenesulfonyl group, a 2-naphthalenesulfonyl group, a phenylcarbonyl group, an o-biphenylylcarbonyl group, an m-biphenylylcarbonyl group, a p-biphenylylcarbonyl group, a 1-naphtylcarbonyl group, a 2-naphtylcarbonyl group, a hydroxyl group, a nitro group, a cyano group, a formyl group, a formamide group, a carbamoyl group, a sulfoamino group, a sulfamoyl group, an amino group or a carboxyl group.

Regarding the case in which the partial ring structure A in Formula (9) and Formula (10) is represented by any one of Formula (a), Formula (b), Formula (c), Formula (d), Formula (e), Formula (f), Formula (g), Formula (h), Formula (i), Formula (j), Formula (k), Formula (l), Formula (m), Formula (n), Formula (o), Formula (p), Formula (q), Formula (r), Formula (s), Formula (t), Formula (u), Formula (v), Formula (w), Formula (x), Formula (y), Formula (z), Formula (aa), Formula (ab), Formula (ac), Formula (ad), Formula (ae), Formula (af), Formula (ag) and Formula (ah): 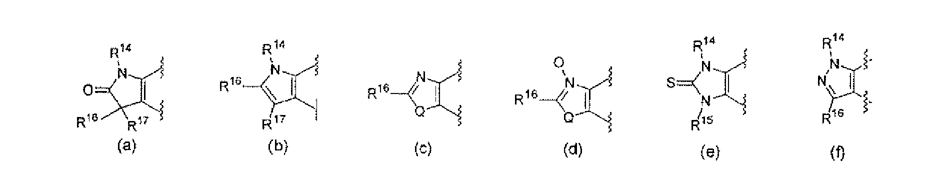 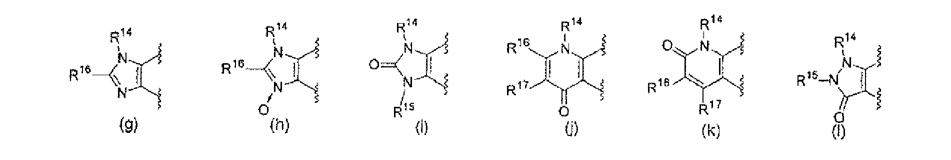 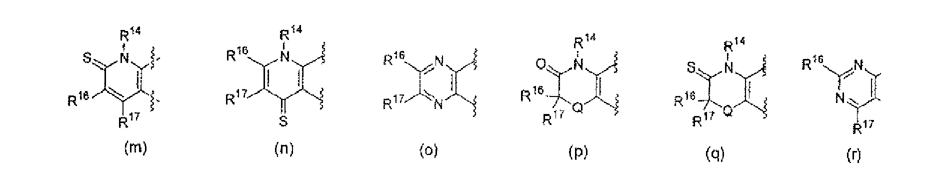 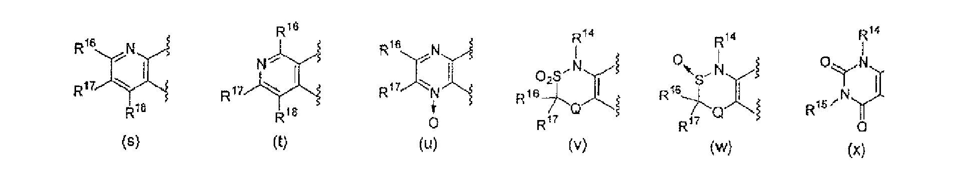 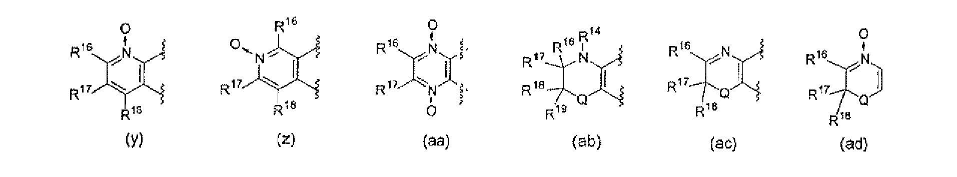 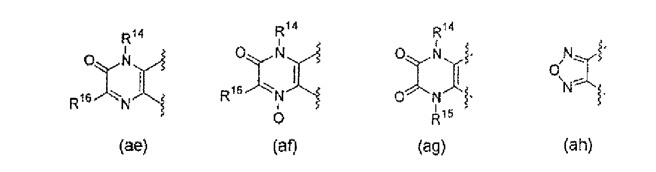 R¹⁴, R¹⁵, R¹⁶, R¹⁷, R¹⁸ and R¹⁹ in Formula (a), Formula (b), Formula (c), Formula (d), Formula (e), Formula (f), Formula (g), Formula (h), Formula (i), Formula (j), Formula (k), Formula (l), Formula (m), Formula (n), Formula (o), Formula (p), Formula (q), Formula (r), Formula (s), Formula (t), Formula (u), Formula (v), Formula (w), Formula (x), Formula (y), Formula (z), Formula (aa), Formula (ab), Formula (ac), Formula (ad), Formula (ae), Formula (af), Formula (ag) and Formula (ah) will be described.

R¹⁴ and R¹⁵ in Formula (a), Formula (b), Formula (e), Formula (f), Formula (g), Formula (h), Formula (i), Formula (j), Formula (k), Formula (I), Formula (m), Formula (n), Formula (p), Formula (q), Formula (v), Formula (w), Formula (x), Formula (ab), Formula (ae), Formula (af) and Formula (ag) will be described. Each of R¹⁴ and R¹⁵ in Formula (a), Formula (b), Formula (e), Formula (f), Formula (g), Formula (h), Formula (i), Formula (j), Formula (k), Formula (1), Formula (m), Formula (n), Formula (p), Formula (q), Formula (v), Formula (w), Formula (x), Formula (ab), Formula (ae), Formula (af) and Formula (ag) is independently a hydrogen atom, a C₁₋₆ alkyl group (the alkyl group is unsubstituted or substituted with a halogen atom, a C₁₋₆ alkoxy group (the alkoxy group is unsubstituted or substituted with a halogen atom), an amino group, a hydroxyl group, a C₆₋₁₄ aryl group, a C₂₋₉ heteroaryl group (each of the aryl group and the heteroaryl group is unsubstituted or substituted with q pieces of R²⁰ (R²⁰ means the same as R¹³, q represents an integer from 1 to 3, and when q is 2 or 3, R²⁰ may be the same or different)), a C₁₋₆ alkylaminocarbonyl group, a di-C₁₋₆ alkylaminocarbonyl group, a C₁₋₆ alkylcarbonyloxy group, a C₁₋₆ alkylcarbonyl group (the alkylcarbonyloxy group and the alkylcarbonyl group are unsubstituted or substituted with a halogen atom), a C₁₋₆ alkylcarbonylamino group, a C₃₋₈ cycloalkylcarbonyl group, a C₁₋₆ alkoxycarbonyl group, a C₁₋₆ alkylsulfonyl group (the cycloalkylcarbonyl group, the alkoxycarbonyl group and the alkylsulfonyl group are unsubstituted or substituted with a halogen atom), a carboxyl group, a C₆₋₁₄ arylcarbonyl group (the arylcarbonyl group is unsubstituted or substituted with a halogen atom) or a C₂₋₉ heteroarylcarbonyl group), a C₆₋₁₄ aryl group, a C₂₋₉ heteroaryl group (each of the aryl group and the heteroaryl group is unsubstituted or substituted with q pieces of R²⁰ (R²⁰ means the same as R¹³, q represents an integer from 1 to 3, and when q is 2 or 3, R²⁰ may be the same or different)), a C₁₋₆ alkylaminocarbonyl group, a di-C₁₋₆ alkylaminocarbonyl group, a C₁₋₆ alkylcarbonyl group, a C₃₋₈ cycloalkylcarbonyl group, a C₁₋₆ alkoxycarbonyl group, a C₁₋₆ alkylsulfonyl group, a C₆₋₁₄ arylsulfonyl group, a C₂₋₉ heteroarylsulfonyl group (each of the arylsulfonyl group and the heteroarylsulfonyl group is unsubstituted or substituted with q pieces of R²⁰ (R²⁰ means the same as R¹³, q represents an integer from 1 to 3, and when q represents 2 or 3, R²⁰ may be the same or different)), a carboxyl group, a C₆₋₁₄ arylcarbonyl group or a C₂₋₉ heteroarylcarbonyl group (each of the arylcarbonyl group and the heteroarylcarbonyl group is unsubstituted or substituted with q pieces of R²⁰ (R²⁰ means the same as R¹³, q represents an integer from 1 to 3, and when q is 2 or 3, R²⁰ may be the same or different)).

Each substituent of R¹⁴ and R¹⁵ in Formula (a), Formula (b), Formula (e), Formula (f), Formula (g), Formula (h), Formula (i), Formula (j), Formula (k), Formula (l), Formula (m), Formula (n), Formula (p), Formula (q), Formula (v), Formula (w), Formula (x), Formula (ab), Formula (ae), Formula (af) and Formula (ag) will be specifically described.
Examples of the C₁₋₆ alkyl group include a methyl group, a trifluoromethyl group, an ethyl group, an n-propyl group, an i-propyl group, an n-butyl group, an i-butyl group, an s-butyl group, a t-butyl group, an n-pentyl group, a 2-pentyl group, a 3-pentyl group, an i-pentyl group, a neopentyl group, a 2,2-dimethylpropyl group, an n-hexyl group, a 2-hexyl group, a 3-hexyl group, a 1-methyl-n-pentyl group, a 1,1,2-trimethyl-n-propyl group, a 1,2,2-trimethyl-n-propyl group, a 3,3-dimethyl-n-butyl group, a methylcarbonyloxymethyl group, an ethylcarbonyloxymethyl group, a methylcarbonyloxyethyl group, an ethylcarbonyloxyethyl group, a methylcarbonylaminomethyl group, a trifluoromethylcarbonylaminomethyl group, an ethylcarbonylaminomethyl group, a methylcarbonylaminoethyl group, an ethylcarbonylaminoethyl group, a methoxycarbonylmethyl group, an ethoxycarbonylmethyl group, a methoxycarbonylethyl group, an ethoxycarbonylethyl group and the like; and examples of the C₆₋₁₄ aryl group include a phenyl group, an o-biphenylyl group, an m-biphenylyl group, a p-biphenylyl group, a 1-naphtyl group, a 2-naphtyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group and the like.
Examples of the C₂₋₉ heteroaryl group include a 5- to 7-membered C₂₋₆ heteromonocyclic group which may contain 1 to 3 atoms of an oxygen atom, a nitrogen atom or a sulfur atom singly or in combination, and a C₅₋₉ fused heterobicyclic group with a constituent atom number of 8 to 10. Examples of the 5-to 7-membered C₂₋₆ heteromonocyclic group include a 2-thienyl group, a 3-thienyl group, a 2-furyl group, a 3-furyl group, a 2-pyranyl group, a 3-pyranyl group, a 4-pyranyl group, a 1-pyrrolyl group, a 2-pyrrolyl group, a 3-pyrrolyl group, a 1-imidazolyl group, a 2-imidazolyl group, a 4-imidazolyl group, a 1-pyrazolyl group, a 3-pyrazolyl group, a 4-pyrazolyl group, a 2-thiazolyl group, a 4-thiazolyl group, a 5-thiazolyl group, a 3-isothiazolyl group, a 4-isothiazolyl group, a 5-isothiazolyl group, a 2-oxazolyl group, a 4-oxazolyl group, a 5-oxazolyl group, a 3-isoxazolyl group, a 4-isoxazolyl group, a 5-isoxazolyl group, a 2-pyridyl group, a 3-pyridyl group, a 4-pyridyl group, a 2-pyrazinyl group, a 2-pyrimidinyl group, a 4-pyrimdinyl group, a 5-pyrimidmyl group, a 3-pyridazinyl group, a 4-pyridazinyl group, a 2-1,3,4-oxadiazolyl group, a 2-1,3,4-thiadiazolyl group, a 3-1,2,4-oxadiazolyl group, a 5-1,2,4-oxadiazolyl group, a 3-1,2,4-thiadiazolyl group, a 5-1,2,4-thiadiazolyl group, a 3-1,2,5-oxadiazolyl group, a 3-1,2,5-thiadiazolyl group and the like; and examples of the C₅₋₉ fused heterobicyclic group with a constituent atom number of 8 to 10 include a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 4-isobenzofuranyl group, a 5-isobenzofuranyl group, a 2-benzothienyl group, a 3-benzothienyl group, a 4-benzothienyl group, a 5-benzothienyl group, a 6-benzothienyl group, a 7-benzothienyl group, a 1-isobenzothienyl group, a 4-isobenzothienyl group, a 5-isobenzothienyl group, a 2-chromenyl group, a 3-chromenyl group, a 4-chromenyl group, a 5-chromenyl group, a 6-chromenyl group, a 7-chromenyl group, an 8-chromenyl group, a 1-indolizinyl group, a 2-indolizinyl group, a 3-indolizinyl group, a 5-indolizinyl group, a 6-indolizinyl group, a 7-indolizinyl group, an 8-indolizinyl group, a 1-isoindolyl group, a 2-isoindolyl group, a 4-isoindolyl group, a 5-isoindolyl group, a i-indalyl group, a 2-indolyl group, a 3-indolyl group, a 4-indolyl group, a 5-indolyl group, a 6-indolyl group, a 7-indolyl group, a 1-indazolyl group, a 2-indazolyl group, a 3-indazolyl group, a 4-indazolyl group, a 5-indazolyl group, a 6-indazolyl group, a 7-indazolyl group, a 1-purinyl group, a 2-purinyl group, a 3-purinyl group, a 6-purinyl group, a 7-purinyl group, an 8-purinyl group, a 2-quinolyl group, a 3-quinolyl group, a 4-quinolyl group, a 5-quinolyl group, a 6-quinolyl group, a 7-quinolyl group, an 8-quinolyl group, a 1-isoquinolyl group, a 3-isoquinolyl group, a 4-isoquinolyl group, a 5-isoquinolyl group, a 6-isoquinolyl group, a 7-isoquinolyl group, an 8-isoquinolyl group, a 1-phthalazinyl group, a 5-phthalazinyl group, a 6-phthalazinyl group, a 1-2,7-naphthyridinyl group, a 3-2,7-naphthyridinyl group, a 4-2,7-naphthyridinyl group, a 1-2,6-naphthyridinyl group, a 3-2,6-naphthyridinyl group, a 4-2,6-naphthyndinyl group, a 2-1,8-naphthyridinyl group, a 3-1,8-naphthyridinyl group, a 4-1,8-naphthyridinyl group, a 2-1,7-naphthyridinyl group, a 3-1,7-naphthyridinyl group, a 4-1,7-naphthyridinyl group, a 5-1,7-naphthyridinyl group, a 6-1,7-naphthyridinyl group, an 8-1,7-naphthyridinyl group, a 2-1,6-naphthyridinyl group, a 3-1,6-naphthyridmyl group, a 4-1,6-naphthyridinyl group, a 5-1,6-naphthyridinyl group, a 7-1,6-naphthyridinyl group, an 8-1,6-naphthyridinyl group, a 2-1,5-naphthyridinyl group, a 3-1,5-naphthyridinyl group, a 4-1,5-naphthyridinyl group, a 6-1,5-naphthyridinyl group, a 7-1,5-naphthyridinyl group, an 8-1,5-naphthyridinyl group, a 2-quinoxalinyl group, a 5-quinoxalinyl group, a 6-quinoxalinyl group, a 2-quinazolinyl group, a 4-quinazolinyl group, a 5-quinazolinyl group, a 6-quinazolinyl group, a 7-quinazolinyl group, an 8-quinazolinyl group, a 3-cinnolinyl group, a 4-cinnolinyl group, a 5-cinnolinyl group, a 6-cinnolinyl group, a 7-cinniolinyl group, an 8-cinnolinyl group, a 2-pteridinyl group, a 4-pteridinyl group, a 6-pteridinyl group, a 7-pteridinyl group and the like.
Examples of the C₁₋₆ alkylaminocarbonyl group include a methylaminocarbonyl group, an ethylaminocarbonyl group, an n-propylaminocarbonyl group, an i-propylaminocarbonyl group, an n-butylamincarbonyl group, an i-butylaminocarbonyl group, an s-butylaminocarbonyl group, a t-butylaminocarbonyl group, a 1-pentylaminocarbonyl group, a 2-pentylaminocarbonyl group, a 3-pentylaminocarbonyl group, an i-pentylaminocarbonyl group, neopentylaminocarbonyl, a t-pentylaminocarbonyl group, a 1-hexylaminocarbonyl group, a 2-hexylaminocarbonyl group, a 3-hexylaminocarbonyl group and the like; examples of the di-C₁₋₆ alkylaminocarbonyl group include a dimethylaminocarbonyl group, a diethylaminocarbonyl group, di-n-propylaminocarbonyl, a di-i-propylaminocarbonyl group, a di-c-propylaminocarbonyl group, a di-n-butylaminocarbonyl group, a di-i-butylaminocarbonyl group, a di-s-butylaminocarbonyl group, a di-t-butylaminocarbonyl group, a di-c-butylaminocarbonyl group, a di-1-pentylaminocarbonyl group, a di-2-pentylaminocarbonyl group, a di-3-pentylaminocarbonyl group, a di-i-pentylaminocarbonyl group, a di-neopentylaminocarbonyl group, a di-t-pentylaminocarbonyl group, a di-c-pentylaminocarbonyl group, a di-1-hexylaminocarbonyl group, a di-2-hexylaminocarbonyl group, a di-3-hexylaminocarbonyl group, a di-c-hexylaminocarbonyl group, a di-(1-methyl-n-pentyl)aminocarbonyl group, a di-(1,1,2-trimethyl-n-propyl)aminocarbonyl group, a di-(1,2,2-trimethyl-n-propyl)aminocarbonyl group, a di-(3,3-dimethyl-n-butyl)aminocarbonyl group, a methyl(ethyl)aminocarbonyl group, a methyl(n-propyl)aminocarbonyl group, a methyl(i-propyl)aminocarbonyl group, a methyl(e-propyl)aminocarbonyl group, a methyl(n-butyl)aminocarbonyl group, a methyl(i-butyl)aminocarbonyl group, a methyl(s-butyl)aminocarbonyl group, a methyl(t-butyl)aminocarbonyl group, a methyl(c-butyl)aminocarbonyl group, an ethyl(n-propyl)aminocarbonyl group, an ethyl(i-propyl)aminocarbonyl group, an ethyl(c-propyl)aminocarbonyl group, an ethyl(n-butyl)aminocarbonyl group, an ethyl(i-butyl)aminocarbonyl group, an ethyl(s-butyl)aminocarbonyl group, an ethyl(t-butyl)aminocarbonyl group, an ethyl(c-butyl)aminocarbonyl group, an n-propyl(i-propyl)aminocarbonyl group, an n-propyl(c-propyl)aminocarbonyl group, an n-propyl(n-butyl)aminocarbonyl group, an n-propyl(i-butyl)aminocarbonyl group, an n-propyl(s-butyl)aminocarbonyl group, an n-propyl(t-butyl)aminocarbonyl group, an n-propyl(c-butyl)aminocarbonyl group, an i-propyl(c-propyl)aminocarbonyl group, an i-propyl(n-butyl)aminocarbonyl group, an i-propyl(i-butyl)aminocarbonyl group, an i-propyl(s-butyl)aminocarbonyl group, an i-propyl(t-butyl)aminocarbonyl group, an i-propyl(c-butyl)aminocarbonyl group, a c-propyl(n-butyl)aminocarbonyl group, a c-propyt(i-butyl)aminocarbonyl group, a c-propyl(s-butyl)aminocarbonyl group, a c-propyl(t-butyl)aminocarbonyl group, a c-propyl(c-butyl)aminocarbonyl group, an n-butyl(i-butyl)aminocarbonyl group, an n-butyl(s-butyl)aminocarbonyl group, an n-butyl(t-butyl)aminocarbonyl group, an n-butyl(c-butyl)aminocarbonyl group, an i-butyi(s-butyl)aminocarbonyl group, an i-butyl(t-butyl)aminocarbonyl group, an i-butyl(c-butyl) aminocarbonyl group, an s-butyl(t-butyl)aminocarbonyl group, an s-butyl(c-butyl)aminocarbonyl group, a t-butyl(c-butyl)aminocarbonyl group and the like; examples of the C₁₋₆ alkylcarbonyl group include a methylcarbonyl group, an ethylcarbonyl group, an n-propylcarbonyl group, an i-propylcarbonyl group, an n-butylcarbonyl group, an i-butylcarbonyl group, an s-butylcarbonyl group, a t-butylcarbonyl group, a i-pentylcarbonyl group, a 2-pentylcarbonyl group, a 3-pentylcarbonyl group, an i-pentylcarbonyl group, a neopentylcarbonyl group, a t-pentylcarbonyl group, a 1-hexylcarbonyl group, a 2-hexylcarbonyl group and a 3-hexylcarbonyl group; examples of the C₃₋₈ cycloalkylcarbonyl group include a c-propylcarbonyl group, a c-butylcarbonyl group, a 1-methyl-c-propylcarbonyl group, a 2-methyl-c-propylcarbonyl group, a c-pentylcarbonyl group, a 1-methyl-c-butylcarbonyl group, a 2-methyl-c-butylcarbonyl group, a 3-methyl-c-butylcarbonyl group, a 1,2-dimethyl-c-propylcarbonyl group, a 2,3-dimethyl-c-propylcarbonyl group, a 1-ethyl-c-propylcarbonyl group, a 2-ethyl-c-propylcarbonyl group, a c-hexylcarbonyl group, a c-heptylcarbonyl group, a c-octylearbonyl group, a 1-methyl-c-hexylcarbonyl group, a 2-methyl-c-hexylcarbonyl group, a 3-methyl-c-hexylcarbonyl group, a 1,2-dimethyl-c-hexylcarbonyl group, a 2,3-dimethyl-c-propylcarbonyl group, a 1-ethyl-c-propylcarbonyl group, a 1-methyl-c-pentylcarbonyl group, a 2-methyl-c-pentylcarbonyl group, a 3-methyl-c-pentylcarbonyl group, a 1-ethyl-c-butylcarbonyl group, a 2-ethyl-c-butylcarbonyl group, a 3-ethyl-c-butylcarbonyl group, a 1,2-dimethyl-c-butylcarbonyl group, a 1,3-dimethyl-c-butylcarbonyl group, a 2,2-dimethyl-c-butylcarbonyl group, a 2,3-dimethyl-c-butylcarbonyl group, a 2,4-dimethyl-c-butylcarbonyl group, a 3,3-dimethyl-c-butylcarbonyl group, a 1-n-propyl-c-propylcarbonyl group, a 2-n-propyl-c-propylcarbonyl group, a 1-i-propyl-c-propylcarbonyl group, a 2-i-propyl-c-propylcarbonyl group, a 1,2,2-trimethyl-c-propylcarbonyl group, a 1,2,3-trimethyl-c-propylcarbonyl group, a 2,2,3-trimethyl-c-propylcarbonyl group, a 1-ethyl-2-methyl-c-propylcarbonyl group, a 2-ethyl-1-methyl-c-propylcarbonyl group, a 2-ethyl-2-methyl-c-propylcarbonyl group, a 2-ethyl-3-methyl-c-propylcarbonyl group and the like; examples of the C₁₋₆ alkoxycarbonyl group include a methoxycarbonyl group, an ethoxycarbonyl group, an n-propoxycarbonyl group, an i-propoxycarbonyl group, an n-butoxycarbonyl group, an i-butoxycarbonyl group, an s-butoxycarbonyl group, a t-butoxycarbonyl group, a 1-pentyloxycarbonyl group, a 2-pentyloxycarbonyl group, a 3-pentyloxycarbonyl group, an i-pentyloxycarbonyl group, a neopentyloxycarbonyl group, a t-pentyloxycarbonyl group, a 1-hexyloxycarbonyl group, a 2-hexyloxycarbonyl group, a 3-hexyloxycarbonyl group and the like; examples of the C₁₋₆ alkylsulfonyl group include a methanesulfonyl group, a trifluoromethanesulfonyl group and an ethanesulfonyl group; and examples of the C₆₋₁₄ arylsulfonyl group include a benzenesulfonyl group, an o-biphenylsulfonyl group, an m-biphenylsulfonyl group, a p-biphenylsulfonyl group, a 1-naphthalenesulfonyl group, a 2-naphthalenesulfonyl group, a 1-anthracenesulfonyl group, a 2-anthracenesulfonyl group, a 9-anthracenesulfonyl group, a 1-phenanthrenesulfonyl group, a 2-phenanthrenesulfonyl group, a 3-phenanthrenesulfonyl group, a 4-phenanthrenesulfonyl group, a 9-phenanthrenesulfonyl group and the like.
Examples of the C₂₋₉ heteroarylsulfonyl group include a 5- to 7-membered C₂₋₆ heteromonocyclic sulfonyl group which may contain 1 to 3 atoms of an oxygen atom, a nitrogen atom or a sulfur atom singly or in combination, and a C₅₋₉ fused heterobicyclic sulfonyl group with a constituent atom number of 8 to 10.
Examples of the 5- to 7-membered C₂₋₆ heteromonocyclic sulfonyl group include a 2-thienylsulfonyl group, a 3-thienylsulfonyl group, a 2-furylsulfonyl group, a 3-furylsulfonyl group, a 2-pyranylsulfonyl group, a 3-pyranylsulfonyl group, a 4-pyranylsulfonyl group, a 1-pyrrolylsulfonyl group, a 2-pyrrolylsulfonyl group, a 3-pyrrolylsulfonyl group, a 1-imidazolylsulfonyl group, a 2-imidazolylsulfonyl group, a 4-imidazolylsulfonyl group, a 1-pyrazolylsulfonyl group, a 3-pyrazolylsulfonyl group, a 4-pyrazolylsulfonyl group, a 2-thiazolylsulfonyl group, a 4-thiazolylsulfonyl group, a 5-thiazolylsulfonyl group, a 3-isothiazolylsulfonyl group, a 4-isothiazolylsulfonyl group, a 5-isothiazolylsulfonyl group, a 2-oxazolylsulfonyl group, a 4-oxazolylsulfonyl group, a 5-oxazolylsulfonyl group, a 3-isoxazolylsulfonyl group, a 4-isoxazolylsulfonyl group, a 5-isoxazolylsulfonyl group, a 2-pyridylsulfonyl group, a 3-pyridylsulfonyl group, a 4-pyridylsulfonyl group, a 2-pyrazinylsulfonyl group, a 2-pyrimidinylsulfonyl group, a 4-pyrimidinylsulfonyl group, a 5-pyrimidinylsulfonyl group, a 3-pyridazinylsulfonyl group, a 4-pyridazinylsulfonyl group, a 2-1,3,4-oxadiazolylsulfonyl group, a 2-1,3,4-thiadiazolylsulfonyl group, a 3-1,2,4-oxadiazolylsulfonyl group, a 5-1,2,4-oxadiazolylsulfonyl group, a 3-1,2,4-thiadiazolylsulfonyl group, a 5-1,2,4-thiadiazolylsulfonyl group, a 3-1,2,5-oxadiazolylsulfonyl group, a 3-1,2,5-thiadiazolylsulfonyl group and the like.
Examples of the C₅₋₉ fused heterobicyclic sulfonyl group with a constituent atom number of 8 to 10 include 2-benzofuranylsulfonyl group, a 3-benzofuranylsulfonyl group, a 4-benzofuranylsulfonyl group, a 5-benzofuranylsulfonyl group, a 6-benzofuranylsulfonyl group, a 7-benzofuranylsulfonyl group, a 1-isobenzofuranylsulfonyl group, a 4-isobenzofuranylsulfonyl group, a 5-isobenzofuranylsulfonyl group, a 2-benzothienylsulfonyl group, a 3-benzothienylsulfonyl group, a 4-benzothienylsulfonyl group, a 5-benzothienylsulfonyl group, a 6-benzothienylsulfonyl group, a 7-benzothienylsulfonyl group, a 1-isobenzothienylsulfonyl group, a 4-isobenzothienylsulfonyl group, a 5-isobenzothienylsulfonyl group, a 2-chromenylsulfonyl group, a 3-chromenylsulfonyl group, a 4-chromenylsulfonyl group, a 5-chromenylsulfonyl group, a 6-chromenylsulfonyl group, a 7-chromenylsulfonyl group, an 8-chromenylsulfonyl group, a 1-indolizinylsulfonyl group, a 2-indolizinylsulfonyl group, a 3-indolizinylsulfonyl group, a 5-indolizinylsulfonyl group, a 6-indolizinylsulfonyl group, a 7-indolizinylsulfonyl group, an 8-indolizinylsulfonyl group, a 1-isoindolylsulfonyl group, a 2-isoindolylsulfonyl group, a 4-isoindolylsulfonyl group, a 5-isoindolylsulfonyl group, a 1-indolylsulfonyl group, a 2-indolylsulfonyl group, a 3-indolylsulfonyl group, a 4-indolylsulfonyl group, a 5-indolylsulfonyl group, a 6-indolylsulfonyl group, a 7-indolylsulfonyl group, a 1-indazolylsulfonyl group, a 2-indazolylsulfonyl group, a 3-indazolylsulfonyl group, a 4-indazolylsulfonyl group, a 5-indazolylsulfonyl group, a 6-indazolylsulfonyl group, a 7-indazolylsulfonyl group, a 1-purinylsulfonyl group, a 2-purinylsulfonyl group, a 3-purinylsulfonyl group, a 6-purinylsulfonyl group, a 7-purinylsulfonyl group, an 8-purinylsulfonyl group, a 2-quinolylsulfonyl group, a 3-quinolylsulfonyl group, a 4-quinolylsulfonyl group, a 5-quinolylsulfonyl group, a 6-quinolylsulfonyl group, a 7-quinolylsulfonyl group, an 8-quinolylsulfonyl group, a 1-isoquinolylsulfonyl group, a 3-isoquinolylsulfonyl group, a 4-isoquinolylsulfonyl group, a 5-isoquinolylsulfonyl group, a 6-isoquinolylsulfonyl group, a 7-isoquinolylsulfonyl group, an 8-isoquinolylsulfonyl group, a 1-phthalazinylsulfonyl group, a 5-phthalazinylsulfonyl group, a 6-phthalazinylsulfonyl group, a 1-2,7-naphthyridinylsulfonyl group, a 3-2,7-naphthyridinylsulfonyl group, a 4-2,7-naphthyridinylsulfonyl group, a 1-2,6-naphthyridinylsulfonyl group, a 3-2,6-naphthyridinylsulfonyl group, a 4-2,6-naphthyridinylsulfonyl group, a 2-1,8-naphthyridinylsulfonyl group, a 3-1,8-naphthyridinylsulfonyl group, a 4-1,8-naphthyridinylsulfonyl group, a 2-1,7-naphthyridinylsulfonyl group, a 3-1,7-naphthyridinylsulfonyl group, a 4-1,7-naphthyridinylsulfonyl group, a 5-1,7-naphthyridinyisulfonyl group, a 6-1,7-naphthyridinylsulfonyl group, an 8-1,7-naphthyridinylsulfonyl group, a 2-1,6-naphthyridinylsulfonyl group, a 3-1,6-naphthyridinylsulfonyl group, a 4-1,6-naphthyridinylsulfonyt group, a 5-1,6-naphthyridinyisulfonyl group, a 7-1,6-naphthyridinylsulfonyl group, an 8-1,6-naphthyridinylsulfonyl group, a 2-1,5-naphthyridinylsulfonyl group, a 3-1,5-naphthyridinylsulfonyl group, a 4-1,5-naphthyridinylsulfonyl group, a 6-1,5-naphthyridinylsulfonyl group, a 7-1,5-naphthyridinylsulfonyl group, an 8-1,5-naphthyridinylsulfonyl group, a 2-quinoxalinylsulfonyl group, a 5-quirzoxalinylsulfonyl group, a 6-quinoxalinylsulfonyl group, a 2-quinazolinylsulfonyl group, a 4-quinazolinylsulfonyl group, a 5-quinazolinylsulfonyl group, a 6-quinazolinylsulfonyl group, a 7-quinazolinylsulfonyl group, an 8-quinazolinylsulfonyl group, a 3-cinnolinylsulfonyl group, a 4-cinnolinylsulfonyl group, a 5-cinnolinylsulfonyl group, a 6-cinnolinylsulfonyl group, a 7-cinnolinylsulfonyl group, an 8-cinnolinylsulfonyl group, a 2-pteridinylsulfonyl group, a 4-pteridinylsulfonyl group, a 6-pteridinylsulfonyl group, a 7-pteridinylsulfonyl group and the like. Examples of the C₆₋₁₄ arylcarbonyl group include a phenylcarbonyl group, an o-biphenylylcarbonyl group, an m-biphenylylcarbonyl group, a p-biphenylylcarbonyl group, a 1-naphtylcarbonyl group, a 2-naphtylcarbonyl group, a 1-anthrylcarbonyl group, a 2-anthrylcarbonyl group, a 9-anthrylcarbonyl group, a 1-phenanthrylcarbonyl group, a 2-phenanthrylcarbonyl group, a 3-phenanthrylcarbonyl group, a 4-phenanthrylcarbonyl group, a 9-phenanthrylcarbonyl group and the like.
Examples of the C₂₋₉ heteroarylcarbonyl group include a 5- to 7-membered C₂₋₆ heteromonocyclic carbonyl group which may contain 1 to 3 atoms of an oxygen atom, a nitrogen atom or a sulfur atom singly or in combination, and a C₅₋₉ fused heterobicyclic carbonyl group with a constituent atom number of 8 to 10. Examples of the 5- to 7-membered C₂₋₆ heteromonocyclic carbonyl group include a 2-thienylcarbonyl group, a 3-thienylcarbonyl group, a 2-furylcarbonyl group, a 3-furylcarbonyl group, a 2-pyranylcarbonyl group, a 3-pyranylcarbonyl group, a 4-pyranylcarbonyl group, a 1-pyrrolylcarbonyl group, a 2-pyrrolylcarbonyl group, a 3-pyrrolylcarbonyl group, a 1-imidazolylcarbonyl group, a 2-imidazolylcarbonyl group, a 4-imidazolylcarbonyl group, a 1-pyrazolylcarbonyl group, a 3-pyrazolylcarbonyl group, a 4-pyrazolylcarbonyl group, a 2-thiazolylcarbonyl group, a 4-thiazolylcarbonyl group, a 5-thiazolylcarbonyl group, a 3-isothiazolylcarbonyl group, a 4-isothiazolylcarbonyl group, a 5-isothiazolylcarbonyl group, a 2-oxazolylcarbonyl group, a 4-oxazolylcarbonyl group, a 5-oxazolylcarbonyl group, a 3-isoxazolylcarbonyl group, a 4-isoxazolylcarbonyl group, a 5-isoxazolylcarbonyl group, a 2-pyridylcarbonyl group, a 3-pyridylcarbonyl group, a 4-pyridylcarbonyl group, a 2-pyrazinylcarbonyl group, a 2-pyrimidinylcarbonyl group, a 4-pyrimidinylcarbonyl group, a 5-pyrimidinylcarbonyl group, a 3-pyridazinylcarbonyl group, a 4-pyridazinylcarbonyl group, a 2-1,3,4-oxadiazolylcarbonyl group, a 2-1,3,4-thiadiazolylcarbonyl group, a 3-1,2,4-oxadiazolylcarbonyl group, a 5-1,2,4-oxadiazolylcarbonyl group, a 3-1,2,4-thiadiazolylcarbonyl group, a 5-1,2,4-thiadiazolylcarbonyl group, a 3-1,2,5-oxadiazolylcarbonyl group, a 3-1,2,5-thiadiazolylcarbonyl group and the like.
Examples of the C₅₋₉ fused heterobicyclic carbonyl group with a constituent atom number of 8 to 10 include a 2-benzofuranylcarbonyl group, a 3-benzofuranylcarbonyl group, a 4-benzofuranylcarbonyl group, a 5-benzofuranylcarbonyl group, a 6-benzofuranylcarbonyl group, a 7-benzofuranylcarbonyl group, a 1-isobenzofuranylcarbonyl group, a 4-isobenzofuranylcarbonyl group, a 5-isobenzofuranylcarbonyl group, a 2-benzothienylcarbonyl group, a 3-benzothienylcarbonyl group, a 4-benzothienylcarbonyl group, a 5-benzothienylcarbonyl group, a 6-benzothienylcarbonyl group, a 7-benzothienylcarbonyl group, a 1-isobcnzothienylcarbonyl group, a 4-isobenzothienylcarbonyl group, a 5-isobenzothienylcarbonyl group, a 2-chromenylcarbonyl group, a 3-chromenylcarbonyl group, a 4-chromenylcarbonyl group, a 5-chromenylcarbonyl group, a 6-chromenylcarbonyl group, a 7-chromenylcarbonyl group, an 8-chromenylcarbonyl group, a 1-indolizinylcarbonyl group, a 2-indolizinylcarbonyl group, a 3-indolizinylcarbonyl group, a 5-indolizinylcarbonyl group, a 6-indolizinylcarbonyl group, a 7-indolizinylcarbonyl group, an 8-indolizinylcarbonyl group, a 1-isoindolylcarbonyl group, a 2-isoindolylcarbonyl group, a 4-isoindolylcarbonyl group, a 5-isoindolylcarbonyl group, a 1-indolylcarbonyl group, a 2-indolylcarbonyl group, a 3-indolylcarbonyl group, a 4-indolylcarbonyl group, a 5-indolylcarbonyl group, a 6-indolylcarbonyl group, a 7-indolylcarbonyl group, a 1-indazolylcarbonyl group, a 2-indazolylcarbonyl group, a 3-indazolylcarbonyl group, a 4-indazolylcarbonyl group, a 5-indazolylcarbonyl group, a 6-indazolylcarbonyl group, a 7-indazolylcarbonyl group, a 1-purmylcarbonyl group, a 2-purinylcarbonyl group, a 3-purinylearbonyl group, a 6-purinylearbonyl group, a 7-purinylcarbonyl group, an 8-purinylcarbonyl group, a 2-quinolylcarbonyl group, a 3-quinolylcarbonyl group, a 4-quinolylcarbonyl group, a 5-quinolylcarbonyl group, a 6-quinolylcarbonyl group, a 7-quinolylcarbonyl group, an 8-quinolylcarbonyl group, a 1-isoquinolylcarbonyl group, a 3-isoquinolylcarbonyl group, a 4-isoquinolylcarbonyl group, a 5-isoquinolylcarbonyl group, a 6-isoquinolylcarbonyl group, a 7-isoquinolylcarbonyl group, an 8-isoquinolylcarbonyl group, a 1-phthalazinylcarbonyl group, a 5-phthalazinylcarbonyl group, a 6-phthalazinylcarbonyl group, a 1-2,7-naphthyridinylcarbonyl group, a 3-2,7-naphthyridinylcarbonyl group, a 4-2,7-naphthyridinylcarbonyl group, a 1-2,6-naphthyridinylcarbonyl group, a 3-2,6-naphthyridinylcarbonyl group, a 4-2,6-naphthyridinylcarbonyl group, a 2-1,8-naphthyridinylcarbonyl group, a 3-1,8-naphthyridinylcarbonyl group, a 4-1,8-naphthyridinylcarbanyl group, a 2-1,7-naphthyridinylcarbonyl group, a 3-1,7-naphthyridinylcarbonyl group, a 4-1,7-naphthyridinylcarbonyl group, a 5-1,7-naphthyridinylcarbonyl group, a 6-1,7-naphthyridinylcarbonyl group, an 8-1,7-naphthyridinylcarbonyl group, a 2-1,6-naphthyridinylcarbonyl group, a 3-1,6-naphthyridinylcarbonyl group, a 4-1,6-naphthyridinylcarbonyl group, a 5-1,6-naphthyridinylcarbonyl group, a 7-1,6-naphthyridinylcarbonyl group, an 8-1,6-naphthyridinylcarbonyl group, a 2-1,5-naphthyridinylcarbonyl group, a 3-1,5-naphthyridinylcarbonyl group, a 4-1,5-naphthyridinylcarbonyl group, a 6-1,5-naphthyridinylcarbonyl group, a 7-1,5-naphthyridinylcarbonyl group, an 8-1,5-naphthyridinylcarbonyl group, a 2-quinoxalinylcarbonyl group, a 5-quinoxalinylcarbonyl group, a 6-quinoxalinylcarbonyl group, a 2-quinazolinylcarbonyl group, a 4-quinazolinylcarbonyl group, a 5-quinazolinylcarbonyl group, a 6-quinazolinylcarbonyl group, a 7-quinazolinylcarbonyl group, an 8-quinazolinylcarbonyl group, a 3-cinnolinyicarbonyl group, a 4-cinnolinylcarbonyl group, a 5-cinnolinylcarbonyl group, a 6-cinnolinylcarbonyl group, a 7-cinnolinylcarbonyl group, an 8-cinnolinylcarbonyl group, a 2-pteridinylcarbonyl group, a 4-pteridinylcarbonyl group, a 6-pteridinylcarbonyl group, a 7-pteridinylcarbonyl group and the like.

Each of R¹⁴ and R¹⁵ in Formula (a), Formula (b), Formula (e), Formula (f), Formula (g), Formula (h), Formula (i), Formula (j). Formula (k), Formula (l), Formula (m), Formula (n), Formula (p), Formula (q), Formula (v), Formula (w), Formula (x), Formula (ab), Formula (ae), Formula (af) and Formula (ag) is preferably independently a hydrogen atom, a methyl group, an ethyl group, an n-propyl group, an i-propyl group, an n-butyl group, an n-pentyl group, an i-pentyl group, a methylcarbonyloxymethyl group, an ethylcarbonyloxymethyl group, a methylcarbonyloxyethyl group, an ethylcarbonyloxyethyl group, a methylcarbonylaminomethyl group, an ethylcarbonylaminoethyl group, a methylcarbonylaminoethyl group, an ethylcarbonylaminoethyl group, a methoxycarbonylmethyl group, an ethoxycarbonylmethyl group, a methoxycarbonylethyl group, an ethoxycarbonylethyl group, a phenyl group, an o-biphenylyl group, an m-biphenylyl group, a p-biphenylyl group, a 1-naphtyl group, a 2-naphtyl group, a 2-pyridyl group, a 3-pyridyl group, a 4-pyridyl group, a methylaminocarbonyl group, an ethylaminocarbonyl group, an n-propylaminocarbonyl group, an i-propylaminocarbonyl group, an n-butylaminocarbonyl group, a dimethylaminocarbonyl group, a diethylaminocarbonyl group, a di-n-propylaminocarbonyl group, a di-i-propylaminocarbonyl group, a di-c-propylaminocarbonyl group, a di-n-butylaminocarbonyl group, a methylcarbonyl group, an ethylcarbonyl group, an n-propylcarbonyl group, an i-propylcarbonyl group, an n-butylcarbonyl group, a c-pentylcarbonyl group, a c-hexylcarbonyl group, a methoxycarbonyl group, an ethoxycarbonyl group, an n-propoxycarbonyl group, an i-propoxycarbonyl group, an n-butoxycarbonyl group, an i-butoxycarbonyl group, an s-butoxycarbonyl group, a t-butoxycarbonyl group, a methanesulfonyl group, a trifluoromethanesulfonyl group, a benzenesulfonyl group, an o-biphenylsulfonyl group, an m-biphenylsulfonyl group, a p-biphenylsulfonyl group, a 1-naphthalenesulfonyl group, a 2-naphthalenesulfonyl group, a 2-pyridylsulfonyl group, a 3-pyridylsulfonyl group, a 4-pyridylsulfonyl group, a phenylcarbonyl group, an o-biphenylylcarbonyl group, an m-biphcnylylcarbonyl group, a p-biphenylylcarbonyl group, a 1-naphtylcarbonyl group, a 2-naphtylcarbonyl group, a 2-pyridylcarbonyl group, a 3-pyridylcarbonyl group or a 4-pyridylcarbonyl group.

R¹⁴, R¹⁵, R¹⁶ and R¹⁷ in Formula (a), Formula (b), Formula (c), Formula (d), Formula (f), Formula (g), Formula (h), Formula (j), Formula (k), Formula (m), Formula (n), Formula (o), Formula (p), Formula (q), Formula (r), Formula (s), Formula (t), Formula (u), Formula (v), Formula (w), Formula (y), Formula (z), Formula (aa), Formula (ab), Formula (ac), Formula (ad), Formula (ae) and Formula (af) will be described. Each of R¹⁶, R¹⁷, R¹⁸ and R¹⁹ in Formula (a), Formula (b), Formula (c), Formula (d), Formula (f), Formula (g), Formula (h), Formula (j), Formula (k), Formula (m), Formula (n), Formula (o), Formula (p), Formula (q), Formula (r), Formula (s), Formula (t), Formula (u), Formula (v), Formula (w), Formula (y), Formula (z), Formula (aa), Formula (ab), Formula (ac), Formula (ad), Formula (ae) and Formula (af) is independently a hydrogen atom, a halogen atom, a C₁₋₆ alkyl group (the alkyl group is unsubstituted or substituted with a halogen atom, a C₁₋₆ alkoxy group (the alkoxy group is unsubstituted or substituted with a halogen atom), an amino group, a hydroxyl group, a C₆₋₁₄ aryl group, a C₂₋₉ heteroaryl group (each of the aryl group and the heteroaryl group is unsubstituted or substituted with r pieces of R²¹ (R²¹ means the same as R¹³ and r means the same as q)), a C₁₋₆ alkylaminocarbonyl group, a di-C₁₋₆ alkylaminocarbonyl group, a C₁₋₆carbonyloxy group, a C₁₋₆ alkylcarbonyl group (the alkylcarbonyloxy group and the alkylcarbonyl group are unsubstituted or substituted with a halogen atom), a C₁₋₆ alkylcarbonylamino group, a C₃₋₈ cycloalkylcarbonyl group, a C₁₋₆ alkoxycarbonyl group, a C₁₋₆ alkylsulfonyl group (the cycloalkylcarbonyl group, the alkoxycarbonyl group and the alkylsulfonyl group are unsubstituted or substituted with a halogen atom), a carboxyl group, a C₆₋₁₄ arylcarbonyl group (the arylcarbonyl group is unsubstituted or substituted with a halogen atom) or a C₂₋₉ heteroarylcarbonyl group), a C₃₋₈ cycloalkyl group (the cycloalkyl group is unsubstituted or substituted with a halogen atom, a C₁₋₆ alkoxy group (the alkoxy group is unsubstituted or substituted with a halogen atom), an amino group or a hydroxyl group), a C₁₋₆ alkoxy group (the alkoxy group is unsubstituted or substituted with a halogen atom, a C₁₋₆ alkoxy group (the alkoxy group is unsubstituted or substituted with a halogen atom), a carboxyl group, an amino group, a hydroxyl group, a C₆₋₄ aryl group or a C₂₋₉ heteroaryl group (each of the aryl group and the heteroaryl group is unsubstituted or substituted with r pieces of R²¹ (R²¹ means the same as R¹³ and r means the same as q))), a C₁₋₆ thioalkoxy group (the thioalkoxy group is unsubstituted or substituted with a halogen atom, a C₁₋₆ alkoxy group (the alkoxy group is unsubstituted or substituted with a halogen atom), a carboxyl group, a hydroxyl group, a C₆₋₁₄ aryl group or a C₂₋₉ heteroaryl group (each of the aryl group and the heteroaryl group is unsubstituted or substituted with r pieces of R²¹ (R²¹ means the same as R¹³ and r means the same as q))), a hydroxyl group, a C₆₋₁₄ aryl group, a C₂₋₉ heteroaryl group (each of the aryl group and the heteroaryl group is unsubstituted or substituted with r pieces of R²¹ (R²¹ means the same as R¹³ and r means the same as q)), a C₁₋₆ alkylcarbonyloxy group, a nitro group, a cyano group, a formyl group, a formamide group, an amino group, a sulfo group, a C₁₋₆ alkylamino group, a di-C₁₋₆ alkylamino group, a C₆₋₁₄ arylamino group, a C₂₋₉ heteroarylamino group (each of the arylamino group and the heteroarylamino group is unsubstituted or substituted with r pieces of R²¹ (R²¹ means the same as R¹³ and r means the same as q)), a C₁₋₆ alkylcarbonylamino group, a C₁₋₆ alkylsulfonamide group, a carbamoyl group, a C₁₋₆ alkylaminocarbonyl group, a di-C₁₋₆ alkylaminocarbonyl group, a C₁₋₆ alkylcarbonyl group, a C₆₋₁₄ arylcarbonyl group, a C₂₋₉ heteroarylcarbonyl group (each of the arylcarbonyl group and the heteroarylcarbonyl group is unsubstituted or substituted with r pieces of R²¹ (R²¹ means the same as R¹³ and r means the same as q)), a C₁₋₆ alkoxycarbonyl group, a sulfamoyl group, a C₁₋₆ alkylsulfonyl group, a C₆₋₁₄ arylsulfonyl group, a C₂₋₉ heteroarylsulfonyl group (each of the arylsulfonyl group and the heteroarylsulfonyl group is unsubstituted or substituted with r pieces of R²¹ (R²¹ means the same as R¹³ and r means the same as q)), a carboxyl group or a C₂₋₉ heterocyclyl group (the heterocyclyl group is unsubstituted or substituted with a halogen atom, a C₁₋₆ alkyl group (the alkyl group is unsubstituted or substituted with a halogen atom, a C₁₋₆ alkoxy group (the alkoxy group is unsubstituted or substituted with a halogen atom), an amino group, a carboxyl group or a hydroxyl group), a C₁₋₆ alkoxy group (the alkoxy group is unsubstituted or substituted with a halogen atom), a C₆₋₁₄ aryl group, a C₂₋₉ heteroaryl group (each of the aryl group and the heteroaryl group is unsubstituted or substituted with r pieces of R²¹ (R²¹ means the same as R¹³ and r means the same as q)), a hydroxyl group, a nitro group, a cyano group, a formyl group, a formamide group, an amino group, a C₁₋₆ alkylamino group, a di-C₁₋₆ alkylamino group, a C₁₋₆ alkylcarbonylamino group, a C₁₋₆ alkylsulfonamide group, a carbamoyl group, a C₁₋₆ alkylaminocarbonyl group, a di-C₁₋₆ alkylaminocarbonyl group, a C₁₋₆ alkylcarbonyl group, a C₁₋₆ alkoxycarbonyl group, a sulfamoyl group, a C₁₋₆ alkylsulfonyl group, a carboxyl group or a C₆₋₁₄ arylcarbonyl group).

Each of the atoms and the substituents of R¹⁶, R¹⁷, R¹⁸ and R¹⁹ in Formula (a), Formula (b), Formula (c), Formula (d), Formula (f), Formula (g), Formula (h), Formula (j), Formula (k), Formula (m), Formula (n), Formula (o), Formula (p), Formula (q), Formula (r), Formula (s), Formula (t), Formula (u), Formula (v), Formula (w), Formula (y), Formula (z), Formula (aa), Formula (ab), Formula (ac), Formula (ad), Formula (ae) and Formula (af) will be specifically described. Examples of the halogen atom include a fluorine atom, a chlorine atom, a bromine atom and an iodine atom; examples of the C₁₋₆ alkyl group include a methyl group, an ethyl group, an n-propyl group, an i-propyl group, an n-butyl group, an i-butyl group, an s-butyl group, a t-butyl group, an n-pentyl group, a 2-pentyl group, a 3-pentyl group, an i-pentyl group, a neopentyl group, a 2,2-dimethylpropyl group, an n-hexyl group, a 2-hexyl group, a 3-hexyl group, a 1-methyl-n-pentyl group, a 1,1,2-trimethyl-n-propyl group, a 1,2,2-trimethyl-n-propyl group, a 3,3-dimethyl-n-butyl group, a methylcarbonyloxymethyl group, an ethylcarbonyloxymethyl group, a methylcarbonyloxyethyl group, an ethylcarbonyloxyethyl group, a methylcarbonylaminomethyl group, an ethylcarbonylaminomethyl group, a methylcarbonylaminoethyl group, an ethylcarbonylaminoethyl group, a methoxycarbonylmethyl group, an ethoxycarbonylmethyl group, a methoxycarbonylethyl group, an ethoxycarbonylethyl group and the like; examples of the C₁₋₆ alkoxy group include a methoxy group, an ethoxy group, an n-propoxy group, an i-propoxy group, an n-butoxy group, an i-butoxy group, an s-butoxy group, a t-butoxy group, a 1-pentyloxy group, a 2-pentyloxy group, 3-pentyloxy, i-pentyloxy, neopentyloxy, 2,2-dimethylpropoxy, 1-hexyloxy, a 2-hexyloxy group, a 3-hexyloxy group, a 1-methyl-n-pentyloxy group, a 1,1,2-trimethyl-n-propoxy group, a 1,2,2-trimethyl-n-propoxy group, a 3,3-dimethyl-n-butoxy group and the like; and examples of the C₆₋₁₄ aryl group include a phenyl group, an o-biphenylyl group, an m-biphenylyl group, a p-biphenylyl group, a 1-naphtyl group, a 2-naphtyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group and the like.
Examples of the C₂₋₉ heteroaryl group include a 5- to 7-membered C₂₋₆ heteromonocyclic group which may contain 1 to 3 atoms of an oxygen atom, a nitrogen atom or a sulfur atom singly or in combination, and a C₅₋₉ fused heterobicyclic group with a constituent atom number of 8 to 10.
Examples of the 5- to 7-membered C₂₋₆ heteromonocyclic group include a 2-thienyl group, a 3-thienyl group, a 2-furyl group, a 3-furyl group, a 2-pyranyl group, a 3-pyranyl group, a 4-pyranyl group, a 1-pyrrolyl group, a 2-pyrrolyl group, a 3-pyrrolyl group, a 1-imidazolyl group, a 2-imidazolyl group, a 4-imidazolyl group, a 1-pyrazolyl group, a 3-pyrazolyl group, a 4-pyrazolyl group, a 2-thiazolyl group, a 4-thiazolyl group, a 5-thiazolyl group, a 3-isothiazolyl group, a 4-isothiazolyl group, a 5-isothiazolyl group, a 2-oxazolyl group, a 4-oxazolyl group, a 5-oxazolyl group, a 3-isoxazolyl group, a 4-isoxazolyl group, a 5-isoxazolyl group, a 2-pyridyl group, a 3-pyridyl group, a 4-pyridyl group, a 2-pyrazinyl group, a 2-pyrimidinyl group, a 4-pyrimidinyl group, a 5-pyrimidinyl group, a 3-pyridazinyl group, a 4-pyridazinyl group, a 2-1,3,4-oxadiazolyl group, a 2-1,3,4-thiadiazolyl group, a 3-1,2,4-oxadiazolyl group, a 5-1,2,4-oxadiazolyl group, a 3-1,2,4-thiadiazolyl group, a 5-1,2,4-thiadiazolyl group, a 3-1,2,5-oxadiazolyl group, a 3-1,2,5-thiadiazolyl group and the like.
Examples of the C₅₋₉ fused heterobicyclic group with a constituent atom number of 8 to 10 include a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 4-isobenzofuranyl group, a 5-isobenzofuranyl group, a 2-benzothienyl group, a 3-benzothienyl group, a 4-benzothienyl group, a 5-benzothienyl group, a 6-benzothienyl group, a 7-benzothienyl group, a 1-isobenzothienyl group, a 4-isobenzothienyl group, a 5-isobenzothienyl group, a 2-chromenyl group, a 3-chromenyl group, a 4-chromenyl group, a 5-chromenyl group, a 6-chromenyl group, a 7-chromenyl group, an 8-chromenyl group, a 1-indolizinyl group, a 2-indolizinyl group, a 3-indolizinyl group, a 5-indolizinyl group, a 6-indolizinyl group, a 7-indolizinyl group, an 8-indolizinyl group, a 1-isoindolyl group, a 2-isoindolyl group, a 4-isoindolyl group, a 5-isoindolyl group, a 1-indolyl group, a 2-indolyl group, a 3-indolyl group, a 4-indolyl group, a 5-indolyl group, a 6-indolyl group, a 7-indolyl group, a 1-indazolyl group, a 2-indazolyl group, a 3-indazolyl group, a 4-indazolyl group, a 5-indazolyl group, a 6-indazolyl group, a 7-indazolyl group, a 1-purinyl group, a 2-purinyl group, a 3-purinyl group, a 6-purinyl group, a 7-purinyl group, an 8-purinyl group, a 2-quinolyl group, a 3-quinolyl group, a 4-quinolyl group, a 5-quinolyl group, a 6-quinolyl group, a 7-quinolyl group, an 8-quinolyl group, a 1-isoquinolyl group, a 3-isoquinolyl group, a 4-isoquinolyl group, a 5-isoquinolyl group, a 6-isoquinolyl group, a 7-isoquinolyl group, an 8-isoquinolyl group, a 1-phthalazinyl group, a 5-phthalazinyl group, a 6-phthalazinyl group, a 1-2,7-naphthyridinyl group, a 3-2,7-naphthyridinyl group, a 4-2,7-naphthyridinyl group, a 1-2,6-naphthyridinyl group, a 3-2,6-naphthyridinyl group, a 4-2,6-naphthyridinyl group, a 2-1,8-naphthyridinyl group, a 3-1,8-naphthyridinyl group, a 4-1,8-naphthyridinyl group, a 2-1,7-naphthyridinyl group, a 3-1,7-naphthyndinyl group, a 4-1,7-naphthyridinyl group, a 5-1,7-naphthyridinyl group, a 6-1,7-naphthyridinyl group, an 8-1,7-naphthyridinyl group, a 2-1,6-naphthyridinyl group, a 3-1,6-naphthyridinyl group, a 4-1,6-naphthyridinyl group, a 5-1,6-naphthyridinyl group, a 7-1,6-naphthyridinyl group, an 8-1,6-naphthyridinyl group, a 2-1,5-naphthyridinyl group, a 3-1,5-naphthyridinyl group, a 4-1,5-naphthyridinyl group, a 6-1,5-naphthyridinyl group, a 7-1,5-naphthyridinyl group, an 8-1,5-naphthyridinyl group, a 2-quinoxalinyl group, a 5-quinoxalinyl group, a 6-quinoxalinyl group, a 2-quinazolinyl group, a 4-quinazolinyl group, a 5-quinazolinyl group, a 6-quiaiazolinyl group, a 7-quinazolinyl group, an 8-quinazolinyl group, a 3-cinnolinyl group, a 4-cinnolinyl group, a 5-cinnolinyl group, a 6-cinnolinyl group, a 7-cinnolinyl group, an 8-cinnolinyl group, a 2-pteridinyl group, a 4-pteridinyl group, a 6-pteridinyl group, a 7-pteridinyl group and the like.
Examples of the C₁₋₆ alkylcarbonyloxy group include a methylcarbonyloxy group, an ethylcarbonyloxy group, an n-propylcarbonyloxy group, an i-propylcarbonyloxy group, an n-butylcarbonyloxy group, an i-butylcarbonyloxy group, an s-butylcarbonyloxy group, a t-butylcarbonyloxy group, a 1-pentylcarbonyloxy group, a 2-pentylcarbonyloxy group, a 3-pentylcarbonyloxy group, an i-pentylcarbonyloxy group, a neopentylcarbonyloxy group, a t-pentylcarbonyloxy group, a 1-hexylcarbonyloxy group, a 2-hexylcarbonyloxy group, a 3-hexylcarbonyloxy group, a 1-methyl-n-pentylcarbonyloxy group, a 1,1,2-trimethyl-n-propylcarbonyloxy group, a 1,2,2-trimethyl-n-propylcarbonyloxy group, a 3,3-dimethyl-n-butylcarbonyloxy group and the like; examples of the C₁₋₆ alkylamino group include a methylamino group, an ethylamino group, an n-propylamino group, an i-propylamino group, a c-propylamino group, an n-butylamino group, an i-butylamino group, an s-butylamino group, a t-butylamino group, a c-butylamino group, a 1-pentylamino group, a 2-pentylamino group, a 3-pentylamino group, an i-pentylamino group, a neopentylamino group, a t-pentylamino group, a c-pentylamino group, a 1-hexylamino group, a 2-hexylamino group, a 3-hexylamino group, a c-hexylamino group, a 1-methyl-n-pentylamino group, a 1,1,2-trimethyl-n-propylamino group, a 1,2,2-trimethyl-n-propylamino group, a 3,3-dimethyl-n-butylamino group and the like; examples of the di-C₁₋₆ alkylamino group includes a dimethylamino group, a diethylamino group, a di-n-propylamino group, a di-i-propylamino group, a di-c-propylamino group, a di-n-butylamino group, a di-i-butylamino group, a di-s-butylamino group, a di-t-butylamino group, a di-c-butylamino group, a di-1-pentylamino group, a di-2-pentylamino group, a di-3-pentylamino group, a di-i-pentylamino group, a di-neopentylainino group, a di-t-pentylamino group, a di-c-pentylamino group, a di-1-hexylamino group, a di-2-hexylamino group, a di-3-hexylamino group, a di-c-hexylamino group, a di-(1-methyl-n-pentyl)amino group, a di-(1,1,2-trimethyl-n-propyl)amino group, a di-(1,2,2-trimethyl-n-propyl)amino group, a di-(3,3-dimethyl-n-butyl)amino group, a methyl(ethyl)amino group, a methyl(n-propyl)amino group, a methyl(i-propyl)amino group, a methyl(c-propyl)amino group, a methyl(n-butyl)amino group, a methyl(i-butyl)amino group, a methyl(s-butyl)amino group, a methyl(t-butyl)amino group, a methyl(c-butyl)amino group, an ethyl(n-propyl)amino group, an ethyl(i-propyl)amino group, an ethyl(c-propyl)amino group, an ethyl(n-butyl)amino group, an ethyl(i-butyl)amino group, an ethyl(s-butyl)amino group, an ethyl(t-butyl)amino group, an ethyl(c-butyl)amino group, an n-propyl(i-propyl)amino group, an n-propyl(c-propyl)amino group, an n-propyl(n-butyl)amino group, an n-propyl(i-butyl)amino group, an n-propyl(s-butyl)amino group, an n-propyl(t-butyl)amino group, an n-propyl(c-butyl)amino group, an i-propyl(c-propyl)amino group, an i-propyl(n-butyl)amino group, an i-propyl(i-butyl)amino group, an i-propyl(s-butyl)amino group, an i-propyl(t-butyl)amino group, an i-propyl(c-butyl)amino group, a c-propyl(n-butyl)amino group, a c-propyl(i-butyl)amino group, a c-propyl(s-butyl)amino group, a c-propyl(t-butyl)amino group, a c-propyl(c-butyl)amino group, an n-butyl(i-butyl)amino group, an n-butyl(s-butyl)amino group, an n-butyl(t-butyl)amino group, an n-butyl(c-butyl)amino group, an i-butyl(s-butyl)amino group, an i-butyl(t-butyl)amino group, an 1-butyl(c-butyl)amino group, an s-butyl(t-butyl)amino group, an s-butyl(c-butyl)amino group, a t-butyl(c-butyl)amino group and the like; and examples of the C₆₋₁₄ arylamino group include a phenylamino group, an o-biphenylylamino group, an m-biphenylylamino group, a p-biphenylylamino group, a 1-naphtylamino group, a 2-naphtylamino group, a 1-anthrylamino group, a 2-anthrylamino group, a 9-anthrylamino group, a 1-phenanthrylamino group, a 2-phenanthrylamino group, a 3-phenanthrylamino group, a 4-phenanthrylamino group and a 9-phenanthrylamino group.
Examples of the C₂₋₉ heteroarylamino group include a 5- to 7-membered C₂₋₆ heteromonocyclic amino group which may contain 1 to 3 atoms of an oxygen atom, a nitrogen atom or a sulfur atom singly or in combination, and a C₅₋₉ fused heterobicyclic amino group with a constituent atom number of 8 to 10. Examples of the 5- to 7-membered C₂₋₆ heteromonocyclic amino group include a 2-thienylamino group, a 3-thienylamino group, a 2-furylamino group, a 3-furylamino group, a 2-pyranylamino group, a 3-pyranylamino group, a 4-pyranylamino group, a 1-pyrrolylamino group, a 2-pyrrolylamino group, a 3-pyrrolylamino group, a 1-imidazolylammo group, a 2-imidazolylamino group, a 4-imidazolylamino group, a 1-pyrazolylamino group, a 3-pyrazolylamino group, a 4-pyrazolylamino group, a 2-thiazolylamino group, a 4-thiazolylamino group, a 5-thiazolylamino group, a 3-isothiazolylamino group, a 4-isothiazolylamino group, a 5-isothiazolylamino group, a 2-oxazolylamino group, a 4-oxazolylamino group, a 5-oxazolylamino group, a 3-isoxazolylamino group, a 4-isoxazolylamino group, a 5-isoxazolylamino group, a 2-pyridylamino group, a 3-pyridylamino group, a 4-pyridylamino group, a 2-pyrazinylamino group, a 2-pyrimidinylamino group, a 4-pyrimidinylamino group, a 5-pyrimidinylamino group, a 3-pyridazinylamino group, a 4-pyridazinylamino group, a 2-1,3,4-oxadiazolylamino group, a 2-1,3,4-thiadiazolylamino group, a 3-1,2,4-oxadiazolylamino group, a 5-1,2,4-oxadiazolylamino group, a 3-1,2,4-thiadiazolylamino group, a 5-1,2,4-thiadiazolylamino group, a 3-1,2,5-oxadiazolylamino group, a 3-1,2,5-thiadiazolylamino group and the like.
Examples of the C₅₋₉ fused heterobicyclic amino group with a constituent atom number of 8 to 10 include a 2-benzofuranylamino group, a 3-benzofuranylamino group, a 4-benzofuranylamino group, a 5-benzofuranylamino group, a 6-benzofuranylamino group, a 7-benzofuranylamino group, a 1-isobenzofuranylamino group, a 4-isobenzofuranylamino group, a 5-isobenzofuranylamino group, a 2-benzothienylamino group, a 3-benzothienylamino group, a 4-benzothienylamino group, a 5-benzothienylamino group, a 6-benzothienylamino group, a 7-benzothienylamino group, a 1-isobenzothienylamino group, a 4-isobenzothienylamino group, a 5-isobenzothienylamino group, a 2-chromenylamino group, a 3-chromenylamino group, a 4-chromenylamino group, a 5-chromenylamino group, a 6-chromenylamino group, a 7-chromenylamino group, an 8-chromenylamino group, a 1-indolizinylamino group, a 2-indolizinylamino group, a 3-indolizinylamino group, a 5-indolizinylamino group, a 6-indolizinylamino group, a 7-indolizinylamino group, an 8-indolizinylamino group, a 1-isoindolylamino group, a 2-isoindolylamino group, a 4-isoindolylamino group, a 5-isoindolylamino group, a 1-indolylamino group, a 2-indolylamino group, a 3-indolylamino group, a 4-indolylamino group, a 5-indolylamino group, a 6-indolylamino group, a 7-indolylamino group, a 1-indazolylamino group, a 2-indazolylamino group, a 3-indazolylamino group, a 4-indazolylamino group, a 5-indazolylamino group, a 6-indazolylamino group, a 7-indazolylamino group, a 1-purinylamino group, a 2-purinylamino group, a 3-purinylamino group, a 6-purinylamino group, a 7-purinylamino group, an 8-purinylamino group, a 2-quinolylamino group, a 3-quinolylamino group, a 4-quinolylamino group, a 5-quinolylamino group, a 6-qumolylamino group, a 7-quinolylamino group, an 8-quinolylamino group, a 1-isoquinolylamino group, a 3-isoquinolylamino group, a 4-isoquinolylamino group, a 5-isoquinolylamino group, a 6-isoquinolylamino group, a 7-isoquinolylamino group, an 8-isoquinolylamino group, a 1-phthalazinylamino group, a 5-phthalazinylamino group, a 6-phthalazinylamino group, a 1-2,7-naphthyridinylamino group, a 3-2,7-naphthyridinylamino group, a 4-2,7-naphthyridinylamino group, a 1-2,6-naphthyridinylamino group, a 3-2,6-naphthyridinylamino group, a 4-2,6-naphthyridinylamino group, a 2-1,8-naphthyridinylamino group, a 3-1,8-naphthyridinylamino group, a 4-1,8-naphthyridinylamino group, a 2-1,7-naphthyridinylamino group, a 3-1,7-naphthyridinylamino group, a 4-1,7-naphthyridinylamino group, a 5-1,7-naphthyridinylamino group, a 6-1,7-naphthyridinylamino group, an 8-1,7-naphthyridinylamino group, a 2-1,6-naphthyridinylamino group, a 3-1,6-naphthyridinylamino group, a 4-1,6-naphthyridinylamino group, a 5-1,6-naphthyridinylamino group, a 7-1,6-naphthyridinylamino group, an 8-1,6-naphthyridinylamino group, a 2-1,5-naphthyridinylamino group, a 3-1,5-naphthyndinylamino group, a 4-1,5-naphthyridinylamino group, a 6-1,5-naphthyridinylamino group, a 7-1,5-naphthyridinylamino group, an 8-1,5-naphthyridinylamino group, a 2-quinoxalinylamino group, a 5-quinoxalinylamino group, a 6-quinoxalinylamino group, a 2-quinazolinylamino group, a 4-quinazolinylamino group, a 5-quinazolinylamino group, a 6-quinazolinylamino group, a 7-quinazolinylamino group, an 8-quinazolinylamino group, a 3-cinnolinylamino group, a 4-cinnolinylamino group, a 5-cinnolinylamino group, a 6-cinnolinylamino group, a 7-cinnolinylamino group, an 8-cinnolinylamino group, a 2-pteridinylamino group, a 4-pteridinylamino group, a 6-pteridinylamino group, a 7-pteridinylamino group and the like.
Examples of the C₁₋₆ alkylcarbonylamino group include a methylcarbonylamino group, an ethylcarbonylamino group, an n-propylcarbonylamino group, an i-propylcarbonylamino group, an n-butylcarbonylamino group, an i-butylearbonylamino group, an s-butylcarbonylamino group, a t-butylcarbonylamino group, a I -pentyl carbonyl amino group, a 2-pentylcarbonylamino group, a 3-pentylcarbonylamino group, an i-pentylcarbonylamino group, a neopentylcarbonylamino group, a t-pentylcarbonylamino group, a 1-hexylcarbonylamino group, a 2-hexylcarbonylamino group, a 3-hexylcarbonylamino group and the like; examples of the C₁₋₆ alkylsulfonamide group include a methanesulfonamide group, an ethanesulfonamide group, an n-propanesulfonamide group, an i-propanesulfonamide group, an n-butanesuifonamide group, an i-butanesulfonamide group, an s-butanesulfonamide group, a t-butanesulfonamide group, a 1-pentanesulfonamide group, a 2-pentanesulfonamide group, a 3-pentanesulfonamide group, an i-pentanesulfonamide group, a neopentanesulfonamide group, a t-pentanesulfonamide group, a 1-hexanesuifonamide group, a 2-hexanesulfonamide group, a 3-hexanesulfonamide group and the like; examples of the C₁₋₆ alkylaminocarbonyl group include a methylaminocarbonyl group, an ethylaminocarbonyl group, an n-propylaminocarbonyl group, an i-propylaminocarbonyl group, an n-butylaminocarbonyl group, an i-butylaminocarbonyl group, an s-butylaminocarbonyl group, a t-butylaminocarbonyl group, a 1-pentylaminocarbonyl group, a 2-pentylaminocarbonyl group, a 3-pentylaminocarbonyl group, an i-pentylaminocarbonyl group, neopentylaminocarbonyl, a t-pentylaminocarbonyl group, a 1-hexylaminocarbonyl group, a 2-hexylaminocarbonyl group, a 3-hexylaminocarbonyl group and the like; examples of the di-C₁₋₆ alkylaminocarbonyl group include a dimethylaminocarbonyl group, a diethylaminocarbonyl group, di-n-propylaminocarbonyl, a di-i-propylaminocarbonyl group, a di-c-propylaminocarbonyl group, a di-n-butylaminocarbonyl group, a di-i-butylaminocarbonyl group, a di-s-butylaminocarbonyl group, a di-t-butylaminocarbonyl group, a di-c-butylaminocarbonyl group, a di-1-pentylaminocarbonyl group, a di-2-pentylaminocarbonyl group, a di-3-pentylaminocarbonyl group, a di-i-pentylaminocarbonyl group, a di-neopentylaminocarbonyl group, a di-t-pentylaminocarbonyl group, a di-c-pentylaminocarbonyl group, a di-1-hexylaminocarbonyl group, a di-2-hexylaminocarbonyl group, a di-3-hexylaminocarbonyl group, a di-c-hexylaminocarbonyl group, a di-(1-methyl-n-pentyl)aminocarbonyl group, a di-(1,1,2-trimethyl-n-propyl)aminocarbonyl group, a di-(1,2,2-trimethyl-n-propyl)aminocarbonyl group, a di-(3,3-dimethyl-n-butyl)aminocarbonyl group, a methyl(ethyl)aminocarbonyl group, a methyl(n-propyl)aminocarbonyl group, a methyl(i-propyl)aminocarbonyl group, a methyl(c-propyl)aminocarbonyl group, a methyl(n-butyl)aminocarbonyl group, a methyl(i-butyl)aminocarbonyl group, a methyl(s-butyl)aminocarbonyl group, a methyl(t-butyl)aminocarbonyl group, a methyl(c-butyl)aminocarbonyl group, an ethyl(n-propyl)aminocarbonyl group, an ethyl(i-propyl)aminocarbonyl group, an ethyl(c-propyl)aminocarbonyl group, an ethyl(n-butyl)aminocarbonyl group, an ethyl(i-butyl)aminocarbonyl group, an ethyl(s-butyl)aminocarbonyl group, an ethyl(t-butyl)aminocarbonyl group, an ethyl(c-butyl)aminocarbonyl group, an n-propyl(i-propyl)aminocarbonyl group, an n-propyl(c-propyl)aminocarbonyl group, an n-propyl(n-butyl)aminocarbonyl group, an n-propyl(i-butyl)aminocarbonyl group, an n-propyl(s-butyl)aminocarbonyl group, an n-propyl(t-butyl)aminocarbonyl group, an n-propyl(c-butyl)aminocarbonyl group, an i-propyl(c-propyl)aminocarbonyl group, an i-propyl(n-butyl)aminocarbonyl group, an i-prapyl(i-butyl)aminocarbonyl group, an i-propyl(s-butyl)aminocarbonyl group, an i-propyl(t-butyl)aminocarbonyl group, an i-propyl(c-butyl)aminocarbonyl group, a c-propyl(n-butyl)aminocarbonyl group, a c-propyl(i-butyl)aminocarbonyl group, a c-propyl(s-butyl)aminocarbonyl group, a c-propyl(t-butyl)aminocarbonyl group, a c-propyl(c-butyl)aminocarbonyl group, an n-butyl(i-butyl)aminocarbonyl group, an n-butyl(s-butyl)aminocarbonyl group, an n-butyl(t-butyl)aminocarbonyl group, an n-butyl(c-butyl)aminocarbonyl group, an i-butyl(s-butyl)aminocarbonyl group, an i-butyl(t-butyl)aminocarbonyl group, an i-butyl(c-butyl)aminocarbonyl group, an s-butyl(i-butyl)aminocarbonyl group, an s-butyl(c-butyl)aminocarbonyl group, a t-butyl(c-butyl)aminocarbonyl group and the like; examples of the C₁₋₆ alkylcarbonyl group include a methylcarbonyl group, an ethylcarbonyl group, an n-propylcarbonyl group, an i-piopylcarbonyl group, an n-butylcarbonyl group, an i-butylcarbonyl group, an s-butylcarbonyl group, a t-butylcarbonyl group, a 1-pentylcarbonyl group, a 2-pentylcarbonyl group, a 3-pentylcarbonyl group, an i-pentylearbonyl group, a neopentylcarbonyl group, a t-pentylcarbonyl group, a 1-hexylcarbonyl group, a 2-hexylcarbonyl group, a 3-hexylcarbonyl group and the like; and examples of the C₆₋₁₄ arylcarbonyl group include a phenylcarbonyl group, an o-biphenylylcarbonyl group, an m-biphenylylcarbonyl group, a p-biphenylylcarbonyl group, a 1-naphtylcarbonyl group, a 2-naphtylcarbonyl group, a 1-anthrylcarbanyl group, a 2-anthrylcarbonyl group, a 9-anthrylcarbonyl group, a 1-phenanthrylcarbonyl group, a 2-phenanthrylcarbonyl group, a 3-phenanthrylcarbonyl group, a 4-phenanthrylcarbonyl group, a 9-phenanthrylcarbonyl group and the like.
Examples of the C₂₋₉ heteroarylcarbonyl group include a 5- to 7-membered C₂₋₆ heteromonocyclic carbonyl group which may contain 1 to 3 atoms of an oxygen atom, a nitrogen atom or a sulfur atom singly or in combination, and a C₅₋₉ fused heterobicyclic carbonyl group with a constituent atom number of 8 to 10.
Examples of the 5- to 7-membered C₂₋₆ heteromonocyclic carbonyl group include a 2-thienylcarbonyl group, a 3-thienylcarbonyl group, a 2-furylcarbonyl group, a 3-furylcarbonyl group, a 2-pyranylcarbonyl group, a 3-pyranylcarbonyl group, a 4-pyranylcarbonyl group, a 1-pyrrolylcarbonyl group, a 2-pyrrolylcarbonyl group, a 3-pyrrolylcarbonyl group, a 1-imidazolylcarbonyl group, a 2-imidazolylcarbonyl group, a 4-imidazolylcarbonyl group, a 1-pyrazolylcarbonyl group, a 3-pyrazolylcarbonyl group, a 4-pyrazolylcarbonyl group, a 2-thiazolylcarbonyl group, a 4-thiazolylcarbonyl group, a 5-thiazolylcarbonyl group, a 3-isothiazolylcarbonyl group, a 4-isothiazolylcarbonyl group, a 5-isothiazolylcarbonyl group, a 2-oxazolylcarbonyl group, a 4-oxazolylcarbonyl group, a 5-oxazolylcarboriyl group, a 3-isoxazolylcarbonyl group, a 4-isoxazolylcarbonyl group, a 5-isoxazolylcarbonyl group, a 2-pyridylcarbonyl group, a 3-pyridylcarbonyl group, a 4-pyridylcarbonyl group, a 2-pyrazinylcarbonyl group, a 2-pyrimidinylcarbonyl group, a 4-pyrimidinylcarbonyl group, a 5-pyrimidinylcarbonyl group, a 3-pyridazinylcarbonyl group, a 4-pyridazinylearbonyl group, a 2-1,3,4-oxadiazolylcarbonyl group, a 2-1,3,4-thiadiazolylcarbonyl group, a 3-1,2,4-oxadizolylcarbonyl group, a 5-1,2,4-oxadiazolylcarbonyl group, a 3-1,2,4-thiadiazolylcarbonyl group, a 5-1,2,4-thiadiazolylcarbonyl group, a 3-1,2,5-oxadiazolylcarbonyl group, a 3-1,2,5-thiadiazolylcarbonyl group and the like.
Examples of the C₅₋₉ fused heterobicyclic carbonyl group with a constituent atom number of 8 to 10 include a 2-benzofuranylcarbonyl group, a 3-benzofuranylcarbonyl group, a 4-benzofuranylcarbonyl group, a 5-benzofuranylcarbonyl group, a 6-benzofuranylcarbonyl group, a 7-benzofuranylcarbonyl group, a 1-isobenzofuranylcarbonyl group, a 4-isobenzofuranyicarbonyl group, a 5-isobenzofuranylcarbonyl group, a 2-benzothienylcarbonyl group, a 3-benzothienylcarbonyl group, a 4-benzothienylcarbonyl group, a 5-benzothienylcarbonyl group, a 6-benzothienylcarbonyl group, a 7-benzothienylcarbonyl group, a 1-isobenzothienylcarbonyl group, a 4-isobenzothienylcarbonyl group, a 5-isobenzothienylcarbonyl group, a 2-chromenylcarbonyl group, a 3-chromenylcarbonyl group, a 4-chromenylcarbonyl group, a 5-chromenylcarbonyl group, a 6-chromenylcarbonyl group, a 7-chromenylcarbonyl group, an 8-chromenylcarbonyl group, a 1-indolizinylcarbonyl group, a 2-indolizinylcarbonyl group, a 3-indolizinylcarbonyl group, a 5-indolizinylcarbonyl group, a 6-indolizinylcarbonyl group, a 7-indolizinylcarbonyl group, an 8-indolizinylcarbonyl group, a 1-isoindolylcarbonyl group, a 2-isoindolylcarbonyl group, a 4-isoindolylcarbonyl group, a 5-isoindolylcarbonyl group, a 1-indolylcarbonyl group, a 2-indolylcarbonyl group, a 3-indolylcarbonyl group, a 4-indolylcarbonyl group, a 5-indolylcarbonyl group, a 6-indolylcarbonyl group, a 7-indolylcarbonyl group, a 1-indazolylcarbonyl group, a 2-indazolylcarbonyl group, a 3-indazolylcarbonyl group, a 4-indazolylcarbonyl group, a 5-indazolylcarbonyl group, a 6-indazolylcarbonyl group, a 7-indazolylcarbonyl group, a 1-purinylcarbonyl group, a 2-purinylcarbonyl group, a 3-purinylcarbonyl group, a 6-purinylcarbonyl group, a 7-purinylcarbonyl group, an 8-purinylcarbonyl group, a 2-quinolylcarbonyl group, a 3-quinolylcarbonyl group, a 4-quinolylcarbonyl group, a 5-quinolylcarbonyl group, a 6-quinolylcarbonyl group, a 7-quinolylcarbonyl group, an 8-quinolylcarbonyl group, a 1-isoquinolylcarbonyl group, a 3-isoquinolylcarbonyl group, a 4-isoquinolylcarbonyl group, a 5-isoquinolylcarbonyl group, a 6-isoquinolylcarbonyl group, a 7-isoquinolylcarbonyl group, an 8-isoquinolylcarbonyl group, a 1-phthalazinylcarbonyl group, a 5-phthalazinylcarbonyl group, a 6-phthalazinylcarbonyl group, a 1-2,7-naphthyridinylcarbonyl group, a 3-2,7-naphthyridinylcarbonyl group, a 4-2,7-naphthyridinylcarbonyl group, a 1-2,6-naphthyridinylcarbonyl group, a 3-2,6-naphthyridinylcarbonyl group, a 4-2,6-naphthyridinylcarbonyl group, a 2-1,8-naphthyridinylcarbonyl group, a 3-1,8-naphthyridinylcarbonyl group, a 4-1,8-naphthyridinylcarbonyl group, a 2-1,7-naphthyridinylcarbonyl group, a 3-1,7-naphthyridinylcarbonyl group, a 4-1,7-naphthyridinylcarbonyl group, a 5-1,7-naphthyridinylcarbonyl group, a G-1,7-naphthyridinylcarbonyl group, an 8-1,7-naphthyridinylcarbonyl group, a 2-1,6-naphthyridinylcarbonyl group, a 3-1,6-naphthyridinylcarhonyl group, a 4-1,6-naphthyridinylcarbonyl group, a 5-1,6-naphthyridinylcarbonyl group, a 7-1,6-naphthyridinylcarbonyl group, an 8-1,6-naphthyridinylcarbonyl group, a 2-1,5-naphthyridinylcarbonyl group, a 3-1,5-naphthyridinylcarbonyl group, a 4-1,5-naphthyridinylcarbonyl group, a 6-1,5-naphthyridinylcarbonyl group, a 7-1,5-naphthyridinylcarbonyl group, an 8-1,5-naphthyridinylcarbonyl group, a 2-quinoxalinylcarbonyl group, a 5-quinoxalinylcarbonyl group, a 6-quinoxalinylcarbonyl group, a 2-quinazolinylcarbonyl group, a 4-quinazolinylcarbonyl group, a 5-quinazolinylcarbonyl group, a 6-quinazolinylcarbonyl group, a 7-quinazolinylcarbonyl group, an 8-quinazolinylcarbonyl group, a 3-cinnolinylcarbonyl group, a 4-cinnolinylcarbonyl group, a 5-cinnolinylcarbonyl group, a 6-cinnolinylcarbonyl group, a 7-cinnolinylcarbonyl group, an 8-cinnolinylcarbonyl group, a 2-pteridinylcarbonyl group, a 4-pteridinylcarbonyl group, a 6-pteridinylcarbonyl group, a 7-pteridinylcarbonyl group and the like.
Examples of the C₁₋₆ alkoxycarbonyl group include a methoxycarbonyl group, an ethoxycarbonyl group, an n-propoxycarbonyl group, an i-propoxycarbonyl group, an n-butoxycarbonyl group, an i-butoxycarbonyl group, an s-butoxycarbonyl group, a t-butoxycarbonyl group, a 1-pentyloxycarbonyl group, a 2-pentyloxycarbonyl group, a 3-pentyloxycarbonyl group, an i-pentyloxycarbonyl group, a neopentyloxycarbonyl group, a t-pentyloxycarbonyl group, a 1-hexyloxycarbonyl group, a 2-hexyloxycarbonyl group, a 3-hexyloxycarbonyl group and the like; and examples of the C₁₋₆ alkylsulfonyl group include a ethanesulfonyl group, a trifluoromethanesulfonyl group and an ethanesulfonyl group. Examples of the C₆₋₁₄ arylsulfonyl group include a benzenesulfonyl group, an o-biphenylsulfonyl group, an m-biphenylsulfonyl group, a p-biphenylsulfonyl group, a 1-naphthalenesulfonyl group, a 2-naphthalenesulfonyl group, a 1-anthracenesulfonyl group, a 2-anthracenesulfonyl group, a 9-anthracenesulfonyl group, a 1-phenanthrenesulfonyl group, a 2-phenanthrenesulfonyl group, a 3-phenanthrenesulfonyl group, a 4-phenanthrenesulfonyl group, a 9-phenanthrenesulfonyl group and the like.
Examples of the C₂₋₉ heteroarylsulfonyl group include a 5- to 7-membered C₂₋₆ heteromonocyclic sulfonyl group which may contain 1 to 3 atoms of an oxygen atom, a nitrogen atom or a sulfur atom singly or in combination, and a C₅₋₉ fused heterobicyclic sulfonyl group with a constituent atom number of 8 to 10.
Examples of the 5- to 7-membered C₂₋₆ heteromonocyclic sulfonyl group include a 2-thienylsulfonyl group, a 3-thienylsulfonyl group, a 2-furylsulfonyl group, a 3-furylsulfonyl group, a 2-pyranylsulfonyl group, a 3-pyranylsulfonyl group, a 4-pyranylsulfonyl group, a 1-pyrrolylsulfonyl group, a 2-pyrrolylsulfonyl group, a 3-pyrrolylsulfonyl group, a 1-imidazolylsulfonyl group, a 2-imidazolylsulfonyl group, a 4-imidazolylsulfonyl group, a 1-pyrazolylsulfonyl group, a 3-pyrazolylsulfonyl group, a 4-pyrazolylsulfonyl group, a 2-thiazolylsulfonyl group, a 4-thiazolylsulfonyl group, a 5-thiazolylsulfonyl group, a 3-isotltiazolylsulfonyl group, a 4-isothiazolylsulfonyl group, a 5-isothiazolylsulfonyl group, a 2-oxazolylsulfonyl group, a 4-oxazolylsulfonyl group, a 5-oxazolylsulfonyl group, a 3-isoxazolylsulfonyl group, a 4-isoxazolylsulfonyl group, a 5-isoxazolylsulfonyl group, a 2-pyridylsulfonyl group, a 3-pyridylsulfonyl group, a 4-pyridylsulfonyl group, a 2-pyrazinylsulfonyl group, a 2-pyrimidinylsulfonyl group, a 4-pyrimidinylsulfonyl group, a 5-pyrimidinylsulfonyl group, a 3-pyridazinylsulfonyl group, a 4-pyridazinylsulfonyl group, a 2-1,3,4-oxadiazolylsulfonyl group, a 2-1,3,4-thiadiazolylsulfonyl group, a 3-1,2,4-oxadiazolylsulfonyl group, a 5-1,2,4-oxadiazolylsulfonyl group, a 3-1,2,4-thiadiazolylsulfonyl group, a 5-1,2,4-thiadiazolylsulfonyl group, a 3-1,2,5-oxadiazolylsulfonyl group, a 3-1,2,5-thiadiazolylsulfonyl group and the like.
Examples of the C₅₋₉ fused heterobicyclic sulfonyl group with a constituent atom number of 8 to 10 include a 2-benzofuranylsulfonyl group, a 3-benzofuranylsulfonyl group, a 4-benzofuranylsulfonyl group, a 5-benzofuranylsulfonyl group, a 6-benzofuranylsulfonyl group, a 7-benzofuranylsulfonyl group, a 1-isobenzofuranylsulfonyl group, a 4-isobenzofuranylsulfonyl group, a 5-isobenzofuranylsulfonyl group, a 2-benzothienylsulfonyl group, a 3-benzothienylsulfonyl group, a 4-benzothienylsulfonyl group, a 5-benzothienylsulfonyl group, a 6-benzothienylsulfonyl group, a 7-benzothienylsulfonyl group, a 1-isobenzothienylsulfonyl group, a 4-isobenzothienylsulfonyl group, a 5-isobeuzothienylsulfonyl group, a 2-chromenylsulfonyl group, a 3-chroznenylsulfonyl group, a 4-chromenylsulfonyl group, a 5-chromenylsulfonyl group, a 6-chromenylsulfonyl group, a 7-chromenylsulfonyl group, an 8-chromenylsulfonyl group, a 1-indolizinylsulfonyl group, a 2-indolizinylsulfonyl group, a 3-indolizinylsulfonyl group, a 5-indolizinyisulfonyl group, a 6-indolizinylsulfonyl group, a 7-indolizinylsulfonyl group, an 8-indoiizinylsuifonyl group, a 1-isoindolylsulfonyl group, a 2-isoindolylsulfonyl group, a 4-isoindolylsulfonyl group, a 5-isoindolylsulfonyl group, a 1-indolylsulfonyl group, a 2-indolylsulfonyl group, a 3-indolylsulfonyl group, a 4-indolylsulfonyl group, a 5-indolylsulfonyl group, a 6-indolylsulfonyl group, a 7-indolylsulfonyl group, a 1-indazolylsulfonyl group, a 2-indazolylsulfonyl group, a 3-indazolylsulfonyl group, a 4-indazolylsulfonyl group, a 5-indazolylsulfonyl group, a 6-indazolylsulfonyl group, a 7-indazolylsulfonyl group, a 1-purinylsulfonyl group, a 2-purinylsulfonyl group, a 3-purinylsulfonyl group, a 6-purinylsulfonyl group, a 7-purinylsulfonyl group, an 8-purinylsulfonyl group, a 2-quinolylsulfonyl group, a 3-quinolylsulfonyl group, a 4-quinolylsulfonyl group, a 5-quinolylsulfonyl group, a 6-quinolylsulfonyl group, a 7-quinolylsulfonyl group, an 8-quinolylsulfonyl group, a 1-isoquinolylsulfonyl group, a 3-isoquinolylsulfonyl group, a 4-isoquinolylsulfonyl group, a 5-isoquinolyisulfonyl group, a 6-isoquinolylsulfonyl group, a 7-isoquinolylsulfonyl group, an 8-isoquinolylsulfonyl group, a 1-phthalazinylsulfonyl group, a 5-phthalazinylsulfonyl group, a 6-phthalazinylsulfonyl group, a 1-2,7-naphthyridinylsulfonyl group, a 3-2,7-naphthyridinylsulfonyl group, a 4-2,7-naphthyridinylsulfonyl group, a 1-2,6-naphthyridinylsulfonyl group, a 3-2,6-naphthyridinylsulfonyl group, a 4-2,6-naphthyridinylsulfonyl group, a 2-1,8-naphthyridinylsulfonyl group, a 3-1,8-naphthyridinylsulfonyl group, a 4-1,8-naphthyridinylsulfonyl group, a 2-1,7-naphthyridinylsulfonyl group, a 3-1,7-naphthyridinylsulfonyl group, a 4-1,7-naphthyridinylsulfonyl group, a 5-1,7-naphthyridinylsulfonyl group, a 6-1,7-naphthyridinylsuifonyl group, an 8-1,7-naphthyridinylsulfonyl group, a 2-1,6-naphthyridinylsulfonyl group, a 3-1,6-naphthyridinylsulfonyl group, a 4-1,6-naphthyridinylsulfonyl group, a 5-1,6-naphthyridinylsulfonyl group, a 7-1,5-naphthyridinylsulfonyl group, an 8-1,6-naphthyridinylsulfonyl group, a 2-1,5-naphthyridinylsulfonyl group, a 3-1,5-naphthyridinylsulfonyl group, a 4-1,5-naphthyridinylsulfonyl group, a 6-1,5-naphthyridinylsulfonyl group, a 7-1,5-naphthyridinylsulfonyl group, an 8-1,5-naphthyridinylsulfonyl group, a 2-quinoxalinylsulfonyl group, a 5-quinoxalinylsulfonyl group, a 6-quinoxalinylsulfonyl group, a 2-quinazolinylsulfonyl group, a 4-quinazolinylsulfonyl group, a 5-quinazolinylsulfonyl group, a 6-quinazolinylsulfonyl group, a 7-quinazolinylsulfonyl group, an 8-quinazolinylsulfonyl group, a 3-cinnolinylsulfonyl group, a 4-cinnolinylsulfonyl group, a 5-cinnolinylsulfonyl group, a 6-cinnolinylsulfonyl group, a 7-cinnolinylsulfonyl group, an 8-cinnolinylsulfonyl group, a 2-pteridinylsulfonyl group, a 4-pteridinylsulfonyl group, a 6-pteridinylsulfonyl group, a 7-pteridinylsulfonyl group and the like.
Examples of the C₂₋₉ heterocyclyl group include heteromonocyclic and fused heterobicyclic groups having 2 to 9 carbon atoms and one or more atoms freely selected from a group consisting of a nitrogen atom, an oxygen atom and a sulfur atom, and specifically include the following groups: 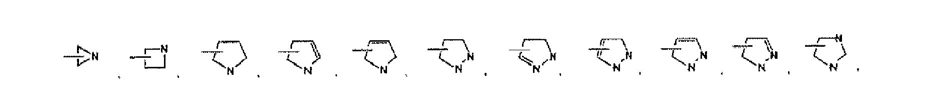 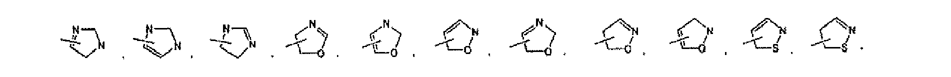 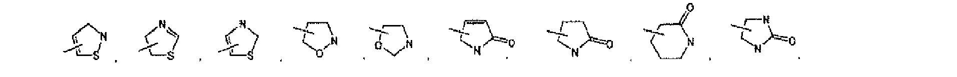 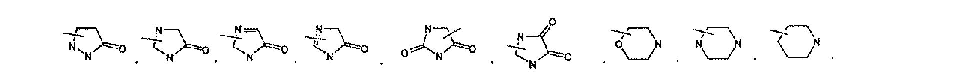 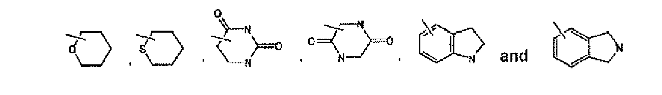 Each "-" (means a chemical bond) on the ring structures in the above-mentioned Formulae means that a substitution site can occupy any position where the substituent can be chemically placed, and does not mean the specific substitution site.

Each of R¹⁶, R¹⁷, R¹⁸ and R¹⁹ in Formula (a), Formula (b), Formula (c), Formula (d), Formula (f), Formula (g), Formula (h), Formula (j), Formula (k), Formula (m), Formula (n), Formula (o), Formula (p), Formula (q), Formula (r), Formula (s), Formula (t), Formula (u), Formula (v), Formula (w), Formula (y), Formula (z), Formula (aa), Formula (ab), Formula (ac), Formula (ad), Formula (ae) and Formula (af) is preferably independently a hydrogen atom, a fluorine atom, a chlorine atom, a bromine atom, a methyl group, an ethyl group, an n-propyl group, an i-propyl group, an n-butyl group, an n-pentyl group, an i-pentyl group, a 3,3-dimethyl-n-butyl group, a methylcarbonyloxymethyl group, an ethylcarbonyloxymethyl group, a methylcarbonyloxyethyl group, an ethylcarbonyloxyethyl group, a methylcarbonylaminomethyl group, an ethylcarbonylaminomethyl group, a methylcarbonylaminoethyl group, an ethylcarbonylaminoethyl group, a methoxycarbonylmethyl group, an ethoxycarbonylmethyl group, a methoxycarbonylethyl group, an ethoxycarbonylethyl group, a methoxy group, an ethoxy group, an n-propoxy group, an i-propoxy group, a phenyl group, an o-biphenylyl group, an m-biphenylyl group, a p-biphenylyl group, a 1-naphtyl group, a 2-naphtyl group, a 2-pyridyl group, a 3-pyridyl group, a 4-pyridyl group, a methylcarbonyloxy group, an ethylcarbonyloxy group, an n-propylcarbonyloxy group, an i-propylcalbonyloxy group, an ii-butylcarbonyloxy group, a t-butylcarbonyloxy group, a methylamino group, an ethylamino group, an n-propylamino group, an i-propylamino group, an n-butylamino group, a dimethylamino group, a diethylamino group, a di-n-propylamino group, a di-i-propylamino group, a di-n-butylamino group, a phenylamino group, an o-biphenylylamino group, an m-biphenylylamino group, a p-biphenylylamino group, a 1-naphtylamino group, a 2-naphtylamino group, a 2-pyridylamino group, a 3-pyridylamino group, a 4-pyridylamino group, a methylcarbonylamino group, an ethylcarbonylamino group, an n-propylcarbonylamino group, an i-propylcarbonylamino group, an n-butylcarbonylamino group, a methanesulfonamide group, an ethanesulfonamide group, an n-propanesulfonamide group, an i-propanesulfonamide group, an n-butanesulfonamide group, a methylaminocarbonyl group, an ethylaminocarbonyl group, an n-propylaminocarbonyl group, an i-propylaminocarbonyl group, an n-butylaminocarbonyl group, a dimethylaminocarbonyl group, a diethylaminocarbonyl group, a di-n-propylaminocarbonyl group, a di-i-propylaminocarbonyl group, a di-c-propylaminocarbonyl group, a di-n-butylaminocarbonyl group, a methylcarbonyl group, an ethylcarbonyl group, an n-propylcarbonyl group, an i-propylcarbonyl group, an n-butylcarbonyl group, a phenylcarbonyl group, an o-biphenylylcarbonyl group, an m-biphenylylcarbonyl group, a p-biphenylylcarbonyl group, a 1-naphtylcarbonyl group, a 2-naphtylcarbonyl group, a 2-pyridylcarbonyl group, a 3-pyridylcarbonyl group, a 4-pyridylcarbonyl group, a methoxycarbonyl group, an ethoxycarbonyl group, an n-propoxycarbonyl group, an i-propoxycarbonyl group, an n-butoxycarbonyl group, an i-butoxycarbonyl group, an s-butoxycarbonyl group, a t-butoxycarbonyl group, a methanesulfonyl group, a trifluoromethanesulfonyl group, a benzenesulfonyl group, an o-biphenyisulfonyl group, an m-biphenylsulfonyl group, a p-biphenylsulfonyl group, a 1-naphthalenesulfonyl group, a 2-naphthalenesulfonyl group, a 2-pyridylsulfonyl group, a 3-pyridylsulfonyl group, a 4-pyridylsulfonyl group, or 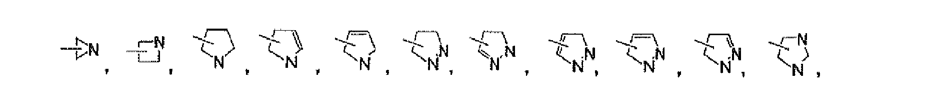 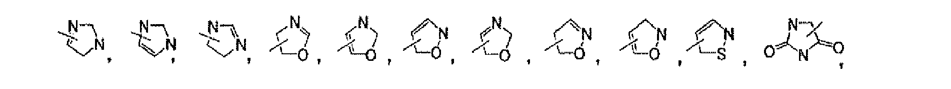 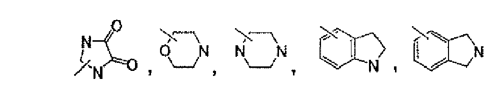 Each "-" (means a chemical bond) on the ring structures in the above mentioned Formulae means that a substitution site can occupy any position where the substituent can be placed chemically, and does not mean the specific substitution site.

Hereinafter, the present invention will be described in more detail by Examples, but the invention is not limited to the Examples.

### [Examples]

### (Example 1)

The optically active titanium-salan complex (3.1 mg, 0.0026 mmol, a catalytic amount of 1 mol%) (synthesized according to the method described in Patent Document 1 : International Publication WO 06/087874, pamphlet) represented by Formula (I): 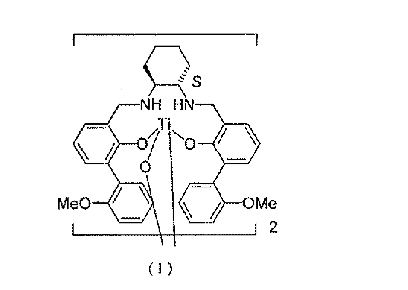 (S in Formula (I) means the absolute configuration (S)) and indene (30 mg, 0,26 mmol) were dissolved in dichloromethane (1.2 mL). A phosphate buffer solution adjusted to pH 7 (50 mM, 90 mg, 0.0045 mmol (calculated on the basis of phosphoric acid)) was added to the reaction solution, subsequently 30% aqueous hydrogen peroxide (44 mg, 0.39 mmol) was added, and the mixture was stirred at 40°C to react. The conversion rate of the reaction (%), the relative area percentage of by-products (%) and the optical purity (%ee) were analyzed by HPLC (analysis conditions: Daicel CHIRALCEL OJ, hexane/isopropanol (8/2 = v/v), a flow rate of 0.8 mL/min, a wavelength of 210 nm, 35°C) after 2 hours and 4 hours, respectively. The conversion rate from indene to (1S,2R)-indene oxide was calculated from the HPLC analysis by using the sensitive ratio at a wavelength of 210 nm. The results are shown in the following Table 1.

### (Table 1)

**Table 1 (Example 1)**

| Reaction time (h) | Conversion rate (%) | HPLC relative area percentage, 210 nm | |
|---|---|---|---|
| | | Retention time 6.2 to 7.8 min | Retention time 10.8 min |
| | | Total relative areas of by-products (%) | Relative area of by-product (%) |
| 2h | 80% | Not detected | Not detected |
| 4h | >99% | 0.2% | Not detected |

The optical purity was 98%ee.

### (Comparative Example 1)

The reaction was carried out in the same experimental procedure as in Example 1 except that the phosphate buffer solution was not added. The conversion rate of the reaction (%), the relative area percentage of by-products (%) and the optical purity (%ee) were analyzed by HPLC (analysis conditions: Daicel CHIRALCEL OJ, hexane/isopropanol (8/2 = v/v), a flow rate of 0.8 mL/min, a wavelength of 210 nm, 35°C) after 2 hours and 4 hours, respectively. The results are shown in the following Table 2.

### (Table 2)

**Table 2 (Comparative Example 1)**

| Reaction time (h) | Conversion rate (%) | HPLC relative area percentage, 210 nm | |
|---|---|---|---|
| | | Retention time 6.2 to 7.8 min | Retention time 10.8 min |
| | | Total relative areas of by-products (%) | Relative area of by-product (%) |
| 2h | 87% | 0.6% | Not detected |
| 4h | >99% | 10% | 14% |

The optical purity was 98%ee. The by-products remarkably increased after 4 hours of the reaction time, and as an observation matter, precipitates not observed in Example 1 were observed in the solution.

### (Example 2: Example 2A, Example 2B and Example 2C)

To a dichloromethane solution (11.6 mL) dissolving the optically active salan ligand (92.6 mg, 0.17 mmol, a catalytic amount of 1 mol%) represented by Formula (II): 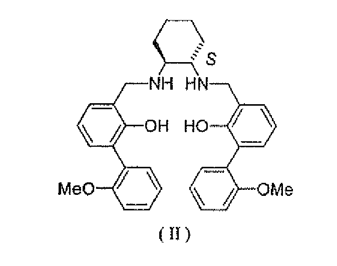 (S in Formula (II) means the absolute configuration (S)), a dichloromethane solution dissolving Ti(Oi-Pr)₄ (48.9 mg, 0.17 mmol, a catalytic amount of 1 mol%) was added and the mixture was stirred at 25°C for 1 hour. Then, without isolation of the optically active titanium-salan complex, dichloromethane (37.7 mL), indene (2.0 g, 17.2 mmol) and a phosphate buffer solution (the mass was 3 times with respect to that of indene, the concentration and pH are shown in the following Table) were continuously added to the reaction solution. Subsequently, commercially available 30% aqueous hydrogen peroxide (2.9 g, 25.8 mmol) was added and the mixture was stirred at a reaction temperature of 40°C to react. The reacted solution was sampled to check the conversion rate. At the end of the reaction, excess aqueous hydrogen peroxide was treated with an aqueous solution of sodium thiosulfate at 0°C, then water-washing and liquid separation were performed, and (1S,2R)-indene oxide was obtained from the dichloromethane solution and analyzed by HPLC (analysis conditions: Daicel CHIRALCEL OJ, hexane/isopropanol (8/2 = v/v), a flow rate of 0.8 mL/min, a wavelength of 210 nm, 35°C) to determine the quantitative yield (%). The conversion rate of the reaction (%), the relative area percentage of by-products (%) and the optical purity (%ee) were also analyzed by HPLC. The results from the phosphate buffer solutions adjusted to pH 8 in 3 concentration conditions (10 mM (Example 2A), 25 mM (Example 2B) and 100 mM (Example 2C)) are shown in the following Table 3.

### (Table 3)

**Table 3 (Examples 2A, 2B and 2C)**

| Example | Concentration of ph 8 ^{note 1} phosphate buffer solution (mM) | Reaction time (h) | Conversion rate (%) | HPLC relative area percentage, 210 nm | | Quantitative yield (%) | Optical purity (%ee) | Remarks |
|---|---|---|---|---|---|---|---|---|
| | | | | Retention time 6.2 to 7.8 min | Retention time 10.8 min | | | |
| | | | | Total relative areas of by-products (%) | Relative area of by-product (%) | | | |
| 2A | 10 mM | 1 h | 75% | 8% | 0.5% | 73 | 98.0 | a |
| | | 3 h | 99% | 15% | 3% | | | |
| | | 5 h | * | * | * | | | |
| 2B | 25 mM | 1 h | 58% | 6% | n.d. | 98 | 98.3 | |
| | | 3 h | 94% | 12% | n.d. | | | |
| | | 5 h | 98% | 9% | n.d. | | | |
| 2C | 100 mM | 1 h | 59% | 6% | n.d. | 97 | 98.3 | |
| | | 3 h | 93% | 8% | n.d. | | | |
| | | 5 h | 98% | 9% | n.d. | | | |

| | | | | | | | | |
|---|---|---|---|---|---|---|---|---|
| Note 1: The concentrations were calculated on the basis of phosphorus. *: Not measured n.d.: Not detected a: Precipitates appeared while the reaction | | | | | | | | |

### (Comparative Example 2)

The reaction was carried out in the same experimental procedure as in Example 2 except that the phosphate buffer solution was not added. The conversion rate of the reaction (%) and the relative area percentage of by-products (%) were analyzed by HPLC (analysis conditions: Daicel CHIRALCEL OJ, hexane/isopropanol (8/2 = v/v), a flow rate of 0.8 mL/min, a wavelength of 210 nm, 35°C) after 1 hour and 3 hours, respectively. The results are shown in the following Table 4.

### (Table 4)

**Table 4 (Comparative Example 2)**

| Reaction time (h) | Conversion rate (%) | HPLC relative area percentage, 210 nm | |
|---|---|---|---|
| | | Retention time 6.2 to 7.8 min | Retention time 10.8 min |
| | | Total relative areas of by-products (%) | Relative area of by-product (%) |
| 1 h | 55% | 26% | 6% |
| 3 h | 69% | 43% | 10% |

As an observation matter after 3 hours of the reaction, by-products increased and precipitates were observed in the reaction solution. The optical purity was 98.3%ee.

### (Example 3)

The reaction was carried out in the same experimental procedure as in Example 2 with the addition of 25 mM phosphate buffer solution adjusted to pH 11. The conversion rate of the reaction (%) and the relative area percentage of by-products (%) were analyzed by HPLC after 1 hour, 3 hours and 4 hours. The results along with the quantitative yield and the optical purity are shown in the following Table 5.

### (Table 5)

**Table 5 (Example 3)**

| Reaction time (h) | Conversion rate (%) | HPLC relative area percentage, 210 nm | | Quantitative yield (%) | Optical purity (%ee) |
|---|---|---|---|---|---|
| | | Retention time 6.2 to 7.8 min | Retention time 10.8 min | | |
| | | Total relative areas of by-products (%) | Relative area of by-product (%) | | |
| 1 h | 69% | 5% | Not detected | 99 | 98.3 |
| 3 h | 95% | 11% | Not detected | | |
| 4 h | 98% | 9% | Not detected | | |

### (Example 4: Example 4A, Example 4B, Example 4C and Example 4D)

The reactions were carried out in the same experimental procedure as in Example 2 with changing the types of buffer solution. The conversion rate (%) after 4 hours of the reaction time was analyzed by HPLC. The results along with the quantitative yields and the optical purities are shown in the following Table 6.

### (Table 6)

**Table 6 (Example 4: Example 4A, Example 4B, Example 4C and Example 4D)**

| Example | Type of buffer solution | Concentration of buffer solution ^{note 1} (mM) | Conversion rate after 4 hours (%) | Quantitative yield (%) | Optical purity (%ee) |
|---|---|---|---|---|---|
| 4A | pH 10 sodium hydrogen carbonate-sodium hydroxide buffer solution | 50 mM | 86 92 (8 h) | 92 | 98.3 |
| 4B | pH 9 boric acid-sodium hydroxide buffer solution | 10 mM | 99 | 99 | 98.3 |
| 4C | pH 8.4 citric acid buffer solution | 100 mM | 98 | 97 | 98.3 |
| 4D | pH 10 Tris ^{note 2} buffer solution | 100 mM | 91 | 82 | 98.1 |

| | | | | | |
|---|---|---|---|---|---|
| Note 1: Each concentration was calculated on the basis of carbonic acid, boric acid, citric acid or Tris ^{note 2}. Note 2: Tris = tris(hydroxylmethyl)aminomethane | | | | | |

### (Example 5)

### (Example 5: Example 5A and Example 5B)

To a dichloromethane solution (0.5 mL) dissolving the salan ligand (2.7 mg, 0.005 mmol) represented by Formula (III): 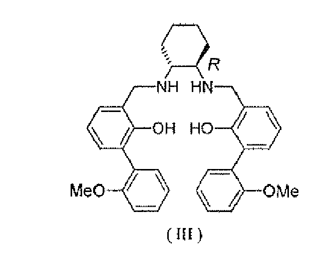 (R in Formula (III) means the absolute configuration (R)), a dichloromethane solution (0.5 mL) dissolving Ti(Oi-Pr)₄ (1.1 mg, 0.004 mmol) was added and the mixture was stirred at 25°C for 1 hour. Then, without isolation of the optically active titanium-salan complex, an unsaturated compound with a carbon-carbon double bond (0.4 mmol) and a phosphate buffer solution adjusted to pH 7.4 (66.7 mM, 68 mg) were continuously added. Subsequently, commercially available 30% aqueous hydrogen peroxide (0.6 mmol) was added and the mixture was stirred at a reaction temperature of 40°C for 6 hours. The solvent of the reaction mixture was removed under reduced pressure and then the residue was purified by a silica gel chromatography to obtain the objective optically active epoxy compound. Furthermore, the optical purity of the obtained optically active epoxy compound was analyzed by HPLC.
The unsaturated compounds with a carbon-carbon double bond represented by Formulae (substrate 1) and (substrate 2): 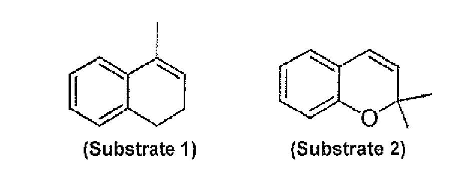 were subjected to the asymmetric epoxidation reaction according to Example 5 to obtain the optically active epoxy compounds represented by Formulae (product 1) and (product 2): 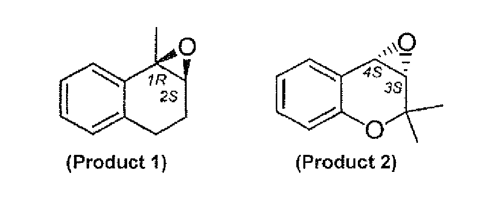 where (product 1) was obtained from (substrate 1) and (product 2) was obtained from (substrate 2). The enantiomeric excess of the product 1 was analyzed by HPLC using a Daicel CHIRALCEL OB-H and a mixed solution of hexane/isopropanol (99/1 = v/v), and the enantiomeric excess of the product 2 was analyzed by HPLC using a Daicel CHIRALCEL OB-H.

### (Example 5: Example 5A and Example 5B)

Example, in which the catalyst was prepared from the salan ligand represented by Formula (III) and Ti(Oi-Pr)₄ in the reaction system and a phosphate buffer solution adjusted to pH 7.4 (66.7 mM) was added to produce the optically active epoxy compound, is shown in the following Table 7.

### (Table 7)

**Table 7 (Example 5: Example 5A and Example 5B)**

| | Substrate *2 (unsaturated compound) | Catalyst (prepared in the reaction system) | | Oxidizing agent | Solvent | Reaction time (hour) | Product *2 (epoxy compound) | Yield (%) | Enantiomeric excess (%ee, absolute configuration) |
|---|---|---|---|---|---|---|---|---|---|
| | | Salan ligand (mol%) *1 | Ti(Oi-Pr)₄ (mol%) *1 | | | | | | |
| Example 5A | Substrate 1 | 1.3 | 1 | Aqueous hydrogen peroxide | Dichloromethane | 6 | Product 1 | 93 | 97, (1R, 2S) |
| Example 5B | Substrate 2 | 1.3 | 1 | Aqueous hydrogen peroxide | Dichloromethane | 6 | Product 2 | 87 | >99, (3S, 4S) |

| | | | | | | | | | |
|---|---|---|---|---|---|---|---|---|---|
| *: 1 The amounts of the salan ligand (III) and Ti(Oi-Pr)₄ used with respect to the substrate. *2 Each structure of the substrate and the product is described above. | | | | | | | | | |

### (Comparative Example 3: Comparative Example 3A and Comparative Example 3B)

Comparative Example 3, in which the catalyst was prepared from the salan ligand represented by Formula (III) and Ti(Oi-Pr)₄ in the reaction system to produce the optically active epoxy compound without the addition of a phosphate buffer solution adjusted to pH 7.4 (66.7 mM), is shown in the following Table 7. The experiment is the same as Example 5 except for the addition of the buffer solution.

### (Table 8)

**Table 8 (Comparative Example 3: Comparative Example 3A and Comparative Example 3B)**

| | Substrate *2 (unsaturated compound) | Catalyst (prepared in the reaction system) | | Oxidizing agent | Solvent | Reaction time (hour) | Product *2 (epoxy compound) | Yield (%) | Enantiomeric excess (%ee, absolute configuration) |
|---|---|---|---|---|---|---|---|---|---|
| | | Sa!an ligand (mol%) *1 | Ti(Oi-Pr)₄ (mol%) *1 | | | | | | |
| Comparative Example 3A | Substrate 1 | 6 | 5 | Aqueous hydrogen peroxide | Dichloro-Methane | 6 | Product 1 | 81 | 96, (1R,2S) |
| Comparative Examples 3B | Substrate 2 | 6 | 5 | Aqueous hydrogen peroxide | Dichloro-Methane | 9 | Product 2 | 75 | >99, (3S,4S) |

| | | | | | | | | | |
|---|---|---|---|---|---|---|---|---|---|
| *1 The amounts of the salan ligand (III) and Ti(Oi-Pr)₄ used with respect to the substrate. *2 Each structure of the substrate and the product is described above. | | | | | | | | | |

### (Reference Experiment A)

The optically active titanium-salan complex (93.1 mg, 0.086 mmol) represented by Formula (IV): 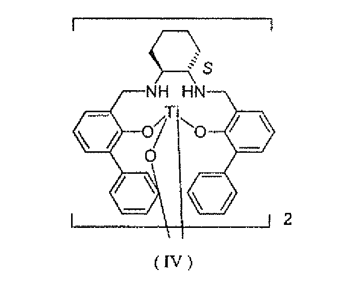 (S in Formula (IV) means the absolute configuration (S)), indene (1000 mg, 8.6 mmol) and ethylbenzene (866 mg, as an internal standard) were dissolved in dichloromethane (24 mL). From the reaction solution two aliquots of 3 mL were taken out to be transferred to 2 reaction containers. To a first reaction solution, a phosphate buffer solution adjusted to pH 8 (67 mM, 375 mg) was added, then 30% aqueous hydrogen peroxide (183 mg) was added and the mixture was stirred at 40°C for 15 min to react. To a second reaction solution, distilled water (375 mg) was added, then 30% aqueous hydrogen peroxide (183 mg) was added and the mixture was stirred at 40°C for 15 min to react. Then, excess aqueous hydrogen peroxide was treated with an aqueous solution of sodium thiosulfate, then water-washing and liquid separation were performed, and each of the dichloromethane solutions was analyzed by HPLC (analysis conditions: Inertsil ODS-3, acetonitrile/20 mM aqueous solution of sodium acetate (96/4 = v/v), a flow rate of 1.0 mL/min, 254 nm, 40°C). The rate of the remaining catalyst in the system with the buffer solution was 71%, and the rate of the remaining catalyst in the system without the buffer solution was 57%.

### INDUSTRIAL APPLICABILITY

According to the production process in the present invention, a prochiral unsaturated compound having a carbon-carbon double bond in the molecule can be epoxidized in high enantioselectivity to produce an optically active epoxy compound. In addition, the optically active epoxy compounds obtained from the production process in the present invention are useful as optically active pharmaceutical intermediates for the compounds effective in the treatments of hypertension, asthma and the like.

## Claims

1. A process for producing an optically active epoxy compound represented by any one of Formula (15), Formula (16), Formula (17), Formula (18), Formula (19) and Formula (20): 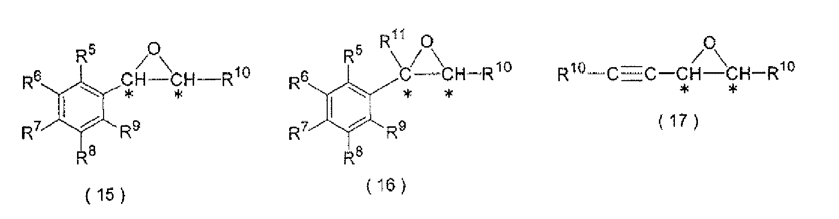 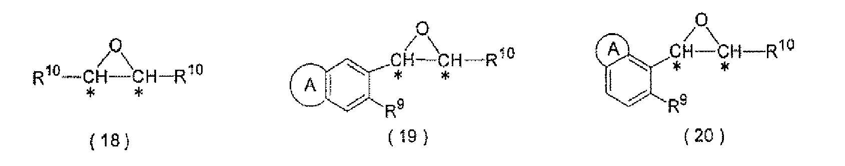 **characterized by** comprising:
asymmetrically epoxidizing an unsaturated compound represented by any one of Formula (5), Formula (6), Formula (7), Formula (8), Formula (9) and Formula (10): 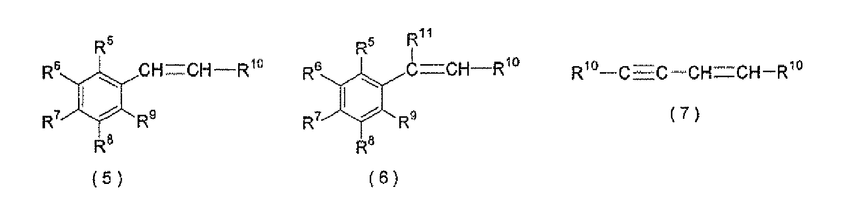 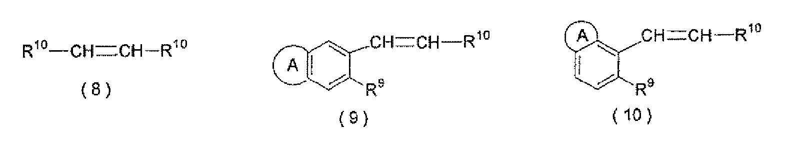 with an oxidizing agent; in the presence of an optically active titanium-salen complex, a titanium-salalen complex or an optically active titanium-salan complex obtained from a reaction of an optically active ligand represented by any one of Formula (1), Formula (1), Formula (2), Formula (2'), Formula (3), Formula (3'), Formula (4) and Formula (4): 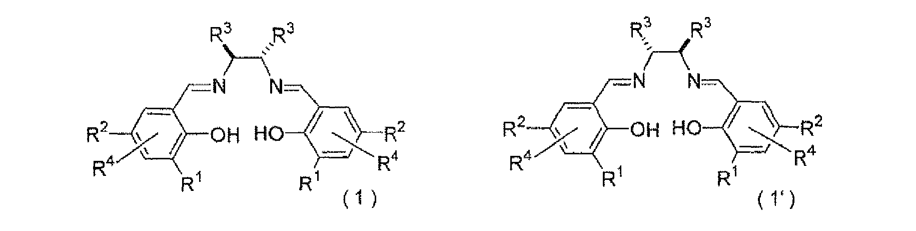 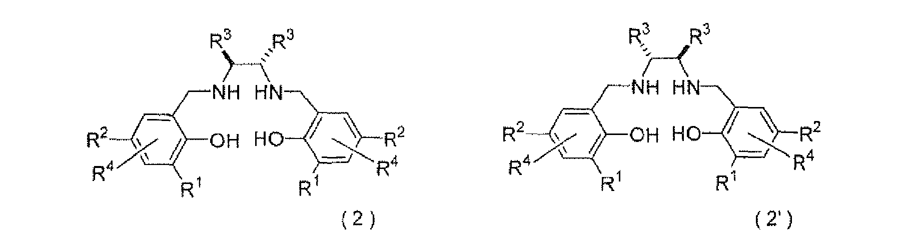 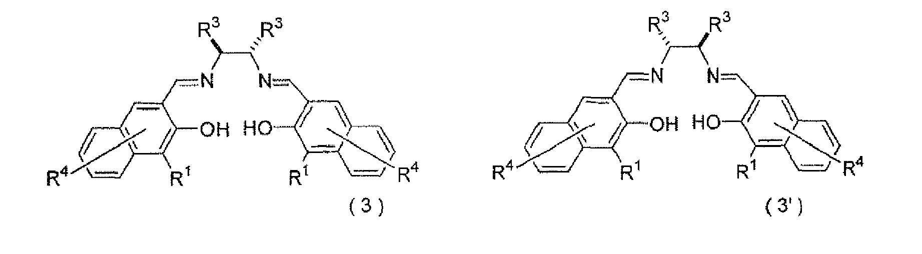 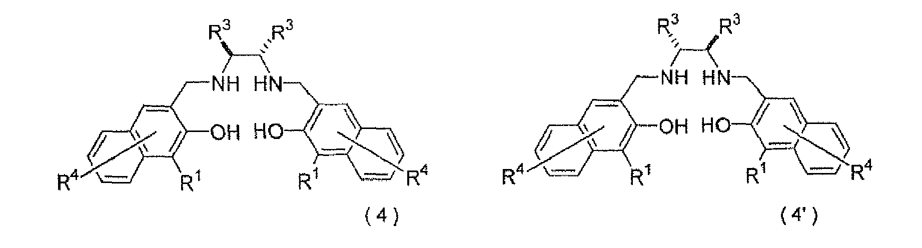 (Formula (1), Formula (1), Formula (3) and Formula (3) are salen ligands and Formula (2), Formula (2'), Formula (4) and Formula (4') are salan ligands), and a titanium compound, and a buffering agent or a buffer solution; wherein
in Formula (1), Formula (1), Formula (2), Formula (2'), Formula (3), Formula (3'), Formula (4) and Formula (4'),
R¹ represents a hydrogen atom, a halogen atom, a C₁₋₄ alkyl group, a C₁₋₄ alkoxy group, a C₆₋₁₂ aryloxy group or a C₆₋₂₂ aryl group (the aryl group is unsubstituted or optionally substituted with a C₁₋₄ alkyl group (the alkyl group is unsubstituted or substituted with a halogen atom), a benzyloxy group or a C₁₋₄ alkoxy group, and is optically active or optically inactive),
R² represents a hydrogen atom, a halogen atom or a C₁₋₄ alkyl group,
R³ represents a C₆₋₁₈ aryl group or, when two R³ are joined together to form a ring, a C₃₋₅ bivalent group, and
each R⁴ independently represents a hydrogen atom, a halogen atom, a C₁₋₄ alkyl group, a C₁₋₄ alkoxy group, a nitro group or a cyano group,
in Formula (5) and Formula (6),
each of R⁵, R⁶, R⁷ and R⁸ independently represents a hydrogen atom, a cyano group, a nitro group, an amino group (the amino group is not protected with a protective group or protected), a halogen atom, a C₁₋₄ alkyl group (the alkyl group is unsubstituted or substituted with a halogen atom), a C₁₋₄ alkoxy group, a carboxy group, a formyl group, a C₁₋₄ alkylcarbonyl group (the alkylcarbonyl group is unsubstituted or substituted with a halogen atom), a C₇₋₁₁ arylcarbonyl group (the arylcarbonyl group is unsubstituted or substituted with a halogen atom), a carbamoyl group, a C₁₋₄ alkylsulfinyl group, a C₆₋₁₀ arylsulfinyl group, a C₁₋₄ alkylsulfonyl group, a C₆₋₁₀ arylsulfonyl group, a sulfamoyl group, a monoalkylaminosulfonyl group or a C₂₋₈ dialkylaminosulfonyl group,
in Formula (5), Formula (6), Formula (9) and Formula (10),
R⁹ represents a hydrogen atom, a C₁₋₄ alkyl group or a C₁₋₄ alkoxy group,
in Formula (5), Formula (6), Formula (9) and Formula (10),
R¹⁰ represents a hydrogen atom, a C₁₋₂₂ alkyl group, a C₁₋₄ alkoxy group or a C₆₋₁₀ aryl group (the aryl group is unsubstituted or substituted with a halogen atom, a C₁₋₄ alkyl group or a C₁₋₄ alkoxy group),
in Formula (6),
R¹¹ represents a C₁₋₄ alkyl group (the alkyl group is unsubstituted or substituted with a halogen atom),
in Formula (7) and Formula (8),
each R¹⁰ independently represents a hydrogen atom, a C₁₋₂₂ alkyl group, a C₁₋₄ alkoxy group or a C₆₋₁₀ aryl group (the aryl group is unsubstituted or substituted with a halogen atom, a C₁₋₄ alkyl group or a C₁₋₄ alkoxy group),
R⁹ and R¹⁰ in Formula (5), Formula (6), Formula (8) and Formula (9) is optionally bind each other to form a bivalent group represented by any one of Formula (10), Formula (11), Formula (12) and Formula (13): 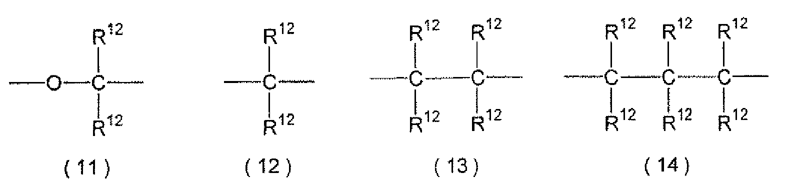
(in Formula (11), Formula (12), Formula (13) and Formula (14),
each R¹² independently represents a hydrogen atom or a C₁₋₆ alkyl group),
in Formula (9) and Formula (10),
a partial ring structure A represents any one of 5-, 6- and 7-membered rings fused with a benzene ring (each of the 5-, 6- and 7-membered rings is unsubstituted or substituted with h pieces of R¹³ (R¹³ represents a halogen atom, a hydroxyl group, a C₁₋₆ alkyl group (the alkyl group is unsubstituted or substituted with a halogen atom, a hydroxyl group, a cyano group, an amino group, a nitro group, a C₁₋₄ alkoxy group, a C₁₋₄ alkylcarbonyloxy group, a C₁₋₄ alkylcarbonylamino group or a C₁₋₄ alkoxycarbonyl group (the alkoxy group is unsubstituted or the alkylcarbonyloxy group, the alkylcarbonylamino group or the alkoxycarbonyl group is substituted with a halogen atom)), a C₁₋₆ alkoxy group (the alkoxy group is unsubstituted or substituted with a halogen atom, a hydroxyl group, a cyano group, an amino group, a nitro group, a C₁₋₄ alkoxy group, a C₁₋₄ alkylcarbonyloxy group, a C₁₋₄ alkylcarbonylamino group or a C₁₋₄ alkoxycarbonyl group (the alkoxy group, the alkylcarbonyloxy group, the alkylcarbonylamino group and the alkoxycarbonyl group are unsubstituted or substituted with halogen atoms)), a nitro group, a cyano group, a formyl group, a formamide group, a carbamoyl group, a sulfo group, a sulfoamino group, a sulfamoyl group, a sulfonyl group, an amino group, a carboxyl group, a C₁₋₆ alkylamino group, a di-C₁₋₆ alkylamino group, a C₁₋₆ alkylcarbonylamino group, a C₁₋₆ alkylsulfonamide group, a C₆₋₁₄ arylsulfonamide group, a C₁₋₆ alkylaminocarbonyl group, a di-C₁₋₆ alkylaminocarbonyl group, a C₁₋₆ alkylcarbonyl group, a C₁₋₆ alkoxycarbonyl group, a C₁₋₆ alkylsulfonyl group, a C₆₋₁₄ arylsulfonyl group or a C₆₋₁₄ arylcarbonyl group (the alkylamino group, the dialkylamino group, the alkylcarbonylamino group, the alkylsulfonamide group, the arylsulfonamide group, the alkylaminocarbonyl group, the dialkylaminocarbonyl group, the alkylcarbonyl group, the alkoxycarbonyl group, the alkylsulfonyl group, the arylsulfonyl group and the arylcarbonyl group are unsubstituted or substituted with halogen atoms), h means an integer from 1 to 6, and when h is 2 to 6, each R¹³ is optionally the same or different), and is capable of including 1 to 3 atoms of an oxygen atom, a nitrogen atom or a sulfur atom singly or in combination as a ring constituent atom, the number of unsaturated bonds in the ring is 1, 2 or 3 including the unsaturated bond in the fused benzene ring, and a carbon atom constituting the ring is optionally carbonyl or thiocarbonyl), and in Formula (15), Formula (16), Formula (17), Formula (18), Formula (19) and Formula (20),
R⁵, R⁶, R⁷, R⁸, R⁹, R¹⁰ and R¹¹ are the same as the above, and the absolute configuration of the carbon atom shown with an asterisk (*) means (R) or (S).

2. The process for producing an optically active epoxy compound according to claim 1, wherein the optically active ligand is represented by Formula (1), Formula (1'), Formula (2), Formula (2'), Formula (3), Formula (3'), Formula (4) and Formula (4')
(in Formula (1), Formula (1'), Formula (2), Formula (2'), Formula (3), Formula (3'), Formula (4) and Formula (4'),
R¹ represents a phenyl group (the phenyl group is substituted with a 2-C₁₋₃ alkyl group (the 2-C₁₋₃ alkyl group is substituted with at least one halogen atom), a benzyloxy group or a 2-C₁₋₄ alkoxy group), a 2-phenyl-1-naphtyl group or a 2-methoxy-1-naphtyl group,
R² represents a hydrogen atom,
R³ represents a tetramethylene group as the bivalent group, and
R⁴ represents a hydrogen atom).

3. The process for producing an optically active epoxy compound according to claim 1, wherein the optically active epoxy compound is represented by Formula (21) or Formula (22): 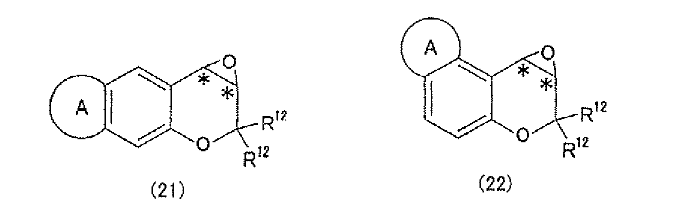 (in Formula (21) and Formula (22),
the partial ring structure A and R¹² are the same as the above, and the absolute configuration of the carbon atom shown with * means (R) or (S)), and **characterized in that** the unsaturated compound is asymmetrically epoxidized with the oxidizing agent, in which the partial ring structure A in Formula (9) or Formula (10) is represented by any one of Formula (a), Formula (b), Formula (c), Formula (d), Formula (e), Formula (f), Formula (g), Formula (h), Formula (i), Formula (j), Formula (k), Formula (l), Formula (m), Formula (n), Formula (o), Formula (p), Formula (q), Formula (r), Formula (s), Formula (t), Formula (u), Formula (v), Formula (w), Formula (x), Formula (y), Formula (z), Formula (aa), Formula (ab), Formula (ac), Formula (ad), Formula (ae), Formula (af), Formula (ag) and Formula (ah): 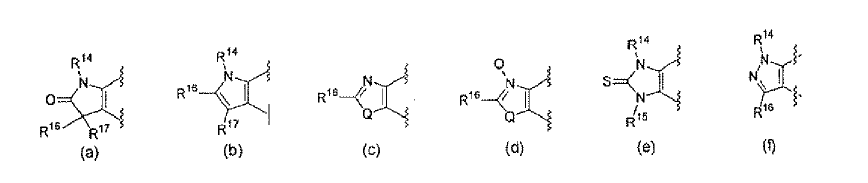 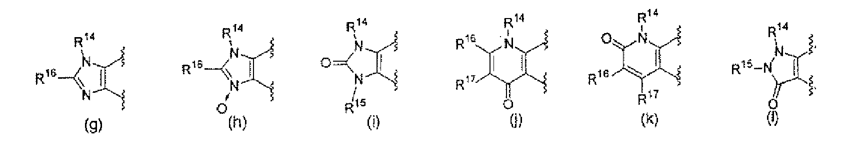 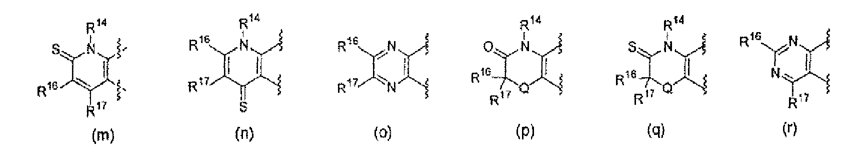 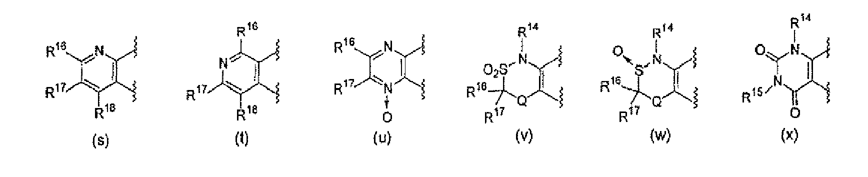 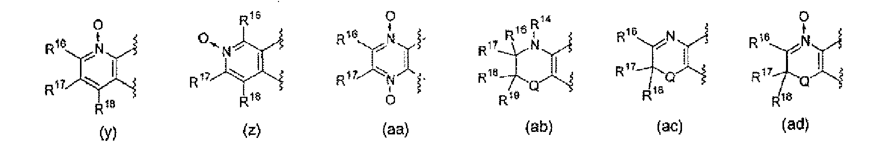 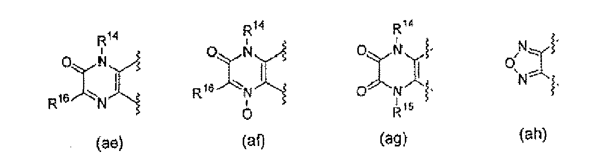 (in Formula (a), Formula (b), Formula (e), Formula (f), Formula (g), Formulae (h), Formula (i), Formula (j), Formula (k), Formulae (l), Formula (m), Formula (n),
Formula (p), Formula (q), Formula (v), Formula (w), Formula (x), Formula (ab), Formula (ae), Formula (af) and Formula (ag),
each of R¹⁴ and R¹⁵ independently represents a hydrogen atom, a C₁₋₆ alkyl group (the alkyl group is unsubstituted or substituted with a halogen atom, a C₁₋₆ alkoxy group (the alkoxy group is unsubstituted or substituted with a halogen atom), an amino group, a hydroxyl group, a C₆₋₁₄ aryl group, a C₂₋₉ heteroaryl group (each of the aryl group and the heteroaryl group is unsubstituted or substituted with q pieces of R²⁰ (R²⁰ means the same as R¹³, q represents an integer from 1 to 3, and when q is 2 or 3, R²⁰ is optionally the same or different)), a C₁₋₆ alkylaminocarbonyl group, a di-C₁₋₆ alkylaminocarbonyl group, a C₁₋₆ alkylcarbonyloxy group, a C₁₋₆ alkylcarbonyl group (the alkylcarbonyloxy group and the alkylcarbonyl group are unsubstituted or substituted with halogen atoms), a C₁₋₆ alkylcarbonylamino group, a C₃₋₈ cycloalkylcarbonyl group, a C₁₋₆ alkoxycarbonyl group, a C₁₋₆ alkylsulfonyl group (the cycloalkylcarbonyl group, the alkoxycarbonyl group and the alkylsulfonyl group are unsubstituted or substituted with halogen atoms), a carboxyl group, a C₆₋₁₄ arylcarbonyl group (the arylcarbonyl group is unsubstituted or substituted with a halogen atom) or a C₂₋₉ heteroarylcarbonyl group), a C₆₋₁₄ aryl group, a C₂₋₉ heteroaryl group (each of the aryl group and the heteroaryl group is unsubstituted or substituted with q pieces of R²⁰ (R²⁰ means the same as R¹³, q represents an integer from 1 to 3, and when q is 2 or 3, R²⁰ is optionally the same or different)), a C₁₋₆ alkylaminocarbonyl group, a di-C₁₋₆ alkylaminocarbonyl group, a C₁₋₆ alkylcarbonyl group, a C₃₋₈ cycloalkylcarbonyl group, a C₁₋₆ alkoxycarbonyl group, a C₁₋₆ alkylsulfonyl group, a C₆₋₁₄ arylsulfonyl group, a C₂₋₉ heteroarylsulfonyl group (each of the arylsulfonyl group and the heteroarylsulfonyl group is unsubstituted or substituted with q pieces of R²⁰ (R²⁰ means the same as R¹³, q represents an integer from 1 to 3, and when q is 2 or 3, R²⁰ is optionally the same or different)), a carboxyl group, a C₆₋₁₄ arylcarbonyl group or a C₂₋₉ heteroarylcarbonyl group (each of the arylcarbonyl group and the heteroarylcarbonyl group is unsubstituted or substituted with q pieces of R²⁰ (R²⁰ means the same as R¹³, q represents an integer from 1 to 3, and when q is 2 or 3, R²⁰ is optionally the same or different)),
in Formula (a), Formula (b), Formula (c), Formula (d), Formula (f), Formula (g), Formula (h), Formula (j). Formula (k), Formula (m), Formula (n), Formula (o), Formula (p), Formula (q), Formula (r), Formula (s), Formula (t), Formula (u), Formula (v), Formula (w), Formula (y), Formula (z), Formula (aa), Formula (ab), Formula (ac), Formula (ad), Formula (ae) and Formula (af),
each of R¹⁶, R¹⁷, R¹⁸ and R¹⁹ independently represents a hydrogen atom, a halogen atom, a C₁₋₆ alkyl group (the alkyl group is unsubstituted or substituted with a halogen atom, a C₁₋₆ alkoxy group (the alkoxy group is unsubstituted or substituted with a halogen atom), an amino group, a hydroxyl group, a C₆₋₁₄ aryl group, a C₂₋₉ heteroaryl group (each of the aryl group and the heteroaryl group is unsubstituted or substituted with r pieces of R²¹ (R²¹ means the same as R¹³ and r means the same as q)), a C₁₋₆ alkylaminocarbonyl group, a di-C₁₋₆ alkylaminocarbonyl group, a C₁₋₆ carbonyloxy group, a C₁₋₆ alkylcarbonyl group (the alkylcarbonyloxy group and the alkylcarbonyl group are unsubstituted or substituted with a halogen atom), a C₁₋₆ alkylcarbonylamino group, a C₃₋₈ cycloalkylcarbonyl group, a C₁₋₆ alkoxycarbonyl group, a C₁₋₆ alkylsulfonyl group (the cycloalkylcarbonyl group, the alkoxycarbonyl group and the alkylsulfonyl group are unsubstituted or substituted with a halogen atom), a carboxyl group, a C₆₋₁₄ arylcarbonyl group (the arylcarbonyl group is unsubstituted or substituted with a halogen atom) or a C₂₋₉ heteroarylcarbonyl group), a C₃₋₈ cycloalkyl group (the cycloalkyl group is unsubstituted or substituted with a halogen atom, a C₁₋₆ alkoxy group (the alkoxy group is unsubstituted or substituted with a halogen atom), an amino group or a hydroxyl group), a C₁₋₆ alkoxy group (the alkoxy group is unsubstituted or substituted with a halogen atom, a C₁₋₆ alkoxy group (the alkoxy group is unsubstituted or substituted with a halogen atom), a carboxyl group, an amino group, a hydroxyl group, a C₆₋₁₄ aryl group or a C₂₋₉ heteroaryl group (each of the aryl group and the heteroaryl group is unsubstituted or substituted with r pieces of R²¹ (R²¹ means the same as R¹³ and r means the same as q))), a C₁₋₆ thioalkoxy group (the thioalkoxy group is unsubstituted or substituted with a halogen atom, a C₁₋₆ alkoxy group (the alkoxy group is unsubstituted or substituted with a halogen atom), a carboxyl group, a hydroxyl group, a C₆₋₁₄ aryl group, or a C₂₋₉ heteroaryl group (each of the aryl group and the heteroaryl group is unsubstituted or substituted with r pieces of R²¹ (R²¹ means the same as R¹³ and r means the same as q))), a hydroxyl group, a C₆₋₁₄ aryl group, a C₂₋₉ heteroaryl group (each of the aryl group and the heteroaryl group is unsubstituted or substituted with r pieces of R²¹ (R²¹ means the same as R¹³ and r means the same as q)), a C₁₋₆ alkylcarbonyloxy group, a nitro group, a cyano group, a formyl group, a formamide group, an amino group, a sulfo group, a C₁₋₆ alkylamino group, a di-C₁₋₆ alkylamino group, a C₆₋₁₄ arylamino group, a C₂₋₉ heteroarylamino group (each of the arylamino group and the heteroarylamino groups is unsubstituted or substituted with r pieces of R²¹ (R²¹ means the same as R¹³ and r means the same as q)), a C₁₋₆ alkylcarbonylamino group, a C₁₋₆ alkylsulfonamide group, a carbamoyl group, a C₁₋₆ alkylaminocarbonyl group, a di-C₁₋₆ alkylaminocarbonyl group, a C₁₋₆ alkylcarbonyl group, a C₆₋₁₄ arylcarbonyl group, a C₂₋₉ heteroarylcarbonyl group (each of the arylcarbonyl group and the heteroarylcarbonyl group is unsubstituted or substituted with r pieces of R²¹ (R²¹ means the same as R¹³ and r means the same as q)), a C₁₋₆ alkoxycarbonyl group, a sulfamoyl group, a C₁₋₆ alkylsulfonyl group, a C₆₋₁₄ arylsulfonyl group, a C₂₋₉ heteroarylsulfonyl group (each of the arylsulfonyl group and the heteroarylsulfonyl group is unsubstituted or substituted with r pieces of R²¹ (R²¹ means the same as R¹³ and r means the same as q)), a carboxyl group or a C₂₋₉ heterocyclyl group (the heterocyclyl group is unsubstituted or substituted with a halogen atom, a C₁₋₆ alkyl group (the alkyl group is unsubstituted or substituted with a halogen atom, a C₁₋₆ alkoxy group (the alkoxy group is unsubstituted or substituted with a halogen atom), an amino group, a carboxyl group or a hydroxyl group), a C₁₋₆ alkoxy group (the alkoxy group is unsubstituted or substituted with a halogen atom), a C₆₋₁₄ aryl group or a C₂₋₉ heteroaryl group (each of the aryl group and the heteroaryl group is unsubstituted or substituted with r pieces of R²¹ (R²¹ means the same as R¹³ and r means the same as q)), a hydroxyl group, a nitro group, a cyano group, a formyl group, a formamide group, an amino group, a C₁₋₆ alkylamino group, a di-C₁₋₆ alkylamino group, a C₁₋₆ alkylcarbonylamino group, a C₁₋₆ alkylsulfonamide group, a carbamoyl group, a C₁₋₆ alkylaminocarbonyl group, a di-C₁₋₆ alkylaminocarbonyl group, a C₁₋₆ alkylcarbonyl group, a C₁₋₆ alkoxycarbonyl group, a sulfamoyl group, a C₁₋₆ alkylsulfonyl group, a carboxyl group or a C₆₋₁₄ arylcarbonyl group), and
in Formula (c), Formula (d), Formula (p), Formula (q), Formula (v), Formula (w), Formula (ab), Formula (ac) and Formula (ad),
Q represents an oxygen atom (O), a sulfur atom (S), a sulfinyl group (SO) or a sulfonyl group (SO₂)),
R⁹ and R¹⁰ in Formula (9) and Formula (10) bind each other to form a bivalent group represented by Formula (11): 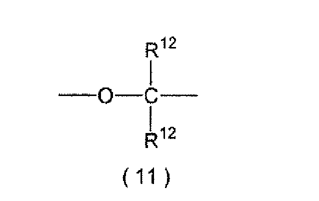
(R¹² in Formula (11) represents a methyl group, the oxygen atom is bonded to the phenyl group, and the carbon atom is bonded to a vinyl group).

4. The process for producing an optically active epoxy compound according to any one of claims 1 to 3, **characterized in that** the oxidizing agent is hydrogen peroxide.

5. The process for producing an optically active epoxy compound according to any one of claims 1 to 4, **characterized in that** pH of a reaction solution is 5 to 12 by an addition of a buffering agent or a buffer solution.

6. The process for producing an optically active epoxy compound according to any one of claims 1 to 5, **characterized in that** the optically active ligand is any one of the optically active ligands represented by Formula (2), Formula (2'), Formula (4) and Formula (4').

7. The process for producing an optically active epoxy compound according to any one of claims 1 to 6, **characterized in that** the optically active ligand is any one of the optically active ligands represented by Formula (1), Formula (1'), Formula (3) and Formula (3'), and the unsaturated compound is asymmetrically epoxidized in the presence of an optically active titanium-salalen complex obtained from a reaction of the optically active ligand and a titanium compound, and a buffering agent or a buffer solution.
